# EUROPEAN PATENT SPECIFICATION

(11) **EP 4 298 237 B1**
(45) Date of publication and mention of the grant of the patent: **25.06.2025**
(21) Application number: 22713485.5
(22) Date of filing: 22.02.2022
(51) Int. Cl.: C12Q 1/6804, C12Q 1/6806, C12Q 1/48

(54) **SINGLE CELL GLYCAN PROFILING**
EINZELZELLGLYCANPROFILIERUNG
PROFILAGE DE GLYCANE MONOCELLULAIRE

(30) Priority: 23.02.2021 US 202163152488 P
(43) Date of publication of application: 03.01.2024
(73) Proprietor: 10X Genomics, Inc., Pleasanton, CA 94588 (US)
(72) Inventor: PATTERSON, David Michael, Pleasanton, California 94588 (US)
(74) Representative: J A Kemp LLP
(86) International application number: PCT/US2022/017356
(87) International publication number: WO 2022/182672

(56) References cited:
- WO-A1-2013/154751
- WO-A1-2017/053905
- US-A1- 2018 179 590

## Description

### FIELD OF INVENTION

The present invention relates to the field of glycobiology and glycomics. The disclosed kits, compositions, systems, and methods allow for profiling of glycans and glycoproteins at a single cell level.

### BACKGROUND

Posttranslational modifications (PTMs) are a central mechanism by which cells regulate the functional capabilities of their proteome. Identifying and characterizing these PTMs remains a major goal in proteomics. Of the more than 300 known protein modifications, glycosylation is one of the most abundant and complex PTMs. The complexity of glycosylation arises from two factors; namely, the diversity of the building blocks as well as the multiple ways in which oligosaccharides can be assembled from these building blocks within the cell. Unlike transcription and translation, glycosylation is not template-driven. Rather glycosylation is a post-translation modification and protein glycosylation is dynamic. Glycoproteins and glycolipids are generated through the enzymatic addition of monosaccharides and complex oligosaccharides onto protein or lipid scaffolds in the secretory pathway. The glycosylation process can produce significant diversity and heterogeneity of products, and glycosylation can alter protein properties, including trafficking, localization, binding specificity, and thermodynamic stability.

Monosaccharide building blocks used during glycosylation can be assembled in diverse linear and branched patterns to generate a complex collection of carbohydrates in an organism, also known as its "glycome". Additional structural diversity results from attachment of monosaccharides to specific sites on protein scaffolds. The concentration of any one glycoprotein may also be very low and a low concentration is particularly challenging in a complex milieu such as a human biological sample. Additionally, any one glycoprotein may be present in multiple glycoforms exhibiting microheterogeneity and macroheterogeneity. In microheterogeneity, different glycan structures are present at a specific site of modification. Furthermore, there are differences in either the location or number of glycan modifications in the glycoprotein. Several monosaccharides are structural isomers of each other and thus have the same molecular weight. Further the linkage chemistry between monosaccharides introduces additional complexity. Because glycosylation is a post-translational process that is not encoded in the genome and because of the complexity of glycan biosynthesis in the secretory pathway, characterizing cell-surface glycan function and diversity is a challenging endeavor. Palaniappian & Bertozzi, Chem. Rev. 116:14277-14306 (2016). WO 2017/053905 and US2018179590 describe affinity-oligonucleotide conjugates (e.g. antibody-oligonucleotide conjugates) which may be used for characterization of cells.

Accordingly, there is a need for a single cell platform that enables a comprehensive and a deeper glycosylation analysis of diverse cell types across multiple pathophysiological states in combination with single-cell sequencing technologies, transcriptome analyses and glycan detection. This disclosure addresses this need.

### SUMMARY

The invention is defined in the appended claims.

Methods of analyzing the glycome are provided herein. One aspect of the present disclosure provides a method of determining the presence of one or more glycans in a sample comprising the steps of: (a) incubating the sample with (i) a first flag molecule comprising a nucleotide sugar and a first reactive molecule of a reaction pair and (ii) a first glycan specific transferase, wherein the first flag molecule is a substrate for the first glycan specific transferase; wherein the first flag molecule is incorporated onto a glycan-modified glycoprotein in the sample, and wherein the sample is selected from the group consisting of a tissue, a cell, a fixed cell, a live cell, and cell lysates; (b) admixing the sample with a first reporter molecule comprising (i) a second reactive molecule of the reaction pair and (ii) a first reporter oligonucleotide comprising a first glycan motif-specific reporter barcode sequence, wherein the second reactive molecule of the reaction pair couples to the first reactive molecule of the reaction pair, whereby the first reporter molecule is conjugated to the glycan-modified glycoprotein via the first flag molecule; (c) removing unincorporated reporter molecules from the sample; (d) partitioning the sample into a plurality of partitions such that a partition comprises (i) a single cell or single cell lysate from the sample and (ii) a plurality of nucleic acid barcode molecules comprising a partition-specific barcode sequence; (e) using the first reporter oligonucleotide and a nucleic acid barcode molecule of the plurality of nucleic acid barcode molecules to generate a first glycan barcoded nucleic acid molecule comprising the partition-specific barcode sequence or complement thereof and the first glycan motif-specific reporter barcode sequence or a complementary sequence thereof; (f) determining the sequence of the first glycan barcoded nucleic acid molecule or derivative thereof to identify (i) the partition-specific barcode sequence or complementary sequence thereof and (ii) the first glycan motif-specific reporter barcode sequence or complementary sequence thereof; and (g) using the identified partition-specific barcode sequence or complementary sequence thereof and the identified first glycan motif-specific reporter barcode sequence or complementary sequence thereof to determine the presence and/or abundance of at least one glycan in the sample.

In some embodiments, the method of determining the presence of one or more glycans in a sample further comprises: (a) incubating the sample with (i) a second flag molecule comprising a second nucleotide sugar and a first reactive molecule of a second reaction pair and (ii) a second glycan specific transferase; and (b) admixing the sample with a second reporter molecule comprising (i) a second reactive molecule of a second reaction pair, wherein the second reactive molecule is capable of coupling to the first reactive molecule of the second reaction pair, and (ii) a second reporter oligonucleotide comprising a reporter barcode sequence that identifies a second glycan motif. In one embodiment, the second flag molecule is incorporated onto a glycan-modified glycoprotein in the sample. In one embodiment, the second reporter molecule is conjugated on the glycan-modified glycoprotein via the second flag molecule.

In one embodiment, the method further comprises the step of: (a) using the second reporter oligonucleotide and the nucleic acid barcode molecule of the plurality of nucleic acid barcode molecules to generate a second glycan barcoded nucleic acid molecule comprising the partition-specific barcode sequence or complement thereof and the second glycan motif-specific reporter barcode sequence or complement thereof; (b) determining the sequence of the second glycan barcoded nucleic acid molecules or derivatives thereof to identify (i) the partition-specific barcode sequences or complements thereof and (ii) the second glycan motif-specific reporter barcode sequence or complement thereof; and using the identified partition-specific barcode sequence or complement thereof and the identified second glycan motif-specific reporter barcode sequence or complement thereof to determine the presence and/or abundance of a first glycan and a second glycan in said sample.

In some embodiments, the method of determining the presence of one or more glycans in a sample further comprises: (a) incubating the sample with (i) a third flag molecule comprising a third nucleotide sugar and a first reactive molecule of a third reaction pair and (ii) a third glycan specific transferase, wherein the third flag molecule is incorporated onto a glycan-modified glycoprotein in the sample; and (b) admixing the sample with a third reporter molecule comprising (i) a second reactive molecule of a third reaction pair, and (ii) a third reporter oligonucleotide comprising a reporter barcode sequence that identifies a third glycan motif, wherein the third reporter molecule is conjugated on the glycan-modified glycoprotein via the third flag molecule. In one embodiment, the second reactive molecule is capable of coupling to the first reactive molecule of the third reaction pair.

In one embodiment, the method further comprises the steps of: (a) using the third reporter oligonucleotide and the nucleic acid barcode molecule of the plurality of nucleic acid barcode molecules to generate a third glycan barcoded nucleic acid molecule comprising the partition-specific barcode sequence or complement thereof and the third glycan motif-specific reporter barcode sequence or complement thereof; (b) determining the sequence of the third glycan barcoded nucleic acid molecules or derivatives thereof to identify (i) the partition-specific barcode sequence or complement thereof and (ii) the third glycan motif-specific reporter barcode sequence or complement thereof; and (c) using the identified partition-specific barcode sequence or complement thereof and the identified third glycan motif-specific reporter barcode sequence or complement thereof to determine the presence and/or abundance of a first glycan, a second glycan motif and a third glycan in the sample.

In some embodiments, the method of determining the presence of one or more glycans in a sample further comprises (a) incubating the sample with (i) a fourth flag molecule comprising a fourth nucleotide sugar and a first reactive molecule of a fourth reaction pair and (ii) a fourth glycan specific transferase; and (b) admixing the sample with a fourth reporter molecule comprising (i) a second reactive molecule of a fourth reaction pair, wherein the second reactive molecule is capable of coupling to the first reactive molecule of said fourth reaction pair, and (iii) a fourth reporter oligonucleotide comprising a reporter barcode sequence that identifies a fourth glycan motif. In one embodiments, the fourth flag molecule is incorporated onto a glycan-modified glycoprotein in the sample. In one embodiment, the fourth reporter molecule is conjugated on the glycan-modified glycoprotein via the fourth flag molecule.

In one embodiment, the method further comprises the steps of: (a) using the fourth reporter oligonucleotide and the nucleic acid barcode molecule of the plurality of nucleic acid barcode molecules to generate a fourth glycan barcoded nucleic acid molecule comprising the partition-specific barcode sequence or complement thereof and the fourth glycan motif-specific reporter barcode sequence or complement thereof; (b) determining the sequence of the fourth barcoded nucleic acid molecules or derivatives thereof to identify (i) the partition-specific barcode sequence or complement thereof and (ii) the fourth glycan motif-specific reporter barcode sequence or complement thereof; and (c) using the identified partition-specific barcode sequence or complement thereof and the identified fourth glycan motif-specific reporter barcode sequence or complement thereof to determine the presence and/or abundance of the first glycan, the second glycan, the third glycan and the fourth glycan in said sample.

In some embodiments, the sample is selected from the group consisting of a tissue, a cell, a fixed cell, a live cell, and cell lysates. In some embodiments, a plurality of partitions receive a single cell from the sample. In some embodiments, a lysate from a single cell is encapsulated in a cell bead, coated on a cell bead, embedded in a cell bead, or any combination thereof.

In some embodiments, the method of determining the presence of one or more glycans in a sample further comprising the steps of: (a) providing a plurality of cell beads comprising a cell and a reporter oligonucleotide sequence comprising a reporter barcode sequence that identifies a glycan motif; and (b) subjecting the plurality of cell beads to conditions sufficient to lyse the cells.

In some embodiments, the conditions sufficient to lyse the cells comprise contacting the cell beads with a lysis agent. In some embodiments, the first, the second, the third or the fourth glycan specific transferase is selected from the group consisting of a β1-4 galatosyltransferase, a glycosyltransferase, sialyltransferase, α1-3-fucosyl transferase; a human blood group A antigen glycosyltransferase (BgtA); WbwK fucosyltransferase; α1-2-fucosyl transferase; β1-4 N-acetyl-galactosylaminotransferase; β-galactoside α2-6 sialyltransferase 1; and β-galactoside α2-3 sialyltransferase 1; ST3Gal1; ST6Gal1; and CgtA. In some embodiments, the first, the second, the third or the fourth flag molecule is selected from the group consisting of UDP-GalNAc; GDP-fucose; UDP-GalNAc; and CMP-Sia. In some embodiments, the glycan-modified glycoprotein comprises a glycan selected from the group consisting of GlcNAc-O-R; LacNAc; Fucα1-2Gal; Galβ1-3GalNAc; Neu5Acα2-3Gal; N-glycans; and O-glycans.

In some embodiments, the first, second, third, or fourth glycan specific transferase is the β1-4 galatosyltransferase, the first, the second, the third or the fourth flag molecule is UDP-GalNAc; and the glycan on the glycan-modified glycoprotein comprises GlcNAc-O-R. In some embodiments, the first, second, third, or fourth glycan specific transferase is the α1-3-fucosyl transferase, the first, the second, the third or the fourth flag molecule is GDP-fucose; and the glycan on the glycan-modified glycoprotein comprises LacNAc. In some embodiments, the first, second, third, or fourth glycan specific transferase is the human blood group A antigen glycosyltransferase (BgtA), the first, second, the third or the fourth flag molecule is UDP-GalNAc; and the glycan on the glycan-modified glycoprotein comprises Fucα1-2Gal. In another embodiment, the first, second, third, or fourth glycan specific transferase is the α1-2-fucosyl transferase, the first, second, the third or the fourth flag molecule is GDP-fucose; and the glycan on the glycan-modified glycoprotein comprises Galβ1-3GalNAc. In yet another embodiment, the first, second, third, or fourth glycan specific transferase is β1-4 N-acetyl-galactosylaminotransferase, the first, the second, the third or the fourth flag molecule is UDP-GalNAc; and the glycan on the glycan-modified glycoprotein comprises Neu5Acα2-3Gal. In a further embodiment, the first, second, third, or fourth glycan specific transferase is β-galactoside α2-6 sialyltransferase 1, the first, the second, the third or the fourth flag molecule is CMP-Sia; and the glycan on the glycan-modified glycoprotein is the N-glycan. In another embodiment, the first, second, third, or fourth glycan specific transferase is β-galactoside α2-3 sialyltransferase 1, the second, the third or the fourth flag molecule is CMP-Sia; and the glycan on the glycan-modified glycoprotein is the O-glycan. In some embodiments, the first glycan specific transferase is β1-4 galatosyltransferase; the second glycan specific transferase is α1-3-fucosyl transferase; and the third glycan specific transferase is β-galactoside α2-3 sialyltransferase 1. In some embodiments, the first glycan specific transferase is specific for UDP-GalNAc; the second glycan specific transferase is specific for GDP-fucose; and the third glycan specific transferase is specific for CMP-Sia. In another embodiment, the first, second, third, or fourth glycan specific transferase is specific for GlcNAc-O-R; LacNAc; Fucα1-2Gal; Galβ1-3GalNAc; Neu5Aca2-3Gal; N-glycans; or O-glycans.

One aspect of the present disclosure provides a method of determining the presence of one or more glycans in a sample comprising one or more living cells, comprising the steps of: (a) incubating the sample with a flag molecule comprising a synthetic sugar and a first reactive molecule of a reaction pair, wherein (i) the flag molecule is a substrate for one or more glycosyltransferases of the one or more living cells, (ii) the flag molecule is incorporated and processed in one or more living cells of the sample to generate one or more substrates for one or more glycosyltransferases of the one or more living cells, and (iii) the flag molecule comprises the first reactive molecule; (b) admixing the sample with a reporter molecule comprising (i) a second reactive molecule of the reaction pair and (ii) a reporter oligonucleotide comprising a glycan motif-specific reporter barcode sequence, wherein the second reactive molecule of the reaction pair couples to the first reactive molecule of the reaction pair, whereby the reporter molecule is conjugated to the glycan-modified glycoprotein via the flag molecule; (c) removing unincorporated reporter molecules; (d) partitioning the sample and a plurality of nucleic acid barcode molecules into a plurality of partitions such that a partition of the plurality comprises a cell from the sample and a plurality of nucleic acid barcode molecules comprising a partition-specific barcode sequence; (e) using the reporter oligonucleotide and the nucleic acid barcode molecule to generate a glycan barcoded nucleic acid molecule comprising the partition-specific barcode sequence or complementary sequence thereof and the glycan motif-specific reporter barcode sequence or complementary sequence thereof; (f) determining the sequence of the first glycan barcoded nucleic acid molecule or derivative thereof to identify (i) the partition-specific barcode sequence or complementary sequence thereof and (ii) the first glycan motif-specific reporter barcode sequence or complementary sequence thereof; and (g) using the identified partition-specific barcode sequence or complementary sequence thereof and the identified first glycan motif-specific reporter barcode sequence or complementary sequence thereof to determine the presence and/or abundance of at least one glycan in the sample.

In some embodiments, the method of determining the presence of one or more glycans in a sample comprising one or more living cells further comprises: (a) determining a sequence of the glycan barcoded nucleic acid molecule or derivative thereof to identify (i) the partition-specific barcode sequence or complement thereof and (ii) the glycan motif-specific reporter barcode sequence or complement thereof. In some embodiment, the method further comprises (b) using the identified partition-specific barcode sequence or complement thereof and the identified glycan motif-specific reporter barcode sequence or complement thereof to determine the presence and/or abundance of at least one glycan in said sample.

In some embodiments, the method of determining the presence of one or more glycans in a sample comprising one or more living cells as disclosed herein further comprises the step of removing unincorporated flag molecules prior to incubating the sample with a reporter molecule comprising a second reactive molecule of the reaction pair.

In some embodiments, the one or more glycosyltransferases are endogenous or heterologous to the one or more living cells; and/or the synthetic sugar is acetylated. In some embodiments, incubating the sample with the flag molecule occurs for a duration selected from the group of ranges comprising about 1 second to about 30 seconds, about 30 seconds to about 1 minute, about 1 minute to about 10 minutes, about 5 minutes to about 60 minutes, and about 1 hour to about 12 hours.

In some embodiments, the one or more glycosyltransferases are endogenous or heterologous to the one or more living cells; and/or the synthetic sugar is acetylated. In some embodiments, incubating the sample with the flag molecule occurs for a duration selected from the group of ranges comprising 1 second to 30 seconds, 30 seconds to 1 minute, 1 minute to 10 minutes, 5 minutes to 60 minutes, and 1 hour to 12 hours.

In some embodiments of the method disclosed herein, the reaction pair is selected from the group of reaction pairs consisting of an azide and an alkyne, an azide and a phosphine, an aldehyde and aminooxy, an aldehyde and a hydrazine, an aldehyde and a hydrazide, a ketone and an aminooxy, a hydrazine and a ketone, cyclopropene and a tetrazine, norbornene and tetrazine, trans-cyclooctene and tetrazine, an alkyne and a tetrazine, a nitrone and an alkene, a nitrone and alkyne, diazo and alkyne, and isonitrile and tetrazine. In another embodiment, the second reactive molecule of a reaction pair is capable of coupling to the first reactive molecule of the reaction pair.

In some embodiments, the synthetic sugar is selected from the group consisting of galactose, sialic acid, fucose, mannose, N-acetylmannosamine and N-acetylgalactosamine; and/or the synthetic sugar. In another embodiment, the synthetic sugar is incorporated into a glycan selected from the group consisting of sialytated glycans; fucosylated glycans; cytosolic O-GlcNAcylated; and mucin type O-linked glycans. In yet another embodiment, the synthetic sugar is glycan class specific; and/or is glycan-motif specific.

One aspect of the present disclosure provides a method of detecting a protein-specific glycosylation pattern in a single cell, the method comprising (a) incubating a plurality of cells with a glycan-motif specific molecule comprising a reporter oligonucleotide conjugated to a glycan-motif specific reporter barcode sequence; (b) providing a component specific molecule comprising an oligonucleotide conjugated to a component specific barcode sequence; (c) removing unincorporated glycan specific molecules and unincorporated component specific molecules; (d) performing a ligation reaction; (e) partitioning the plurality of cells into a plurality of partitions such that a partition comprises (i) a single cell, single cell lysate, two adjacent cells or lysates of two adjacent cells from the sample and (ii) a plurality of nucleic acid barcode molecules comprising a partition-specific barcode sequence; and (f) amplifying the glycan-component reporter sequence and one of the plurality of nucleic acid barcode molecules to generate a first barcoded nucleic acid molecule comprising the glycan-component reporter sequence or complement thereof and the partition-specific barcode sequence or complement thereof, or derivatives thereof. In some embodiments, wherein the glycan-motif specific molecule binds to a glycan on a glycoprotein. In some embodiment, the component specific molecule binds to a glycoprotein. In one embodiment, the oligonucleotide of the glycan specific reporter barcode sequence is ligated to the oligonucleotide of the component specific barcode sequence to generate a ligated glycan-component reporter sequence; and the glycoprotein, the glycan-motif specific molecule and the component specific molecule form a complex.

In some embodiments, partitioning the plurality of cells in the plurality of partitions occurs before or after performing the ligation reaction. In some embodiment, the ligated glycan-component reporter sequence is generated when: (i) the glycan-motif specific molecule and the component specific molecule bind to the same glycoprotein; or (ii) the glycan-motif specific molecule and the component specific molecule bind different glycoproteins and the glycoproteins are in closed proximity. In some embodiments, method of detecting a protein-specific glycosylation pattern in a single cell further comprises (a) determining the sequence of the first barcoded nucleic acid molecule or derivatives thereof to identify (i) the partition-specific barcode sequence or complement thereof and (ii) the glycan-component reporter barcode sequence or complement thereof; and/or (b) using the identified partition-specific barcode sequence or complement thereof and the identified glycan-component reporter barcode sequence or complement thereof to identify the glycan and the glycosylated pattern of the protein

**In** one embodiment, the ligation is performed in the presence of a splint oligonucleotide. In another embodiment, the ligation is performed in the presence of a splint oligonucleotide, and further wherein said splint is selected from the group consisting of a triazole and a nucleotide barcode splint. In another embodiment, the glycan specific molecule is selected from the group consisting of: (a) a glycan specific lectin; (b) a glycan specific antibody; (c) a synthetic nucleotide sugar; (d) a synthetic sugar; and (e) an inactivated glycan specific transferase.

In some embodiment, the method of detecting a protein-specific glycosylation pattern in a single cell, further comprises providing a glycan specific transferase.

In some embodiments, the component specific molecule is selected from the group consisting of antibodies, lectins, synthetic nucleotide sugars, nucleotide sugars, and synthetic sugars; and/or the component of interest is a protein, glycan, sugar, nucleotide sugar or synthetic nucleotide sugar.

In some embodiments, the glycan or the glycan motif is selected from the group consisting of O-GlcNAc residues, LacNac-containing glycans, Fuc-α1,2-Gal containing glycans, GalB1-3GalNAc containing glycans, GlcNAc-O-R, Neu5Acα2-3 Gal, and GalT1Y289L. In some embodiments, the partition is a droplet; and/or a well. In some embodiments, at least a subset of the plurality of nucleic acid barcode molecules are releasably attached to a gel bead. In some embodiments, at least a subset of the plurality of nucleic acid barcode molecules are releasably attached to a gel bead and further comprising releasing the nucleic acid barcode molecules from the gel bead prior to generating the barcoded nucleic acid molecule.

In some embodiments of any of the method disclosed herein, the plurality of nucleic acid barcode molecules in a partition further comprise a unique molecular identifier (UMI) sequence; and/or the UMI sequence of a nucleic acid barcode molecule in a partition differs from the UMI sequence of another nucleic acid barcode molecule in the partition. In some embodiments, the plurality of nucleic acid barcode molecules in a partition further comprise a functional sequence; and/or the plurality of nucleic acid barcode molecules in a partition further comprise a capture sequence; and/or the plurality of nucleic acid barcode molecules in a partition further comprise a capture sequence, and the capture sequence comprises: a template switch oligonucleotide (TSO) sequence; or a polyT sequence. In some embodiments, the plurality of nucleic acid barcode molecules in a partition further comprise a capture sequence and the capture sequence comprises a polyT sequence; and the second component is selected from the group consisting of a DNA analyte, RNA analyte, a protein analyte, a cell feature, a cell surface feature, and a metabolite.

In some embodiments, the partition further comprises a plurality of additional nucleic acid barcode molecules comprising the partition-specific barcode sequence and a capture sequence capable of binding to a second component of the single cell or single cell lysate or to a sequence of an additional reporter molecule that binds to the second component. In some embodiment, the second component is not a glycan or glycan motif; and/or the additional reporter molecule is configured to couple to a protein or to a metabolite; and/or the additional reporter molecule is configured to couple to a protein or to a metabolite and further wherein the additional reporter molecule comprises an additional reporter oligonucleotide comprising a different reporter barcode sequence that identifies the glycoprotein; and/or the additional reporter molecule is configured to couple to a protein or to a metabolite and further wherein the additional reporter molecule comprises an additional reporter oligonucleotide comprising a different reporter barcode sequence that identifies the glycan.

In some embodiments, the reporter oligonucleotide further comprises a capture handle comprising a sequence complementary to the capture sequence. In some embodiments, the capture handle comprises a sequence complementary to a template switching oligonucleotide (TSO) sequence; or the capture handle comprises a sequence complementary to a polyT sequence. In some embodiments of any of the method disclosed herein, the glycan is selected from the group consisting of O-linked glycans, N-linked glycans, mucin type O-linked glycans, O-linked glycans core 1, and O-linked glycans core 2.

The methods provide for determining the presence of one or more glycans in a sample, determining the presence of one or more glycans in a sample comprising one or more living cells, and/or detecting the proximity of a glycan to another component. In addition, the methods provide for determining the presence of one or more glycan motifs in a sample, determining the presence of one or more glycan motifs in a sample comprising one or more living cells, and/or detecting the proximity of a glycan motif to another component. Certain methods also allow determination of the prevalence of a glycan component or understanding of glycome changes over time.

Methods of determining the presence of one or more glycans or glycan motifs in a sample are provided. In one embodiment, the methods comprise the steps of (a) incubating the sample with (i) a flag molecule comprising a nucleotide sugar and a first reactive molecule of a reaction pair and (ii) a first glycan specific transferase; (b) incubating the sample with a reporter molecule comprising (i) a second reactive molecule of a reaction pair and (ii) a reporter oligonucleotide comprising a glycan-motif specific reporter barcode sequence; (c) removing unincorporated reporter molecules from the sample; (d) partitioning the sample into a plurality of partitions such that a partition comprises (i) a single cell or single cell lysate from the sample and (ii) a plurality of nucleic acid barcode molecules comprising a partition-specific barcode sequence; and (e) using the reporter oligonucleotide and a nucleic acid barcode molecule of the plurality of nucleic acid barcode molecules to generate a first barcoded nucleic acid molecule comprising the partition-specific sequence or complement thereof and the reporter barcode sequence or complement thereof.

In various aspects, the method may further comprise determining the sequence of the first barcoded nucleic acid molecule or derivative thereof to identify (i) the partition-specific barcode sequence or complement thereof and (ii) the reporter barcode sequence or complement thereof. Any of the methods may further comprise using the identified partition-specific barcode sequence or complement thereof and the identified reporter barcode sequence or complement thereof to determine the presence and/or abundance of at least one glycan or glycan motif in the cell.

In various aspects of the methods, the methods may further comprise (a) incubating the sample with a (i) second flag molecule comprising a second nucleotide sugar and a first reactive molecule of a second reaction pair and (ii) a second glycan specific transferase and (b) incubating the sample with a second reporter molecule comprising (i) a second reactive molecule of a second reaction pair, wherein the second reactive molecule is capable of coupling to the first reactive molecule of the second reaction pair, and (ii) a second reporter oligonucleotide comprising a reporter barcode sequence that identifies a second glycan motif. The methods may further comprise the steps of determining the sequence of a plurality of barcoded nucleic acid molecules or derivatives thereof to identify the partition specific barcode sequence or complement thereof and the reporter barcode sequence or complement thereof and using the identified partition-specific barcode sequence or complement thereof and the identified reporter barcode sequence or complement thereof to determine the presence and/or abundance of a first glycan and second glycan in the cell.

In other aspects, the methods may further comprise (a) incubating the sample with (i) a third flag molecule comprising a third nucleotide sugar and a first reactive molecule of a third reaction pair and (ii) a third glycan specific transferase and (b) incubating the sample with a third reporter molecule comprising (i) a second reactive molecule of a third reaction pair, wherein the second reactive molecule is capable of coupling to the first reactive molecule of the third reaction pair and (ii) a third reporter oligonucleotide comprising a reporter barcode that identifies a third glycan motif. In another aspect, the methods may further comprise the steps of (a) determining the presence of a plurality of barcoded nucleic acid molecules or derivative thereof to identify (i) the partition-specific barcode sequence or complement thereof and (ii) the reporter barcode sequence or complement thereof and (b) using the identified partition-specific barcode sequence or complement thereof and the identified reporter barcode sequence or complement thereof to determine the presence and/or abundance of a first glycan, a second glycan and/or a third glycan.

In another aspect, the methods may further comprise incubating the sample with (i) a fourth flag molecule comprising a fourth nucleotide sugar and a first reactive molecule of a fourth reaction pair and (ii) a fourth glycan specific transferase and incubating the sample with a fourth reporter molecule comprising a second reactive molecule of a fourth reaction pair, wherein the second reactive molecule is capable of coupling to the first reactive molecule of the fourth reaction pair; and (iii) a fourth reporter oligonucleotide comprising a reporter barcode sequence that identifies a fourth glycan motif. Aspects of the method may further comprise the steps of (a) determining the sequence of a plurality of barcoded nucleic acid molecules or derivative thereof to identify (i) the partition-specific barcode sequence or complement thereof and (ii) the reporter barcode sequence or complement thereof and (b) using the identified partition-specific barcode sequence or complement thereof to determine the presence and/or abundance of a first glycan, a second glycan, a third glycan and a fourth glycan in the cell.

In the various methods, the sample can be selected from the group consisting of tissues, cells, fixed cells, live cells and cell lysates. In any of the various methods, a plurality of partitions receive a single cell from the sample. In other aspects of the methods, a lysate from a single cell is encapsulated in a cell bead, coated on a cell bead, or a combination thereof. Aspects of the methods may further comprise providing a plurality of cell beads comprising a cell and a reporter oligonucleotide sequence comprising a reporter barcode sequence that identifies a glycan motif and subjecting said plurality of cell beads to conditions sufficient to lyse the cells. In some aspects, the conditions sufficient to lyse the cells comprise contacting the cell beads with a lysis agent.

In any of the methods, the glycan specific transferase may be selected from the group consisting of glycosyltransferase, sialyltransferase, α1,3-fucosyl transferase; BGTA; WbwK fucosyltransferase; α1,2-fucosyl transferase; β1-4 N-acetyl-galactosylaminotransferase; β-galactoside α2,6 sialyltransferase 1; and β-galactoside α2,3 sialyltransferase 1.

Various methods further comprise the steps of determining the sequence of a plurality of barcoded nucleic acid molecules or derivatives thereof to identify (i) the partition-specific barcode sequences or complements thereof and (ii) the reporter barcode sequence or complement thereof and using the identified partition-specific barcode sequence or complement thereof and the identified reporter barcode sequence or complement thereof to determine the presence and/or abundance of a first glycan motif and a second glycan motif in the cell. Methods may comprise the steps of determining the sequence of a plurality of barcoded nucleic acid molecules or derivatives thereof to identify (i) the partition-specific barcode sequences or complements thereof and (ii) the reporter barcode sequence or complement thereof and using the identified partition-specific barcode sequence or complement thereof and the identified reporter barcode sequence or complement thereof to determine the presence and/or abundance of a first glycan motif, a second glycan motif and a third glycan motif in the cell. The methods may comprise the steps of determining the sequence of a plurality of barcoded nucleic acid molecules or derivatives thereof to identify (i) the partition-specific barcode sequences or complements thereof and (ii) the reporter barcode sequence or complement thereof and using the identified partition-specific barcode sequence or complement thereof and the identified reporter barcode sequence or complement thereof to determine the presence and/or abundance of a first glycan motif, a second glycan motif, third glycan motif and a fourth glycan motif in the cell.

In an embodiment, methods of determining the presence of one or more glycans in a sample comprising one or more living cells are provided. The methods comprise the steps of (a) incubating a sample comprising one or more living cells with a flag molecule comprising a synthetic sugar and a first reactive molecule of a reaction pair, wherein the flag molecule is a flag substrate for one or more glycosyltransferases of the one or more living cells; (b) incubating the sample with a reporter molecule comprising (i) a second reactive molecule of the reaction pair and (ii) a reporter oligonucleotide comprising a glycan motif-specific reporter barcode sequence; (c) removing unincorporated reporter molecules; (d) partitioning the sample and a plurality of nucleic acid barcode molecules into a plurality of partitions such that a partition of the plurality comprises a cell from the sample and a plurality of nucleic acid barcode molecules comprising a partition-specific barcodes sequence; and (e) using the reporter oligonucleotide and the nucleic acid barcode molecule to generate a barcoded nucleic acid molecule comprising the partition-specific barcode sequence or complement thereof and the glycan-motif specific reporter barcode sequence or complement thereof. In various aspects of the method the flag molecule is incorporated and processed in one or more living cells of the sample to generate one or more flag substrates for one or more glycosyltransferases of the one or more living cells and wherein the flag substrate comprises the first reactive molecule. Aspects of the method further comprise determining a sequence of a barcoded nucleic acid molecule or derivative thereof to identify (i) the partition-specific barcode sequence or complement thereof and (ii) the glycan motif-specific reporter barcode sequence or complement thereof. Aspects of the methods may further comprise using the identified partition-specific barcode sequence or complement thereof and the identified glycan motif-specific reporter barcode sequence or complement thereof to determine the presence and/or abundance of at least one glycan motif in the sample. The methods may comprise the step of removing unincorporated flag molecules prior to incubating the sample with a reporter molecule comprising a second reactive molecule of the reaction pair.

In the aspects of the methods, the one or more glycosyltransferase is endogenous to the one or more living cells. In some aspects of the methods, the synthetic sugar is acetylated. In certain aspects of the methods, the step of incubating the sample with a flag molecule occurs for a duration selected from the group of ranges comprising about 1 second to about 30 seconds, about 30 seconds to about 1 minute, about 1 minute to about 10 minutes, about 5 minutes to about 60 minutes and about 1 hour to about 12 hours.

In the aspects of the methods, the one or more glycosyltransferase is endogenous to the one or more living cells. In some aspects of the methods, the synthetic sugar is acetylated. In certain aspects of the methods, the step of incubating the sample with a flag molecule occurs for a duration selected from the group of ranges comprising 1 second to 30 seconds, 30 seconds to 1 minute, 1 minute to 10 minutes, 5 minutes to 60 minutes and 1 hour to 12 hours.

In any of the methods, the reaction pair may be selected from the group of reaction pairs comprising an azide and an alkyne, an azide and a phosphine, an aldehyde and aminooxy, an aldehyde and a hydrazine, an aldehyde and a hydrazide, a ketone and an aminooxy, a hydrazine and a ketone, cyclopropene and a tetrazine, norbornene and tetrazine, trans-cyclooctene and tetrazine, an alkyne and a tetrazine, a nitrone and an alkene, a nitrone and alkyne, diazo and alkyne, and isonitrile and tetrazine. In aspects of the methods, the second reactive molecule of reaction pair is capable of coupling to the first reactive molecule of the reaction pair.

In various aspects of the methods, the synthetic sugar is selected from the group consisting of N-acetylmannosamine and N-acetylgalactosamine. In some aspects of the methods, the synthetic sugar is incorporated into a glycan selected from the group consisting of sialic acid and mucin type O-linked glycans. In aspects of the methods, the synthetic sugar is glycan class specific.

In an embodiment, methods of detecting proximity of a glycan to another component are provided. The methods comprise the steps of (a) incubating a sample with a glycan-motif specific molecule comprising a reporter oligonucleotide comprising a glycan-motif specific reporter barcode sequence; (b) providing a component specific molecule comprising an oligonucleotide comprising a component specific barcode sequence; (c) removing unincorporated glycan specific molecules and unincorporated component specific molecules; (d) providing a splint and performing a ligation reaction with the oligonucleotide comprising a glycan specific reporter barcode sequence and the oligonucleotide comprising a component specific barcode sequence to generate a glycan-component reporter sequence; (d) partitioning the sample into a plurality of partitions such that a partition comprises (i) a single cell, single cell lysate, two adjacent cells or lysates of two adjacent cells from the sample and (ii) a plurality of nucleic acid barcode molecules comprising a partition-specific barcode sequence; and (e) using the glycan-component reporter sequence and a nucleic acid barcode molecule to generate a barcoded molecule comprising the glycan-component reporter sequence or complement thereof and the partition-specific barcode sequence or complement thereof. In aspects of the method, the ligation is performed in the presence of a splint oligonucleotide. In aspects of the method, partitioning the sample in a plurality of partitions occurs before performing the ligation reaction. In aspects of the method, partitioning the sample in a plurality of partitions occurs after performing the ligation reaction. Aspects of the method may further comprise determining the sequence of the first barcoded nucleic acid molecule or derivative thereof to identify (i) the partition-specific barcode sequence or complement thereof and (ii) the glycan-component reporter barcode sequence or complement thereof. The methods may further comprise using the identified partition-specific barcode sequence or complement thereof and the identified glycan-component reporter barcode sequence or complement thereof to determine the presence and or abundance of a glycan in proximity to the component of interest.

In various aspects, the glycan specific molecule is selected from the group consisting of a glycan specific lectin, a glycan specific antibody, a synthetic nucleotide sugar, a synthetic sugar and an inactivated glycan specific transferase. Aspects of the method may further comprise providing a glycan specific transferase. In aspects of the method, the component specific molecule is selected from the group consisting of antibodies, lectins, synthetic nucleotide sugars, nucleotide sugars and synthetic sugars. In various aspects, the component of interest is a protein, glycan, sugar, nucleotide sugar or synthetic nucleotide sugar. In aspects of the method, the splint is selected from the group consisting of a triazole or a nucleotide barcode splint.

In any of the methods, the glycan or glycan motif can be selected from the group consisting of O-GlcNAc residues, LacNac-containing glycans, Fuc-α1,2-Gal containing glycans, Galβ1-3GalNAc containing glycans, GlcNAc-OR, Neu5Acα2-3 Gal and GalT1Y289L.

In any aspect of the methods, the partition is a droplet. In any aspect of the methods, the partition is a well. In any aspect of the methods at least a subset of the plurality of nucleic acid barcode molecules are releasably attached to a gel bead. The methods may comprise releasing the nucleic acid barcode molecules from the gel bead prior to generating the barcoded nucleic acid molecule. In any aspect of the methods, the plurality of nucleic acid barcode molecules in a partition further comprise a unique molecular identifier sequence (UMI). In aspects, the UMI sequence of a nucleic acid barcode molecule in a partition differs from the UMI sequence of another nucleic acid barcode molecule in the partition. In aspects of the methods, the plurality of nucleic acid barcode molecules in a partition further comprise a functional sequence. In any aspect of the methods, the plurality of nucleic acid barcode molecules in a partition further comprise a capture sequence. In various aspects, the capture sequence comprises a template switch oligonucleotide sequence. In aspects, the capture sequence comprises a polyT sequence.

In any aspect, the partition can further comprise a plurality of additional nucleic acid barcode molecules comprising the partition-specific barcode sequence and a binding sequence capable of binding to a second analyte of the single cell or single cell lysate. In various aspects, the second analyte is not a glycan or glycan motif. In various aspects, the second analyte is selected from the group consisting of a DNA analyte, RNA analyte, an additional reporter molecule configured to couple to a protein and an additional reporter molecule configured to couple to a metabolite. In various aspects, the additional reporter molecule comprises an additional reporter oligonucleotide comprising a different reporter barcode sequence that identifies a protein of interest. In various aspects, the additional reporter molecule comprises an additional reporter oligonucleotide comprising a different reporter barcode sequence that identifies the metabolite of interest. In other aspects, the reporter oligonucleotide further comprises a capture handle comprising sequence complementary to the capture sequence. In some aspects, the capture sequence comprises a sequence complementary to a template switching oligonucleotide (TSO) sequence. In certain aspects, the capture sequence comprises a sequence complementary to a polyT sequence.

In any aspect the glycan class can be selected from the group consisting of O-linked glycans, N-linked glycans, mucin type O-linked glycans, O-linked glycans core 1 and O-linked glycans core 2.

Another aspect of the present disclosure provides a use of a method disclosed herein for screening, diagnosing, or staging a subject at risk of, or suffering from, a disease or condition related to abnormal glycosylation patterns or motifs. In some embodiments, the disease or condition is selected from the group consisting of neurodegenerative diseases or conditions, cancer, congenital disorders of glycosylation, and inflammatory conditions.

Another aspect of the present disclosure provides a composition for determining the presence of one or more glycans in a sample comprising one or more living cells comprising: (i) a plurality of flag molecules, each comprising a nucleotide sugar and a first reactive molecule of a reaction pair, (ii) a plurality of glycan specific transferases, and (iii) a plurality of reporter molecules, each comprising a second reactive molecule of a reaction pair and a reporter oligonucleotide comprising a glycan motif-specific reporter barcode sequence. the present disclosure provides a composition for determining the presence of one or more glycans in a sample comprising a plurality of flag molecules, each comprising a synthetic sugar and a first reactive molecule of a reaction pair, (ii) a plurality of reporter molecules, each comprising a second reactive molecule of the reaction pair and a reporter oligonucleotide comprising a glycan motif-specific reporter barcode sequence. In some embodiments, the flag molecule is a flag substrate for one or more glycosyltransferases of the one or more living cells.

Another aspect of the present disclosure provides a composition of detecting a protein-specific glycosylation pattern in a single cell comprising (i) a plurality of glycan-motif specific molecules, each comprising a reporter oligonucleotide conjugated to a glycan-motif specific reporter barcode sequence, (ii) a plurality of component specific molecules, each comprising an oligonucleotide conjugated to a component specific barcode sequence, wherein the component specific molecule binds to a glycoprotein; and optionally (iii) a splint oligonucleotide. In some embodiments, the glycan-motif specific molecule binds to a glycan on a glycoprotein.

Another aspect of the present disclosure provides a kit for determining the presence of one or more glycans in a sample comprising: (i) a plurality of flag molecules, each comprising a nucleotide sugar and a first reactive molecule of a reaction pair, (ii) a plurality of glycan specific transferases, (iii) a plurality of reporter molecules, each comprising a second reactive molecule of a reaction pair and a reporter oligonucleotide comprising a glycan motif-specific reporter barcode sequence, and (iv) instructions for performing a method for determining the presence of one or more glycans as disclosed herein.

Another aspect of the present disclosure provides a kit for determining the presence of one or more glycans in a sample comprising one or more living cells comprising: (i) a plurality of flag molecules, each comprising a synthetic sugar and a first reactive molecule of a reaction pair, (ii) a plurality of reporter molecules, each comprising a second reactive molecule of the reaction pair and a reporter oligonucleotide comprising a glycan motif-specific reporter barcode sequence, and (iii) instructions for performing a method for determining the presence of one or more glycans in a sample comprising one or more living cells as disclosed herein. In some embodiments, wherein the flag molecule is a flag substrate for one or more glycosyltransferases of the one or more living cells.

Another aspect of the present disclosure provides a kit of detecting a protein-specific glycosylation pattern in a single cell comprising (i) a plurality of glycan-motif specific molecules, each comprising a reporter oligonucleotide conjugated to a glycan-motif specific reporter barcode sequence, (ii) a plurality of component specific molecules, each comprising an oligonucleotide conjugated to a component specific barcode sequence, wherein the component specific molecule binds to a glycoprotein; and optionally (iii) a splint oligonucleotide, and (iv) instructions for performing a method of detecting a protein-specific glycosylation pattern in a single cell as disclosed herein. In some embodiments, the glycan-motif specific molecule binds to a glycan on a glycoprotein.

One aspect of the present invention provides a system, comprising: (a) (i) a plurality of flag molecules, each comprising a nucleotide sugar and a first reactive molecule of a reaction pair, (ii) a plurality of glycan specific transferases, and (iii) a plurality of reporter molecules, each comprising a second reactive molecule of a reaction pair and a reporter oligonucleotide comprising a glycan motif-specific reporter barcode sequence; (b) a plurality of nucleic acid barcode molecules. In some embodiments, a first nucleic acid barcode molecule of the plurality of nucleic acid barcode molecules comprises a partition barcode sequence. In some embodiments, the plurality of nucleic acid barcode molecules is attached to a bead, and the partition barcode sequence identifies the bead. In some embodiments, the system further comprises a plurality of partitions, optionally the plurality of partitions comprises a plurality of droplets and/or a plurality of wells. In some embodiments, the system further comprises an apparatus comprising a microfluidic channel structure configured to generate a plurality of partitions, optionally the apparatus comprises (i) a first channel in fluid communication with a first source comprising a plurality of cells labeled with a glycan or a glycan-specific molecule using the method for determining the presence of one or more glycans in a sample as disclosed herein, (ii) a second channel in fluid communication with a second source comprising the plurality of nucleic acid barcode molecules, and (iii) a junction that brings a first phase comprising said plurality of cells from the first channel and the plurality of nucleic acid barcode molecules from said second channel in contact with a second phase that is immiscible with said first phase, to yield a plurality of droplets comprising the plurality of cells and said plurality of nucleic acid barcode molecules. In some embodiments, a droplet of said plurality of droplets comprises said cell and said barcode bead.

Another aspect of the present invention provides a system, comprising: (a) (i) a plurality of flag molecules, each comprising a synthetic sugar and a first reactive molecule of a reaction pair, wherein the flag molecule is a flag substrate for one or more glycosyltransferases of the one or more living cells, (ii) a plurality of reporter molecules, each comprising a second reactive molecule of the reaction pair and a reporter oligonucleotide comprising a glycan motif-specific reporter barcode sequence; (b) a plurality of nucleic acid barcode molecules. In some embodiments, a first nucleic acid barcode molecule of the plurality of nucleic acid barcode molecules comprises a partition barcode sequence. In some embodiments, the plurality of nucleic acid barcode molecules is attached to a bead, and the partition barcode sequence identifies the bead. In some embodiments, the system further comprising a plurality of partitions, optionally wherein the plurality of partitions comprises a plurality of droplets and/or a plurality of wells.

In some embodiments, the system further comprises an apparatus comprising a microfluidic channel structure configured to generate a plurality of partitions, optionally the apparatus comprises (i) a first channel in fluid communication with a first source comprising a plurality of cells labeled with a glycan or a glycan-specific molecule using the method for determining the presence of one or more glycans in a sample comprising one or more living cells, (ii) a second channel in fluid communication with a second source comprising the plurality of nucleic acid barcode molecules, and (iii) a junction that brings a first phase comprising said plurality of cells from the first channel and the plurality of nucleic acid barcode molecules from said second channel in contact with a second phase that is immiscible with said first phase, to yield a plurality of droplets comprising the plurality of cells and said plurality of nucleic acid barcode molecules. In some embodiments, a droplet of said plurality of droplets comprises said cell and said barcode bead.

One aspect of the present invention provides a system, comprising: (a) (i) a plurality of glycan-motif specific molecules, each comprising a reporter oligonucleotide conjugated to a glycan-motif specific reporter barcode sequence, (ii) a plurality of component specific molecules, each comprising an oligonucleotide conjugated to a component specific barcode sequence, wherein the component specific molecule binds to a glycoprotein; and optionally (iii) a splint oligonucleotide; (b) a plurality of nucleic acid barcode molecules. In some embodiments, the glycan-motif specific molecule binds to a glycan on a glycoprotein. In some embodiments, a first nucleic acid barcode molecule of the plurality of nucleic acid barcode molecules comprises a partition barcode sequence. In some embodiments, the plurality of nucleic acid barcode molecules is attached to a bead, and wherein the partition barcode sequence identifies the bead. In some embodiments, the system further comprising a plurality of partitions, optionally the plurality of partitions comprises a plurality of droplets and/or a plurality of wells. In some embodiments, the system further comprises an apparatus comprising a microfluidic channel structure configured to generate a plurality of partitions, optionally the apparatus comprises (i) a first channel in fluid communication with a first source comprising a plurality of cells labeled with a glycan or a glycan-specific molecule using the method of detecting a protein-specific glycosylation pattern in a single cell as disclosed herein (ii) a second channel in fluid communication with a second source comprising the plurality of nucleic acid barcode molecules, and (iii) a junction that brings a first phase comprising said plurality of cells from the first channel and the plurality of nucleic acid barcode molecules from said second channel in contact with a second phase that is immiscible with said first phase, to yield a plurality of droplets comprising the plurality of cells and said plurality of nucleic acid barcode molecules. In some embodiments, a droplet of said plurality of droplets comprises said cell and said barcode bead.

In some embodiments of the systems provided herein, the cell is encapsulated in a cell bead, coated on a bead cell, embedded in a bead cell, or any combination thereof. In some embodiments, the system provided herein further comprises a third channel in fluid communication with a third source comprising additional reagents. In one embodiment, the first phase comprises additional reagents. In some embodiments, the system provided herein further comprises a fourth channel in fluid communication with a fourth source comprising additional reagents. In one embodiment, the first phase comprises said additional reagents. In some embodiments, the additional reagents are reagents for nucleic acid amplification, or reagents that: (i) can degrade or dissolve cells, cell beads and/or barcode beads, (ii) degrade linkages between barcodes and barcode beads, or any combination thereof.

Both the foregoing summary and the following description of the drawings and detailed description are exemplary and explanatory. They are intended to provide further details of the disclosure, but are not to be construed as limiting. Other objects, advantages, and novel features will be readily apparent to those skilled in the art from the following detailed description of the disclosure.

### BRIEF DESCRIPTION OF THE DRAWINGS

**FIG. 1** shows an example of a microfluidic channel structure for partitioning individual biological particles.
**FIG. 2** shows an example of a microfluidic channel structure for the controlled partitioning of beads into discrete droplets.
**FIG. 3** illustrates an example of a barcode carrying bead.
**FIG. 4** illustrates another example of a barcode carrying bead.
**FIG. 5** schematically illustrates an example microwell array.
**FIG. 6** schematically illustrates an example workflow for processing nucleic acid molecules.
**FIG. 7** schematically illustrates example labelling agents with nucleic acid molecules attached thereto.
**FIG. 8A** schematically shows an example of labelling agents. **FIG. 8B** schematically shows another example workflow for processing nucleic acid molecules. **FIG. 8C** schematically shows another example workflow for processing nucleic acid molecules.
**FIG. 9** schematically shows another example of a barcode-carrying bead.

### DETAILED DESCRIPTION

The following passages describe different aspects of the invention in greater detail. Each aspect, embodiment, or feature of the invention may be combined with any other aspect, embodiment, or feature the invention unless clearly indicated to the contrary.

### I. OVERVIEW

The last two decades of glycobiology work has firmly established the importance of glycans in biological processes, including immunology, oncology, development, protein folding, and more. Glycans (i.e., carbohydrates or polysaccharides) are an important class of biomolecules that play critical roles in biological processes such as protein trafficking, cell-cell communication and immune responses. Glycan analysis (e.g., quality control, disease diagnosis, etc.) is of great value in academic research, pharmaceutical industry and healthcare because: (1) aberrant glycosylation (e.g. glycans abnormality) is associated with numerous diseases such as cancer, dementia, and autoimmune disorders; and (2) the potency and stability of many biologics, such as monoclonal antibodies, are impacted by their associated glycans.

Protein glycosylation mediates a diversity of cellular processes. Glycoproteins on the exterior of the cell impact processes such as cell recognition, cell signaling and cell adhesion. Abnormal glycosylation or glycosylation patterns, such as under-expression and over-expression of naturally-occurring glycans or neo-expression of glycans normally restricted to embryonic tissues, are considered hallmarks of many diseases or disease stages, including microbial pathogenesis, immune deficiencies, neurodegenerative disease, and many cancers. Glycans play a critical role in many biological processes including, but not limited to, immunology, oncology, development, protein folding and signal transduction. Unlike the biological molecules whose production is blueprinted (for example, DNA or amino acid sequences), glycans are highly variable and complex. The glycome contains substantial information about the state of a cell and the cell's response to its environment or other stimuli. In addition, understanding the glycome status and alterations to the glycome could provide insights into these diseases. Given the importance of glycans in health and disease, the development of methods and tools for the analysis and characterization of glycans as well as glycan-containing proteins is a top priority for those in the field of glycobiology.

Numerous techniques have been developed to analyze cellular glycosylation. However, current techniques for analyzing glycans are inherently complex, and have significant disadvantages. Conventional detections suffer from a lack of specificity and sensitivity. In addition, many of the methods used in the art to analyze glycosylation are destructive methods and cannot be used in functional biological assay systems. Furthermore, existing multimodal single-cell sequencing technologies, that enable the in-depth characterization of heterogeneous cell populations and facilitate the identification of unique cell differentiation states within a complex cellular pool, do not provide a comprehensive snapshot of the cell glycosylation state or glycome because they do not incorporate essential posttranslational information that can provide important insights into a cell's glycome.

The complexity of glycosylation arises from numerous factors, which make single cell assays to identify glycans surprising and unexpected. For example, unlike DNA transcription and RNA translation, glycosylation is not a template-driven process. Rather glycosylation is a posttranslation modification. Glycoproteins and glycolipids are generated through the enzymatic addition of monosaccharides and complex oligosaccharides onto protein or lipid scaffolds in the secretory pathway (e.g., golgi). Glycosylation can produce significant diversity and heterogeneity. Indeed, there is a diversity of monosaccharide building blocks used in glycosylation, and there are multiple ways in which these monossacharides can be assembled to form oligosaccharides within the cell.

In particular, conventional detections of cell and tissue glycans rely almost entirely on lectins and antibodies that target specific classes of glycoproteins. Lectins are glycan-binding proteins isolated from various plants, bacteria, and animal sources and have played an important role in the isolation, purification, and characterization of glycoproteins. While useful for enriching N-linked glycoproteins by binding to a conserved pentasaccharide core structure, lectins lack specificity and are often considered to be promiscuous. Further, the detection of glycans using antibodies is also challenging because glycan-binding antibodies are difficult to raise and often have low affinity for the target glycan. Therefore, the utility of lectins and antibodies for glycoprotein and glycopeptide enrichment is limited by low substrate binding affinities and/or poor specificities. Additionally, technologies such as mass spectrometry; hydrophilic interaction liquid chromatography (HILIC); NMR spectroscopy, liquid chromatography, electrophoresis; and glycan or lectin microarray technologies, which are currently used to analyze glycosylation are destructive methods and cannot be used in functional biological assay systems. Furthermore, existing multimodal single-cell sequencing technologies, that enable the in-depth characterization of heterogeneous cell populations and facilitate the identification of unique cell differentiation states within a complex cellular pool, do not provide a comprehensive snapshot of the cell glycosylation state or glycome because they do not incorporate essential posttranslational information that can provide important insights into a cell's glycome.

### Exemplary Advantages Provided by the Disclosure

Glycosylation is an important and highly regulated mechanism of secondary protein processing within cells. It plays a critical role in determining protein structure, function and stability. Changes in these complexes result in alterations in how they recruit, interact and activate signaling proteins. Thus, the ability to precisely identify glycosylation, as well as glycan motifs, in a single cell is highly desirable. As described herein, the novel methods enable the determination of different glycans, glycan motifs and/or glycan classes which may further be used to elucidate a cell's glycome. In addition, the methods may be used to determine the abundance of at least one glycan or glycan motif. Moreover, the described methods may be used to identify glycans in close proximity to each other. The methods may also be used to evaluate protein-specific glycosylation. Without being limited by mechanism, the glycan-motif specific molecule interacts with the target glycan motif and the component specific molecule interacts with the component of interest. In another example, the described methods can be used to evaluate glycans and gene expression, changes in the glycome related to gene expression and the relationship between the glycome and gene expression. The analysis may be used to evaluate changes to the glycome throughout a tissue or collection of cells in conjunction with changes to the transcriptome.

The single cell profiling analysis will provide the glycome profile of each cell. In addition, the single cell profiling will provide the abundance of each type of glycan on the cell surface based on a sequenceable readout at the single cell level. Furthermore, it is expected that the results will identify and distinguish subsets of cells with unique glycosylation profiles. *See* e.g., Example 2.

As described in Example 3, protein glycosylation is one of the most ubiquitous modifications of eukaryotic proteins as it is estimated that over 50% of all eukaryotic proteins are glycosylated. Two main forms of protein glycosylation pattern exists: N-linked and O-linked glycosylation. The glycosylation pattern of the protein is dependent on the tissue of origin of that protein and reflects the status of the cell. In addition, aberrant glycosylation has been associated with several illnesses including cancer development and progression. Glycosylation also affects the biological activities of numerous proteins. For example, the activity of many antibodies is dependent on the glycosylation pattern of the antibody. O-glycan alteration, such as the expression of the truncated O-glycan epitope sialyl Tn (STn) is involved in cancer development and progression. In addition, the activity of cell adhesion molecules, such as E-cadherin, is influenced by glycosylation. Alteration of a branched N-glycan structure in E-cadherin, or the addition of the β1,6GlcNAc branched glycans to E-cadherin, disturbs the normal function of the protein and promotes tumor cell development and progression. Conversely, the presence of a bisecting N-acetlyglucosamine (GlcNAc) N-glycans in E-cadherin inhibits E-cadherin-mediated cancer progression and prevents epithelial-to-mesenchymal transition process.

Thus, in one aspect the methods, compositions, kits, and systems described herein can be used to screen, diagnose, and/or stage subjects at risk of or suffering from various conditions related to abnormal glycosylation patterns or motifs. Such conditions include but not limited to neurodegenerative diseases or conditions (Moll et al., "Disrupted glycosylation of lipids and proteins is a cause of neurodegeneration," Brain, 143(5):1332-1340 (2020)), cancer (Wang et al., Clin. Chem. Lab. Med., 2019, Mar 26: 57(4):407-416), congenital disorders of glycosylation, and inflammatory conditions such as such as rheumatoid arthritis, inflammatory bowel disease, systemic lupus erythematosus, Tn syndrome, Granulomatosis with polyangiitis, IgA nephropathy, and Diabetes mellitus (Reily et al., "Glycosylation in health and disease," Nature Rev Nephrology, 15:346-366 (2019)). By "stage" it is meant to determine the progression or stage of a disease, such as a cancer stage, onset or progression of a neurodegenerative condition, etc.

More recently, it has been reported that glycosylation of amyloid beta plaques and neurofibrillary tangles is altered in Alzheimer's disease subjects, suggesting a potential implication of this process in disease pathology. Haukedal and Freude, "Implications of Glycosylation in Alzheimer's Disease," Front. Neurosci., 14:625348 (2021), doi.org/10.3389/fnins.2020.625348. *See also* Conroy et al., "Emerging roles ofN-linked glycosylation in brain physiology and disorders," Trends in Endocrinology & Metabolism, 32(12):980-993 (Dec. 2021) ("N-linked glycans impact nearly all neuronal functions, including maintenance of resting membrane potential, axon firing, and synaptic vesicle release.").

In another aspect, encompassed is a method of diagnosis, as detailed above, followed by treating the patient based on the diagnosis. For example, a stage 2 cancer diagnosis may have a different treatment protocol as compared to a stage 4 diagnosis. Similarly, clearly and unambiguously diagnosing a subject with a particular inflammatory condition, neurodegenerative disease, etc., can aid in prescribing the most appropriate treatment protocol.

The present disclosure provides surprisingly effective and novel methods, compositions, systems, and kits for detecting and analyzing the glycosylation of healthy and diseased cells, as well as methods for detecting and analyzing the protein-specific glycosylation state of glycoproteins using single cell-profiling methodologies. The single cell platform disclosed herein enables deeper glycosylation analyses of diverse cell types across multiple pathophysiological states by combining single-cell profiling technologies, transcriptome analyses and advanced glycan detection methods. Moreover, the methods disclosed herein are a significant and dramatic improvement over prior art of glycan identification methods because they leverage the sensitivity and specificity of ultra-high throughput single-cell profiling, which uses barcoding of large numbers (e.g., millions) of cells for targeted analysis of proteins glycosylation and bioorthogonal chemical reactions, (e.g, bioorthogonal chemical reporter strategy with azide- or alkyne-tagged monosaccharide precursors or higher order glycan) to profile the glycome of a single cell. The single cell glycosylation profiling will reveal the abundance of each type of glycan on the cell surface and how the glycosylation pattern of glycoproteins changes with diseased state, based on a sequenceable readout at the single cell level. Furthermore, the methods disclosed herein will identify and distinguish subsets of cells with unique glycosylation profiles at a single-cell level and novel biomarkers that may be used for diagnosis or as targets for drug development. This specificity and detailed analysis is not possible with current analytic methods for analyzing glycans.

The methods, compositions, kits, and systems disclosed herein are significantly superior as compared to current approaches known in the art because they provide a multifaceted recognition system for the detection of the proteins and their post-translation modifications in a single cell under physiological conditions, based on a sequenceable readout. Further, the methods disclosed herein will provide precise single cell data on the glycosylation state in combination with transcriptional analysis of the cell.

The detection of protein specific glycosylation and glycome profiling using single cell methodologies as disclosed herein may be used to identify disease biomarkers, and will offer an unprecedented knowledge into post-translation modifications associated with specific cell types in health and disease and associated changes in protein glycosylation pattern. The methods disclosed herein also provide a more sensitive and a more specific detection of protein-specific glycosylation states in a single cell by improving, and optimizing, proximity ligation assay (PLA) and integrating the optimized PLA into single-cell profiling methodologies, thereby providing a comprehensive snapshot of protein-specific glycosylation state in a single cell.

In one aspect, the present disclosure provides methods, compositions, systems, and kits for determining the glycan profile or profiling the glycome of a cell or tissue. In another aspect, the present disclosure provides methods, compositions, systems, and kits for determining the presence of one or more glycans in a sample by combining, optimizing and integrating chemoezymatic labeling of higher order glycans with oligonucleotide barcode molecules using bioorthogonal chemical reactions, and single cell profiling analysis of chemoenzymatically labeled glycans. In another aspect, the present disclosure provides methods of determining the presence of one or more glycans in a sample comprising one or more living cells by combining, optimizing and integrating metabolic labeling of glycans using unnatural monosaccharides for single cell profiling analysis of the glycome profile. In yet another aspect, the present disclosure provides methods of detecting protein-specific glycosylation patterns in a single cell by combining, optimizing and integrating proximity ligation assay to detect a glycoprotein and its associated post-translation modifications (e.g., glycans) and single cell profiling analysis. In one aspect, the disclosure provides methods, compositions, systems, and kits for labeling specific proteoglycans (glycoprotein), glycans, or glycan motifs and identifying the glycans or glycan motifs from the same cell or cells in close proximity to each other. Each glycan or glycan motif comprises specific sugar residues attached to a glycoprotein (e.g., substrate) through a defined linkage.

In one aspect, the present disclosure provides a use of any methods disclosed herein for screening, diagnosing, or staging a subject at risk of, or suffering from, a disease or condition related to abnormal glycosylation patterns or motifs. In some embodiments, the disease or condition is selected from the group consisting of neurodegenerative diseases or conditions, cancer, congenital disorders of glycosylation, and inflammatory conditions.

### II. SINGLE CELL GLYCAN PROFILING

In one aspect, the present disclosure provides a method of determining the presence of one or more glycans in a sample comprising the steps of: (a) incubating the sample with (i) a first flag molecule comprising a nucleotide sugar and a first reactive molecule of a reaction pair and (ii) a first glycan specific transferase; (b) admixing the sample with a first reporter molecule comprising (i) a second reactive molecule of a reaction pair and (ii) a first reporter oligonucleotide comprising a first glycan motif-specific reporter barcode sequence; (c) removing unincorporated reporter molecules from the sample; (d) partitioning the sample into a plurality of partitions such that a partition comprises (i) a single cell or single cell lysate from the sample and (ii) a plurality of nucleic acid barcode molecules comprising a partition-specific barcode sequence; and (e) using the first reporter oligonucleotide and a nucleic acid barcode molecule of the plurality of nucleic acid barcode molecules to generate a first glycan barcoded nucleic acid molecule comprising the partition-specific barcode sequence or complement thereof and the first glycan motif-specific reporter barcode sequence or complement thereof. In some embodiments, the first flag molecule is incorporated onto a glycan-modified glycoprotein in the sample. In some embodiments, the first reporter molecule is conjugated on the glycan-modified glycoprotein via the first flag molecule.

In another aspect, the present disclosure provides a method of determining the presence of one or more glycans in a sample comprising the steps of: (a) incubating said sample with (i) a flag molecule comprising a nucleotide sugar and a first reactive molecule of a reaction pair and (ii) a first glycan specific transferase; (b) incubating said sample with a reporter molecule comprising (i) a second reactive molecule of a reaction pair and (ii) a reporter oligonucleotide comprising a glycan motif-specific reporter barcode sequence; (c) removing unincorporated reporter molecules from said sample; (d) partitioning said sample into a plurality of partitions such that a partition comprises (i) a single cell or single cell lysate from the sample and (ii) a plurality of nucleic acid barcode molecules comprising a partition-specific barcode sequence; and (e) using the reporter oligonucleotide and a nucleic acid barcode molecule of the plurality of nucleic acid barcode molecules to generate a first barcoded nucleic acid molecule comprising the partition-specific barcode sequence or complement thereof and the reporter barcode sequence or complement thereof.

In some embodiments, the method further comprises (a) determining the sequence of the first glycan barcoded nucleic acid molecule or derivative thereof to identify (i) the partition-specific barcode sequence or complement thereof and (ii) the first glycan motif-specific reporter barcode sequence or complement thereof; and/or (b) using the identified partition-specific barcode sequence or complement thereof and the identified first glycan motif-specific reporter barcode sequence or complement thereof to determine the presence and/or abundance of at least one glycan in the sample.

In various embodiments of the present disclosure, flag molecules comprising a sugar and a first reactive molecule of a reaction pair are provided in a sample. A glycotransferase incorporates the sugar into a glycan; the first reactive molecule of a reaction pair remains attached to the sugar resulting in a glycan comprising the sugar and a first reactive molecule of a reaction pair. The sample is incubated with a reporter molecule comprising a second reactive molecule of the reaction pair and a reporter oligonucleotide comprising a glycan motif-specific reporter barcode sequence. The first reactive molecule of the reaction pair and the second reactive molecule interact with each other, thus coupling a reporter oligonucleotide to the glycan and tagging the specific glycan motif.

Further it is recognized that the methods may involve the use of a first reaction pair; a first and second reaction pair; a first, second and third reaction pair; a first, second, third and fourth reaction pair; a first, second, third, fourth and fifth reaction pair; or a first, second, third, fourth, fifth and at least one additional reaction pair. One skilled in the art would select reaction pairs that are compatible with each other while maintaining pair specificity. The second reactive molecule of one reaction pair may be the first reactive molecule of a different reaction pair.

One or more glycosyltransferases may be provided in the methods. When working with fixed cells or dead cells, the glycosyltransferase must be provided. When the methods involve living cells, the methods may use an endogenous glycosyltransferase. When the methods involve living cells, the methods may use an exogenous glycosyltransferase. As used herein an exogenous glycosyltransferase is any glycosyltransferase provided to the sample including both glycosyltransferases that do not occur in a cell in the sample and externally sourced glycosyltransferases that may occur within a cell in the sample. When an exogenous glycosyltransferease is provided, the specificity of the glycosyltransferase is known. An endogenous glycosyltransferase occurs naturally in a cell.

Methods of removing unincorporated molecules include, but are not limited to, washing with reaction buffer, washing with an aqueous buffer, and the like. An aqueous buffer may include but is not limited to PBS, a PBS buffer comprising bovine serum albumin (BSA) and PBS plus 0.1%-1% BSA. Washing may be performed once or multiple times, such as for example three washing cycles. Unincorporated flag molecules external to cells or absorbed by cells may be removed by any method known in the art. Unincorporated reporter molecules may be removed by any method known in the art.

After the reaction to link the first reactive molecule and the second reactive molecule, a quencher, quenching agent or deactivator may be used to deactivate the first reactive molecule or the second reactive molecule. In an embodiment, an azide reactive group may be deactivated with TCEP (Tris(2-carboxyethyl)phosphine hydrochloride). TCEP may convert azide reactive groups to an amino group, thus preventing further reactions and off target reactions.

The sample is partitioned into a plurality of partitions such that a partition comprises a single cell or single cell lysate from the sample and a plurality of nucleic acid barcode molecules comprising a partition-specific barcode sequence. Partitioning is discussed below.

In another aspect, the present disclosure provides a method of determining the presence of one or more glycans in a sample comprising one or more living cells, comprising the steps of: (a) incubating the sample with a flag molecule comprising a synthetic sugar and a first reactive molecule of a reaction pair; (b) admixing the sample with a reporter molecule comprising (i) a second reactive molecule of the reaction pair and (ii) a reporter oligonucleotide comprising a glycan motif-specific reporter barcode sequence; (c) removing unincorporated reporter molecules; (d) partitioning the sample and a plurality of nucleic acid barcode molecules into a plurality of partitions such that a partition of the plurality comprises a cell from the sample and a plurality of nucleic acid barcode molecules comprising a partition-specific barcode sequence; and (e) using the reporter oligonucleotide and the nucleic acid barcode molecule to generate a glycan barcoded nucleic acid molecule comprising the partition-specific barcode sequence or complement thereof and the glycan motif-specific reporter barcode sequence or complement thereof.

In some embodiments, the flag molecule is a flag substrate for one or more glycosyltransferases of the one or more living cells. In some embodiments, the flag molecule is incorporated and processed in one or more living cells of the sample to generate one or more flag substrates for one or more glycosyltransferases of the one or more living cells. In some embodiments, the flag molecule substrate comprises the first reactive molecule. In some embodiments, the reporter molecule is conjugated on the glycan-modified glycoprotein via the flag molecule.

In another aspect, the present disclosure provides a method of determining the presence of one or more glycans in a sample comprising one or more living cells, comprising the steps of: (a) incubating said sample with a flag molecule comprising a synthetic sugar and a first reactive molecule of a reaction pair; (b) incubating said sample with a reporter molecule comprising (i) a second reactive molecule of said reaction pair and (ii) a reporter oligonucleotide comprising a glycan motif-specific reporter barcode sequence; (c) removing unincorporated reporter molecules; (d) partitioning said sample and a plurality of nucleic acid barcode molecules into a plurality of partitions such that a partition of the plurality comprises a cell from the sample and a plurality of nucleic acid barcode molecules comprising a partition-specific barcode sequence; and (e) using the reporter oligonucleotide and the nucleic acid barcode molecule to generate a barcoded nucleic acid molecule comprising the partition-specific barcode sequence or complement thereof and the glycan motif-specific reporter barcode sequence or complement thereof. In some embodiments, the flag molecule is a flag substrate for one or more glycosyltransferases of the one or more living cells.

In yet another aspect, the present disclosure provides a method of detecting a protein-specific glycosylation pattern in a single cell, the method comprising: (a) incubating a plurality of cells with a glycan-motif specific molecule comprising a reporter oligonucleotide conjugated to a glycan-motif specific reporter barcode sequence; (b) providing a component specific molecule comprising an oligonucleotide conjugated to a component specific barcode sequence, wherein the component specific molecule binds to a glycoprotein; (c) removing unincorporated glycan specific molecules and unincorporated component specific molecules; (d) performing a ligation reaction; (e) partitioning the plurality of cells into a plurality of partitions such that a partition comprises (i) a single cell, single cell lysate, two adjacent cells or lysates of two adjacent cells from the sample and (ii) a plurality of nucleic acid barcode molecules comprising a partition-specific barcode sequence; and (f) amplifying the glycan-component reporter sequence and one of the plurality of nucleic acid barcode molecules to generate a first barcoded nucleic acid molecule comprising the glycan-component reporter sequence or complement thereof and the partition-specific barcode sequence or complement thereof, or derivatives thereof. In some embodiments, the glycan-motif specific molecule binds to a glycan on a glycoprotein. In some embodiments, the oligonucleotide of the glycan specific reporter barcode sequence is ligated to the oligonucleotide of the component specific barcode sequence to generate a ligated glycan-component reporter sequence. In some embodiments, the glycoprotein, the glycan-motif specific molecule and the component specific molecule form a complex.

In another aspect, the present disclosure provides a method of detecting proximity of a glycan to a component of interest, said method comprising: (a) incubating a sample with a glycan-motif specific molecule comprising a reporter oligonucleotide comprising a glycan-motif specific reporter barcode sequence; (b) providing a component specific molecule comprising an oligonucleotide comprising a component specific barcode sequence; (c) removing unincorporated glycan specific molecules and unincorporated component specific molecules; (d) performing a ligation reaction with the oligonucleotide comprising a glycan specific reporter barcode sequence and the oligonucleotide comprising a component specific barcode sequence to generate a glycan-component reporter sequence; (e) partitioning said sample into a plurality of partitions such that a partition comprises (i) a single cell, single cell lysate, two adjacent cells or lysates of two adjacent cells from the sample and (ii) a plurality of nucleic acid barcode molecules comprising a partition-specific barcode sequence; and (f) using the glycan-component reporter sequence and a nucleic acid barcode molecule to generate a barcoded molecule comprising the glycan-component reporter sequence or complement thereof and the partition-specific barcode sequence or complement thereof.

For any of the glycan profiling methods disclosed herein, methods of using a reporter oligonucleotide and a nucleic acid molecule of the plurality of nucleic acid barcode molecules to generate a barcoded nucleic acid molecule are may include but are not limited to ligation, reverse transcription, amplification, primer extension, splint ligation, and template switching. It is recognized that the process or method of generating a barcoded nucleic acid molecule may result in a barcoded nucleic acid molecule comprising a sequence of interest or the reverse complement of the sequence of interest. For example, a first barcoded nucleic acid molecule may comprise a partition-specific barcode sequence or the reverse complement thereof and a reporter barcode sequence or reverse complement thereof. The methods may comprise providing a plurality of cell beads comprising a cell and a reporter oligonucleotide sequence comprising a reporter barcode sequence and subjecting the plurality of cell beads to conditions sufficient to lyse the cells. For any of the glycan profiling methods disclosed herein, the reporter barcode sequence can be used to identify a glycan, a glycan-motif, a glycan class, or a glycan-component combination.

Any of the glycan profiling methods disclosed herein can further comprise sequencing at least a portion of the barcoded nucleic acid molecule or derivative thereof, to identify one or more of a reporter barcode sequence (e.g., a reporter barcode sequence used to identify a glycan, a glycan-motif, a glycan class, or a glycan-component) and a partition-specific barcode sequence. In general, a reporter barcode sequence can be determined and the identified reporter barcode sequence or complement thereof can be used to back-determine the presence of a molecule of interest or indicate molecules having a shared feature. Molecules of interest may include, but are not limited to, a glycan, a glycan motif, a glycan class, an analyte of interest, a metabolite, and a component. Shared features may include but are not limited to a shared partition, a shared cell of origin, a shared sample of origin, a shared analytic processing step, and a shared metabolic processing step.

A glycan motif-specific reporter barcode sequence can be determined and used to back-determine the particular glycan motif present; the back-determination may occur through back-determination of a reaction pair. Thus, reporter barcode sequences may directly or indirectly determine the presence of a glycan, glycan motif or glycan class of interest. A partition-specific barcode sequence can be determined and used to back-determine the molecules from the same partition. A reporter sequence that identifies a glycan motif can be determined and used to back-determine the particular glycan motif present. A glycan specific reporter barcode sequence can be determined and used to back-determine the presence of a glycan of interest.

The abundance of each barcoded nucleic acid molecule may be determined. For example, the abundance of each barcoded nucleic acid molecule may be used to back-determine the abundance of at least one glycan or glycan motif. The methods may comprise determining the abundance of at least two, at least three, at least four, at least five, at least six, at least seven, at least eight or more glycans or glycan motifs.

The methods may be used with one or more different glycans, glycan motifs, glycan classes, analytes, and compounds of interest. Determination of different glycans, glycan motifs and/or glycan classes may be used to elucidate the cell's glycome. A partition may include an additional reporter molecule that is capable of coupling to an additional glycan, glycan motif, glycan class, analyte or compound of interest. Glycan processing or analysis, glycan motif processing or analysis, and/or glycan class processing or analysis can be combined with other analyses in the partition.

Another aspect of the present disclosure provides compositions, kits, and systems for determining the presence of one or more glycans in a sample or for detecting a protein-specific glycosylation pattern in a single cell.

### A. Glycan Profiling Using Chemoenzymatic Labeling

A chemoenzymatic labeling approach to glycome profiling may be used to identify glycan structures on live cell surfaces or on lysates for intracellular targets. The methods involve incubating a sample with a flag molecule comprising a nucleotide sugar and a first reactive molecule of the reaction pair and a first glycan specific transferase. The specificity of the exogenous glycosyltransferase or transferase allows identification of specific glycan structures. The glycan specific transferase incorporates the tagged nucleotide sugar in a glycan. Glycan specific transferases can include, but are not limited to, glycosyltransferase, sialyltransferase, α1,3-fucosyl transferase; BGTA; WbwK fucosyltransferase; α1,2-fucosyl transferase; β1-4 N-acetyl-galactosylaminotransferase; β-galactoside α2,6 sialyltransferase 1; and β-galactoside α2,3 sialyltransferase 1.

One aspect of the present disclosure provides a method of determining the presence of one or more glycans in a sample comprising the steps of: (a) incubating the sample with (i) a first flag molecule comprising a nucleotide sugar and a first reactive molecule of a reaction pair and (ii) a first glycan specific transferase; (b) admixing the sample with a first reporter molecule comprising (i) a second reactive molecule of a reaction pair and (ii) a first reporter oligonucleotide comprising a first glycan motif-specific reporter barcode sequence; (c) removing unincorporated reporter molecules from the sample; (d) partitioning the sample into a plurality of partitions such that a partition comprises (i) a single cell or single cell lysate from the sample and (ii) a plurality of nucleic acid barcode molecules comprising a partition-specific barcode sequence; and (e) using the first reporter oligonucleotide and a nucleic acid barcode molecule of the plurality of nucleic acid barcode molecules to generate a first glycan barcoded nucleic acid molecule comprising the partition-specific barcode sequence or complement thereof and the first glycan motif-specific reporter barcode sequence or complement thereof. In some embodiments, the first flag molecule is incorporated onto a glycan-modified glycoprotein in the sample. In some embodiments, the first reporter molecule is conjugated on the glycan-modified glycoprotein via the first flag molecule.

The sample is incubated with a reporter molecule comprising a second reactive molecule of a reaction pair and a reporter oligonucleotide comprising a glycan motif-specific reporter barcode sequence. The second reactive molecule of a reaction pair interacts with the first reactive molecule of a reaction pair, bringing the reporter molecule with the glycan motif-specific reporter barcode sequence to the glycan. Exemplary glycans and glycan motifs are known in the art. A glycan or glycan motif may be selected from the group consisting of, but not limited to, O-GlcNAc residues, LacNac-containing glycans, Fuc-α1,2-Gal containing glycans, Galβ1-3GalNAc containing glycans, GlcNAc-O-R, Neu5Acα2-3 Gal, and GalT1Y289L. Glycan classes include, but are not limited to, O-linked glycans, N-linked glycans, mucin type O-linked glycans, O-linked glycans core I, and O-linked glycans core 2.

Unincorporated reporter molecules are removed from the sample and the sample is partitioned into a plurality of partitions such that a partition comprises a single cell or single cell lysate from the sample and a plurality of nucleic acid barcode molecules comprising a partition-specific barcode sequence. The reporter oligonucleotide and a nucleic acid barcode molecule of the plurality of nucleic acid barcode molecules is used to generate a first barcoded nucleic acid molecule comprising the partition-specific barcode sequence or complement thereof and the reporter barcode sequence or complement thereof.

**In** some embodiments, the method disclosed herein further comprises (a) determining the sequence of the first glycan barcoded nucleic acid molecule or derivative thereof to identify (i) the partition-specific barcode sequence or complement thereof and (ii) the first glycan motif-specific reporter barcode sequence or complement thereof; and/or (b) using the identified partition-specific barcode sequence or complement thereof and the identified first glycan motif-specific reporter barcode sequence or complement thereof to determine the presence and/or abundance of at least one glycan in the sample.

It is recognized that in various aspects of the methods one or more glycan specific transferases may be incubated with the sample. Each glycan specific transferase preferentially transfers a different nucleotide sugar into a glycan. It is recognized the multiple glycan specific transferases may transfer the nucleotide sugars into the same glycan structure or into different glycan structures. The glycan specific transferase incorporates the nucleotide sugar into a defined glycan motif. The second reactive molecule of a reaction pair on the reporter molecule interacts with the first reactive molecule of a reaction pair on the incorporated nucleotide sugar and bringing the glycan motif-specific reporter barcode sequence to the glycan.

**In** various aspects of the methods, multiple flag molecules may be used. In such cases, a first flag molecule comprises a first nucleotide sugar and a first reactive molecule of a first reaction pair and a second flag molecule comprise a second nucleotide sugar and a first reactive molecule of a second reaction pair. The first nucleotide sugar and the second nucleotide sugar are different nucleotide sugars. Similarly, a third flag molecule comprises a third nucleotide sugar and a first reactive molecule of a third reaction pair. The third nucleotide sugar differs from the first and second nucleotide sugars. Similarly, a fourth flag molecule comprises a fourth nucleotide sugar and a first reactive molecule of a third reaction pair. A plurality of nucleic acid molecules in a partition may further comprise a capture sequence. A reporter oligonucleotide may further comprise a capture handle comprising a sequence complementary to the capture sequence. The capture handle may comprises a sequence complementary to the TSO sequence, to the polyT sequence or both.

The fourth nucleotide sugar differs from the first, second and third nucleotide sugars. Each additional flag molecule would comprise a different additional nucleotide sugar and first reactive molecule of an additional reaction pair. Multiple flag molecules allow identification of multiple glycan motifs in the same sample.

In various aspects of the methods, multiple glycan specific transferases are provided to the sample. The methods may involve a second glycan specific transferase, a third glycan specific transferase, a fourth glycan specific transferase, a fifth glycan specific transferase, a sixth glycan specific transferase, a seventh glycan specific transferase or additional glycan specific transferases. Each glycan specific transferase preferentially transfers a different nucleotide sugar onto a glycan acceptor.

Other aspects of the methods may involve sequential labeling in which a first glycan specific transferase and a first flag molecule are provided to the sample. After the incubation and washing or flushing of the sample and addition of first reporter molecule and removal of unincorporated reporter, a second glycan specific transferase and second flag molecule are incubated the sample. After the incubation, the sample is again washed or flushed and incubated with a second reporter molecule under conditions which allow the first reactive molecule and the second reactive molecule to interact or couple. Unincorporated molecules are removed. Additional rounds of sequential labeling may be performed. A third glycan specific transferase and a third flag molecule may be incubated with the sample. After the incubation, the sample is washed and incubated with a third reporter molecule under conditions which allow the first reactive molecule and the second reactive molecule of the third reaction pair to interact. Unincorporated molecules are removed. A fourth glycan specific transferase and a fourth flag molecule may be incubated with the sample. After the incubation, the sample is washed and incubated with a fourth reporter molecule under conditions which allow the first reactive molecule and the second reactive molecule of the fourth reaction pair to interact. Unincorporated molecules are removed. A fifth glycan specific transferase and a fifth flag molecule may be incubated with the sample. After the incubation, the sample is washed and incubated with a fifth reporter molecule under conditions which allow the first reactive molecule and the second reactive molecule of the fifth reaction pair to interact. Unincorporated molecules are removed. A sixth glycan specific transferase and a sixth flag molecule may be incubated with the sample. After the incubation, the sample is washed and incubated with a sixth reporter molecule under conditions which allow the first reactive molecule and the second reactive molecule of the sixth reaction pair to interact. Unincorporated molecules are removed. A seventh glycan specific transferase and a seventh flag molecule may be incubated with the sample. After the incubation, the sample is washed and incubated with a seventh reporter molecule under conditions which allow the first reactive molecule and the second reactive molecule of the seventh reaction pair to interact. Unincorporated molecules are removed. An eighth glycan specific transferase and an eighth flag molecule may be incubated with the sample. After the incubation, the sample is washed and incubated with an eighth reporter molecule under conditions which allow the first reactive molecule and the second reactive molecule of the eighth reaction pair to interact. Unincorporated molecules are removed. A ninth glycan specific transferase and a ninth flag molecule may be incubated with the sample. After the incubation, the sample is washed and incubated with a ninth reporter molecule under conditions which allow the first reactive molecule and the second reactive molecule of the ninth reaction pair to interact. Unincorporated molecules are removed. A tenth glycan specific transferase and a tenth flag molecule may be incubated with the sample. After the incubation, the sample is washed and incubated with a tenth reporter molecule under conditions which allow the first reactive molecule and the second reactive molecule of the tenth reaction pair to interact. Unincorporated molecules are removed. Further additional rounds of sequential labeling are similar. Sequential labeling may involve 2, 3, 4, 5, 6, 7, 8, 9, 10, 12, 14, 16, 18, 20, 30, 40 or more rounds of labeling.

In some embodiments, the method further comprises (a) incubating the sample with (i) a second flag molecule comprising a second nucleotide sugar and a first reactive molecule of a second reaction pair and (ii) a second glycan specific transferase; and (b) admixing the sample with a second reporter molecule comprising (i) a second reactive molecule of a second reaction pair, and (ii) a second reporter oligonucleotide comprising a reporter barcode sequence that identifies a second glycan motif. In some embodiments, the second flag molecule is incorporated onto a glycan-modified glycoprotein in the sample. In one embodiments, the second reactive molecule is capable of coupling to the first reactive molecule of the second reaction pair. In some embodiments, the second reporter molecule is conjugated on the glycan-modified glycoprotein via the second flag molecule.

In some embodiments, the method further comprises (a) incubating the sample with (i) a third flag molecule comprising a third nucleotide sugar and a first reactive molecule of a third reaction pair and (ii) a third glycan specific transferase; and (b) admixing the sample with a third reporter molecule comprising (i) a second reactive molecule of a third reaction pair, wherein the second reactive molecule is capable of coupling to the first reactive molecule of the third reaction pair, and (ii) a third reporter oligonucleotide comprising a reporter barcode sequence that identifies a third glycan motif. In one embodiment, the third flag molecule is incorporated onto a glycan-modified glycoprotein in the sample. In another embodiment, the third reporter molecule is conjugated on the glycan-modified glycoprotein via the third flag molecule.

In some embodiments, the method further comprises: (a) incubating the sample with (i) a fourth flag molecule comprising a fourth nucleotide sugar and a first reactive molecule of a fourth reaction pair and (ii) a fourth glycan specific transferase; and (b) admixing the sample with a fourth reporter molecule comprising (i) a second reactive molecule of a fourth reaction pair; and (iii) a fourth reporter oligonucleotide comprising a reporter barcode sequence that identifies a fourth glycan motif, wherein the fourth reporter molecule is conjugated on the glycan-modified glycoprotein via the fourth flag molecule. In one embodiment, the fourth flag molecule is incorporated onto a glycan-modified glycoprotein in the sample. In another embodiment, the second reactive molecule is capable of coupling to the first reactive molecule of said fourth reaction pair.

In methods involving sequential labeling, a flag molecule may comprise a nucleotide sugar used in a flag molecule in a different round of the sequential labeling. In an aspect of sequential labeling multiple distinct glycan specific transferases that transfer the same sugar may be provided to the sample. In various aspects, when multiple distinct glycan specific transferases transfer the same sugar, each distinct glycan specific transferase transfers the sugar into a different glycan motif. In an aspect of sequential labeling, each distinct glycan specific transferase transfers a different nucleotide sugar.

In some embodiments, the first, the second, the third or the fourth glycan specific transferase described herein is selected from the group consisting of a β1-4 galatosyltransferase, a glycosyltransferase, sialyltransferase, α1-3-fucosyl transferase; a human blood group A antigen glycosyltransferase (BgtA); WbwK fucosyltransferase; α1-2-fucosyl transferase; β1-4 N-acetyl-galactosylaminotransferase; β-galactoside α2-6 sialyltransferase 1; and β-galactoside α2-3 sialyltransferase 1; ST3Gal1; ST6Gal1; and CgtA. In some embodiments, the first, the second, the third or the fourth flag molecule is selected from the group consisting of UDP-GalNAc; GDP-fucose; UDP-GalNAc; and CMP-Sia. In some embodiments, the glycan-modified glycoprotein comprises a glycan selected from the group consisting of GlcNAc-O-R; LacNAc; Fucα1-2Gal; Galβ1-3GalNAc; Neu5Acα2-3Gal; N-glycans; and O-glycans.

In one embodiment, the first, second, third, or fourth glycan specific transferase is the β1-4 galatosyltransferase, the first, the second, the third or the fourth flag molecule is UDP-GalNAc; and the glycan on the glycan-modified glycoprotein comprises GlcNAc-O-R. In one embodiment, the first, second, third, or fourth glycan specific transferase is the α1-3-fucosyl transferase, the first, the second, the third or the fourth flag molecule is GDP-fucose; and the glycan on the glycan-modified glycoprotein comprises LacNAc. In one embodiment, the first, second, third, or fourth glycan specific transferase is the human blood group A antigen glycosyltransferase (BgtA), the first, second, the third or the fourth flag molecule is UDP-GalNAc; and the glycan on the glycan-modified glycoprotein comprises Fucα1-2Gal. In one embodiment, the first, second, third, or fourth glycan specific transferase is the α1-2-fucosyl transferase, the first, second, the third or the fourth flag molecule is GDP-fucose; and the glycan on the glycan-modified glycoprotein comprises Galβ1-3GalNAc. In another embodiment, the first, second, third, or fourth glycan specific transferase is β1-4 N-acetyl-galactosylaminotransferase, the first, the second, the third or the fourth flag molecule is UDP-GalNAc; and the glycan on the glycan-modified glycoprotein comprises Neu5Acα2-3Gal. In yet another embodiment, the first, second, third, or fourth glycan specific transferase is β-galactoside α2-6 sialyltransferase 1, the first, the second, the third or the fourth flag molecule is CMP-Sia; and the glycan on the glycan-modified glycoprotein is the N-glycan. In another embodiment, the first, second, third, or fourth glycan specific transferase is β-galactoside α2-3 sialyltransferase 1, the second, the third or the fourth flag molecule is CMP-Sia; and the glycan on the glycan-modified glycoprotein is the O-glycan. In one embodiment, the first glycan specific transferase is β1-4 galatosyltransferase; the second glycan specific transferase is α1-3-fucosyl transferase; and the third glycan specific transferase is β-galactoside α2-3 sialyltransferase 1. In another embodiment, the first glycan specific transferase is specific for UDP-GalNAc; the second glycan specific transferase is specific for GDP-fucose; and the third glycan specific transferase is specific for CMP-Sia. In yet another embodiment, the first, second, third, or fourth glycan specific transferase is specific for GlcNAc-O-R; LacNAc; Fucα1-2Gal; Galβ1-3GalNAc; Neu5Acα2-3Gal; N-glycans; or O-glycans.

A reporter molecule may be non-cleavable or cleavable. By "cleavable" is intended the reporter oligonucleotide may be cleaved, separated or removed from the remainder of the reporter molecule. It is recognized that cleaving the reporter oligonucleotide from the reporter molecule may occur after partitioning of the sample occurs. It is recognized that cleaving the reporter molecule may occur after a ligation. It is recognized that cleaving the reporter molecule may occur after a splint reaction is performed. A cleavable linkage between the reporter oligonucleotide and the remainder of the reporter molecule may be a disulfide linker. In various aspects, the cleavable linker may be the same type of cleavable linker used with a plurality of nucleic acid molecules comprising a partition-specific sequence, used with a gel bead or used in aspect of a typical single cell workflow. In various aspects, the cleavable linker may be a different type of cleavable linker than a linker used in a typical single cell workflow. In various aspects the cleavable linker may be selected from the group consisting of DTT-cleavable linkers and reducing agent-cleavable linkers.

The sample is selected from the group of samples comprising tissues, cells, fixed cells, live cells and cell lysates. In various aspects the plurality of partitions receives a single cell or lysate of a single cell from the sample. A cell or lysate from a single cell may be encapsulated in a cell bead, coating the surface of a cell bead, embedded in a cell bead, or any combination thereof. It is also recognized that cell lysis may occur after partitioning of the cells into a plurality of partitions. The methods may comprise the step of subjecting a plurality of cell beads to conditions sufficient to lyse the cells. The conditions sufficient to lyse the cells may involve contacting the cell beads with a lysis agent.

The methods may further comprise the steps of determining the sequence of a plurality of barcoded nucleic acid molecules or derivatives thereof to identify the partition-specific barcode sequence or complement thereof and the glycan motif-specific reporter barcode sequence and using the sequence of the identified partition-specific barcode sequence and the identified glycan motif-specific reporter barcode sequence to determine the presence and/or abundance of one or more glycan motifs in a shared partition. When a partition includes only one cell or lysate from a single cell, then all the identified glycan motifs with the same partition-identifying sequence are from the same cell. The amount, prevalence or abundance of each identified glycan motif may also be determined. In some instances, the reporter barcode sequence comprises a UMI portion that allows identification of each unique instance of the glycan motif. The number of different reporter UMIs indicates the number of different glycan motifs tagged in the original sample.

Partitions are discussed elsewhere herein. Any suitable partition type may be used in the methods. Partitions include but are not limited to droplets, wells, beads, and cell beads. Any plurality of nucleic acid barcode molecules or subset of the plurality of nucleic acid barcode molecules may be provided on a bead. Such a bead may be a gel bead. At least a subset of the plurality of nucleic acid barcode molecules maybe releasably attached to a gel bead. The plurality of nucleic acid barcode molecules in a partition may further comprise a UMI sequence. The UMI sequence of a nucleic acid barcode molecule in a partition may differ from the UMI sequence of another nucleic acid barcode molecule in the partition. The UMI sequence of a nucleic acid barcode molecule in a partition may differ from the UMI sequence of a plurality of nucleic acid barcode molecule in the partition. The UMI sequence of a nucleic acid barcode molecule in a partition may differ from the UMI sequence of a majority of nucleic acid barcode molecule in the partition.

A plurality of nucleic acid molecules in a partition may further comprise a functional sequence. A plurality of nucleic acid molecules in a partition may further comprise a capture sequence. In various aspects the capture sequence comprises a template switch oligonucleotide (TSO) sequence, a polyT sequence or both. A reporter oligonucleotide may further comprise a capture handle comprising a sequence complementary to the capture sequence. The capture handle may comprises a sequence complementary to the TSO sequence, to the polyT sequence or both.

In one aspect the present invention provides a composition for determining the presence of one or more glycan in a sample comprising: (a) a plurality of flag molecules, each comprising a nucleotide sugar and a first reactive molecule of a reaction pair, (b) a plurality of glycan specific transferases, and (c) a plurality of reporter molecules, each comprising a second reactive molecule of a reaction pair and a reporter oligonucleotide comprising a glycan motif-specific reporter barcode sequence.

In some embodiments, each of the plurality of glycan specific transferases is selected from the group consisting of a β1-4 galatosyltransferase, a glycosyltransferase, a sialyltransferase, an α1-3-fucosyl transferase; a human blood group A antigen glycosyltransferase (BgtA); a WbwK fucosyltransferase; an α1-2-fucosyl transferase; a β1-4 N-acetyl-galactosylaminotransferase; a β-galactoside α2-6 sialyltransferase 1; a β-galactoside α2-3 sialyltransferase 1; an ST3Gal1; an ST6Gal1; and a CgtA. In some embodiments, each of the plurality of the glycan specific transferases is specific for GlcNAc-O-R; LacNAc; Fucα1-2Gal; Galβ1-3GalNAc; Neu5Acα2-3Gal; N-glycans; or O-glycans. In some embodiments, each of the plurality of the flag molecules is selected from the group consisting of UDP-GalNAc; GDP-fucose; UDP-GalNAc; and CMP-Sia. In some embodiments, the glycan or the glycan motif is selected from the group consisting of O-GlcNAc residues, LacNac-containing glycans, Fuc-α1,2-Gal containing glycans, Galβ1-3GalNAc containing glycans, GlcNAc-O-R, Neu5Acα2-3 Gal, and GalT1Y289L.

In some embodiments, the reaction pair is selected from the group of reaction pairs consisting of an azide and an alkyne, an azide and a phosphine, an aldehyde and aminooxy, an aldehyde and a hydrazine, an aldehyde and a hydrazide, a ketone and an aminooxy, a hydrazine and a ketone, cyclopropene and a tetrazine, norbornene and tetrazine, trans-cyclooctene and tetrazine, an alkyne and a tetrazine, a nitrone and an alkene, a nitrone and alkyne, diazo and alkyne, and isonitrile and tetrazine. In some embodiments, the second reactive molecule of a reaction pair is capable of coupling to the first reactive molecule of the reaction pair.

**In** some embodiments, the composition further comprises a plurality of cell beads comprising a cell and a reporter oligonucleotide sequence comprising a reporter barcode sequence that identifies a glycan motif. The cell bead may further comprise additional reagents selected from primers, reverse transcriptase enzymes, polymerases, nucleotides, proteases, transposons, endonucleases, switch oligonucleotides, or any combination thereof.

**In** another aspect, the present disclosure provides a composition comprising a plurality of nucleic acid barcode molecules. Each of the plurality of nucleic acid barcode molecules comprises: a partition-specific barcode sequence; unique molecular identifier (UMI) sequenc; and a capture sequence. In one embodiment, the UMI is a functional sequence.

### B. Glycan Profiling Using Metabolic Labeling

One aspect of the present invention discloses a method of determining the presence of one or more glycans in a sample comprising one or more living cells, comprising the steps of: (a) incubating the sample with a flag molecule comprising a synthetic sugar and a first reactive molecule of a reaction pair; (b) admixing the sample with a reporter molecule comprising (i) a second reactive molecule of the reaction pair and (ii) a reporter oligonucleotide comprising a glycan motif-specific reporter barcode sequence; (c) removing unincorporated reporter molecules; (d) partitioning the sample and a plurality of nucleic acid barcode molecules into a plurality of partitions such that a partition of the plurality comprises a cell from the sample and a plurality of nucleic acid barcode molecules comprising a partition-specific barcode sequence; and (e) using the reporter oligonucleotide and the nucleic acid barcode molecule to generate a glycan barcoded nucleic acid molecule comprising the partition-specific barcode sequence or complement thereof and the glycan motif-specific reporter barcode sequence or complement thereof. In one embodiment, the flag molecule is a flag substrate for one or more glycosyltransferases of the one or more living cells. In another embodiment, the flag molecule is incorporated and processed in one or more living cells of the sample to generate one or more flag substrates for one or more glycosyltransferases of the one or more living cells. In yet another embodiment, the flag substrate comprises the first reactive molecule. In one embodiment, the reporter molecule is conjugated on the glycan-modified glycoprotein via the flag molecule.

In some embodiments, the reaction pair is selected from the group of reaction pairs consisting of an azide and an alkyne, an azide and a phosphine, an aldehyde and aminooxy, an aldehyde and a hydrazine, an aldehyde and a hydrazide, a ketone and an aminooxy, a hydrazine and a ketone, cyclopropene and a tetrazine, norbornene and tetrazine, trans-cyclooctene and tetrazine, an alkyne and a tetrazine, a nitrone and an alkene, a nitrone and alkyne, diazo and alkyne, and isonitrile and tetrazine. In some embodiment, the second reactive molecule of a reaction pair described herein is capable of coupling to the first reactive molecule of the reaction pair.

A metabolic labeling approach to glycome profiling may be used to determine the presence of one or more glycans in living cells or on cell surfaces of live cells. The methods involve incubating a sample comprising one or more living cells with a flag molecule comprising a synthetic sugar and first reactive molecule of a reaction pair wherein the flag molecule is a flag substrate for one or more glycosyltransferases of the one or more living cells. It is understood that the flag molecule may be processed by the cell prior to its use by a glycosyltransferase. For example, a flag molecule comprising N-acetylglucosamine may be processed into a flag substrate comprising N-acetylgalactosamine. The cell may than incorporate the N-acetylgalactosamine into a mucin-type O-linked glycan. In another non-limiting example N-acetylmannosamine may be processed into sialic acid.

It is further recognized that one or more endogenous glycosyltransferases may transfer the flag substrate into a glycan or glycan structure. As the method relies on endogenous glycosyltransferases, specificity of the metabolic labelling methods is determined by the flag substrate and particularly the synthetic sugar. Thus, the method may involve transferring the target substrate to one or more different acceptors or the target substrate may be linked to an acceptor through a different linkage. It is understood that a flag substrate comprising a synthetic sugar may be a specific substrate for one endogenous glycosyltransferase or a substrate for more than one endogenous glycosyltransferases. In some embodiments, it may be preferable to select a synthetic sugar that is a specific substrate for a limited number of endogenous glycosyltransferases.

In some embodiments, it may be preferable to select a synthetic sugar that is a substrate for a larger number of endogenous glycosyltransferases.In one embodiment, the synthetic sugar is selected from the group consisting of galactose, sialic acid, fucose, mannose, N-acetylmannosamine and N-acetylgalactosamine. In one embodiments, the synthetic sugar is incorporated into a glycan selected from the group consisting of sialytated glycans; fucosylated glycans; cytosolic O-GlcNAcylated; and mucin type O-linked glycans. In another embodiment, the synthetic sugar is glycan class specific and/or is glycan-motif specific.

Synthetic sugars are discussed elsewhere herein. In various aspects the synthetic sugar is incorporated into a glycan selected from the group consisting of sialic acid and a mucin type O-linked glycans. A synthetic sugar may be glycan motif-specific or glycan class specific. In aspects of the methods, the synthetic sugar may be acetylated. A synthetic sugar may be selected from the group consisting of N-acetylmannosamine and N-acetylgalactosamine.

It is recognized that the incubation time for methods utilizing endogenous glycosyltransferase may be increased over the incubation time for methods involving exogenous glycosyltransferases. The incubation time may be selected to allow sufficient time for the flag molecule to enter the cell and be processed into a flag substrate, if necessary. The incubation duration may be a duration selected from the group of ranges comprising 1 second to 30 seconds, 30 seconds to 1 minute, 1 minute to 10 minutes, 5 minutes to 60 minutes and 1 hour to 12 hours.

The methods involve incubating the sample with a reporter molecule comprising a second reactive molecule of a reaction pair and a reporter oligonucleotide comprising a glycan-motif specific reporter barcode sequence. The duration of the incubation with a reporter molecule may differ from the duration of the incubation with the flag molecule. The second reactive molecule of a reaction pair on the reporter molecule couples with the first reactive molecule of the reaction pair.

Unincorporated reporter molecules are removed as described elsewhere herein. The sample is partitioned into a plurality of partitions as described elsewhere herein. A partition of the plurality of partitions comprise a cell from the sample and a plurality of nucleic acid barcode molecules comprising a partition-specific barcode sequence. The reporter oligonucleotide on the reporter molecule and a nucleic acid barcode molecule are used to generate a barcoded nucleic acid molecule comprising the partition-specific barcode sequence or complement thereof and the glyan-motif specific reporter barcode sequence or complement thereof.

The methods may further comprise determining the sequence of a barcoded nucleic acid molecule or derivative thereof to identify the partition-specific barcode sequence or complement thereof and the glycan-motif specific reporter barcode sequence or complement thereof. The methods may involve using the identified partition-specific barcode sequence or complement thereof and the identified glycan motif-specific reporter barcode sequence or complement thereof to determine the presence and/or abundance of at least one glycan motif in the sample. In some embodiments, the method disclosed herein further comprises (a) determining a sequence of the glycan barcoded nucleic acid molecule or derivative thereof to identify (i) the partition-specific barcode sequence or complement thereof and (ii) the glycan motif-specific reporter barcode sequence or complement thereof. In some embodiments, the method further comprises using the identified partition-specific barcode sequence or complement thereof and the identified glycan motif-specific reporter barcode sequence or complement thereof to determine the presence and/or abundance of at least one glycan in said sample.

Another aspect of the present disclosure provides a composition for determining the presence of one or more glycans in a sample comprising: (a) a plurality of flag molecules, each comprising a synthetic sugar and a first reactive molecule of a reaction pair; and (b) a reporter molecule comprising a second reactive molecule of the reaction pair and a reporter oligonucleotide comprising a glycan motif-specific reporter barcode sequence. In some embodiments, each flag molecule is a flag substrate for one or more glycosyltransferases of the one or more living cells.

In some embodiments, each of the plurality of glycan specific transferases is selected from the group consisting of a β1-4 galatosyltransferase, a glycosyltransferase, a sialyltransferase, an α1-3-fucosyl transferase; a human blood group A antigen glycosyltransferase (BgtA); a WbwK fucosyltransferase; an α1-2-fucosyl transferase; a β1-4 N-acetyl-galactosylaminotransferase; a β-galactoside α2-6 sialyltransferase 1; a β-galactoside α2-3 sialyltransferase 1; an ST3Gal1; an ST6Gal1; and a CgtA. In some embodiments, each of the plurality of the glycan specific transferases is specific for GlcNAc-O-R; LacNAc; Fucα1-2Gal; Galβ1-3GalNAc; Neu5Acα2-3Gal; N-glycans; or O-glycans. In some embodiments, each of the plurality of the flag molecules is selected from the group consisting of UDP-GalNAc; GDP-fucose; UDP-GalNAc; and CMP-Sia. In some embodiments, the glycan or the glycan motif is selected from the group consisting of O-GlcNAc residues, LacNac-containing glycans, Fuc-α1,2-Gal containing glycans, Galβ1-3GalNAc containing glycans, GlcNAc-O-R, Neu5Acα2-3 Gal, and GalT1Y289L.

In some embodiments, the reaction pair is selected from the group of reaction pairs consisting of an azide and an alkyne, an azide and a phosphine, an aldehyde and aminooxy, an aldehyde and a hydrazine, an aldehyde and a hydrazide, a ketone and an aminooxy, a hydrazine and a ketone, cyclopropene and a tetrazine, norbornene and tetrazine, trans-cyclooctene and tetrazine, an alkyne and a tetrazine, a nitrone and an alkene, a nitrone and alkyne, diazo and alkyne, and isonitrile and tetrazine. In some embodiments, the second reactive molecule of a reaction pair is capable of coupling to the first reactive molecule of the reaction pair.

In some embodiments, the composition further comprises a plurality of cell beads comprising a cell and a reporter oligonucleotide sequence comprising a reporter barcode sequence that identifies a glycan motif. The cell bead may further comprise additional reagents selected from primers, reverse transcriptase enzymes, polymerases, nucleotides, proteases, transposons, endonucleases, switch oligonucleotides, or any combination thereof.

In another aspect, the present disclosure provides a composition comprising a plurality of nucleic acid barcode molecules. Each of the plurality of nucleic acid barcode molecules comprises: a partition-specific barcode sequence; unique molecular identifier (UMI) sequenc; and a capture sequence. In one embodiment, the UMI is a functional sequence.

### C. Glycan Profiling Using Proximity Ligation

One aspect of the present disclosure provides A method of detecting a protein-specific glycosylation pattern in a single cell, the method comprising: (a) incubating a plurality of cells with a glycan-motif specific molecule comprising a reporter oligonucleotide conjugated to a glycan-motif specific reporter barcode sequence; (b) providing a component specific molecule comprising an oligonucleotide conjugated to a component specific barcode sequence; (c) removing unincorporated glycan specific molecules and unincorporated component specific molecules; (d) performing a ligation reaction; (e) partitioning the plurality of cells into a plurality of partitions such that a partition comprises (i) a single cell, single cell lysate, two adjacent cells or lysates of two adjacent cells from the sample and (ii) a plurality of nucleic acid barcode molecules comprising a partition-specific barcode sequence; and (f) amplifying the glycan-component reporter sequence and one of the plurality of nucleic acid barcode molecules to generate a first barcoded nucleic acid molecule comprising the glycan-component reporter sequence or complement thereof and the partition-specific barcode sequence or complement thereof, or derivatives thereof. In one embodiment, the glycan-motif specific molecule binds to a glycan on a glycoprotein. In one embodiment, the component specific molecule binds to a glycoprotein. In one embodiment, the oligonucleotide of the glycan specific reporter barcode sequence is ligated to the oligonucleotide of the component specific barcode sequence to generate a ligated glycan-component reporter sequence. In another embodiment, the glycoprotein, the glycan-motif specific molecule and the component specific molecule form a complex.

A proximity ligation approach to glycome profiling may be used to identify glycan structures in proximity to a component of interest. The component of interest may be on a cell surface, an extracellular component or an intracellular component. The component of interest may be on or in the same cell or on an adjacent cell. Components of interest are described elsewhere herein. The methods involve incubating a sample with a glycan-motif specific molecule comprising a reporter oligonucleotide comprising a glycan-motif specific reporter barcode sequence and providing a component specific molecule comprising an oligonucleotide comprising a component specific barcode sequence. By way of example, not limitation, a component specific molecule may be an antibody to a protein of interest. The glycan-motif specific molecule interacts with a target glycan motif on the protein of interest. In some embodiments, the glycan-motif specific molecule may comprise a lectin. In some embodiments, the glycan-motif specific molecule may comprise a glycan motif-specific antibody. The methods may be used to identify a post-translation modification on the protein of interest. In another non-limiting example, the component specific molecule may be a lectin with specificity to a component of interest. The glycan-motif specific molecule interacts with a target glycan motif and the lectin binds a carbohydrate in close proximity. The methods may be used to identify glycans in close proximity to each other. The methods may also be used to evaluate protein-specific glycosylation. Without being limited by mechanism, the glycan-motif specific molecule interacts with the target glycan motif and the component specific molecule interacts with the component of interest.

Methods of removing unbound glycan specific molecules and unbound component specific molecules are known in the art. Methods of washing or removing unbound or unincorporated molecules are discussed elsewhere herein. Any method of washing or removing unbound materials may be used in the methods.

In some embodiments, a splint is provided and a ligation reaction is performed with the oligonucleotide comprising a glycan-specific reporter barcode and the oligonucleotide comprising a component specific barcode sequence while the oligonucleotides are hybridized to the splint to generate a glycan-component reporter sequence. In some cases, an oligonucleotide comprising a glycan specific reporter barcode sequence and an oligonucleotide comprising a component specific barcode sequence can each hybridize to the respective first and second portions of the splint molecule such that a gap with a length of about 0, 1, 2, 3, 4, 5, 6, 7, 8, 9, 10, or more nucleotides is generated between the 3'-end of the nucleic acid sequence of one oligonucleotide and the 5'-end of the other oligonucleotide.

In operation, for example, a splint molecule that is in fluidic contact with (i) glycan specific molecule couple to a glycan and (ii) a component specific molecule coupled to a component can hybridize either sequentially or simultaneously with the oligonucleotide of the glycan specific molecule and the oligonucleotide of the component specific molecule. Sequential hybridization includes hybridization of an oligonucleotide comprising a glycan specific barcode to the first portion of the splint molecule, followed by hybridization of an oligonucleotide comprising the nucleic acid sequence of a component barcode molecule to the second portion of the same splint molecule, or vice versa. In various instances, the splint molecule can be any molecule capable of hybridizing (either sequentially of simultaneously) with (i) an oligonucleotide comprising a glycan specific barcode sequence and (ii) an oligonucleotide comprising a component specific barcode sequence.

In various aspects, the splint molecule, also referred to herein as a bridging sequence or bridging oligonucleotide, can comprise DNA and/or RNA. In some cases, the splint sequence is a splint DNA sequence. A splint sequence can comprise 1, 2, 3, 4, or more portions. Such portions can have different nucleic acid sequences, and thus be capable of hybridizing with different, complementary nucleic acid sequences. In various instances herein, a splint sequence comprises or consists of 2 portions, having either identical or different nucleic acid sequences, and wherein the splint sequence is a splint DNA sequence. In such instances, a splint DNA sequence comprises 2 portions, a first portion capable of hybridizing with an oligonucleotide sequence comprising a glycan specific barcode sequence, and a second portion capable of hybridizing with an oligonucleotide comprising a component specific barcode sequence.

In some instances, a bridging molecule herein can comprise or consist of an amino acid sequence, e.g., peptide nucleic acid (PNA). A splint sequence can comprise at least about 2, 5, 10, 13, 15, 16, 18, 20, 25, 30, 35, 40, 45, 50, 100 or more nucleotides. In some cases, a splint sequence can comprise at most about 100, 50, 45, 40, 35, 30, 25, 20, 18, 16, 15, 13, 10, or 5 nucleotides. In various cases, a splint sequence herein comprises or consists of about 15, 16, 18, or 20 nucleotides. Thus, a splint molecule, e.g., a splint DNA or RNA sequence can be about 16-20 nucleotides in length.

A splint molecule can be designed and/or altered to provide a high binding affinity, e.g., hybridization affinity, for both the first nucleic acid sequence of a capture molecule and a functional nucleic acid sequence of a nucleic acid barcode molecule. Such methods can comprise in silico approaches for the rational design of splint molecules to enhance binding. In some instances, the hybridization affinity of the splint oligonucleotide to their respective first and second handle sequences is < 100 nM. Proximity ligation enables a glycan specific component comprising an oligonucleotide comprising a glycan motif specific reporter barcode sequence to hybridize to a first portion of a splint, which further hybridizes, via a second portion, with a nucleic acid sequence that is part of a component specific barcode sequence attached to a component specific molecule. A ligation reaction is performed yielding a glycan-component barcode sequence. In methods comprising a ligation reaction, the partitioning of the sample may occur before performing the ligation reaction or after performing the ligation reaction. The resulting glycan-component barcode sequence may further be ligated to a partition-specific barcode sequence. In the following steps, the resulting ligation product may be amplified. Furthermore, the amplified product may be prepared for sequence libraries using fragmentation, end-repair, A-tailing, and adapter ligation steps to then perform sample index PCR and library QC. The resulting sequencing data can be analyzed to determine the glycome of single cells of a cell sample. The resulting sequencing data can be analyzed to determine glycans in proximity to a component of interest. The glycan and the component of interest may be on the same cell or adjacent cells.

The ligation reaction may be performed before or after partitioning the sample into a plurality of partitions. The sample is partitioned into a plurality of partitions such that a partition comprises a single cell, lysate from a single cell, two adjacent cells or lysates from two adjacent cells from the sample and a plurality of nucleic acid barcode molecules comprising a partition specific barcode sequence. In some embodiments, the partition is a droplet, or a well. In some embodiments, at least a subset of the plurality of nucleic acid barcode molecules are releasably attached to a gel bead. In another embodiment, at least a subset of the plurality of nucleic acid barcode molecules are releasably attached to a gel bead and further comprising releasing the nucleic acid barcode molecules from the gel bead prior to generating the barcoded nucleic acid molecule. In another embodiment, the plurality of nucleic acid barcode molecules in a partition further comprise a unique molecular identifier (UMI) sequence. In one embodiment, the UMI sequence of one nucleic acid barcode molecule in a partition differs from the UMI sequence of another nucleic acid barcode molecule in the partition. In one embodiment, the plurality of nucleic acid barcode molecules in a partition further comprise a functional sequence; and/or a capture sequence. In one embodiment, the capture sequence comprises a template switch oligonucleotide (TSO) sequence; or a polyT sequence. In one embodiment, the capture sequence comprises a polyT sequence; and the second component is selected from the group consisting of a DNA analyte, RNA analyte, a protein analyte, a cell feature, a cell surface feature, and a metabolite.

The methods involve using the glycan-component reporter sequence and a nucleic acid barcode molecule to generate a barcoded molecule comprising the glycan-component reporter sequence or complement thereof and the partition-specific barcode sequence or complement thereof. The method may involve determining the sequence of a barcoded molecule or derivative thereof to identify the partition-specific barcode sequence or complement thereof and the glycan-component reporter barcode sequence or complement thereof.

The method may further comprise using the identified partition-specific barcode sequence or complement thereof and the identified glycan-component reporter barcode sequence or complement thereof to determine the presence and/or abundance of a glycan in close proximity to compound of interest. A glycan-component reporter barcode sequence can be determined and used to back-determine a glycan and a component that were in proximity to each other. A glycan-component reporter sequence can be determined and used to back-determine a glycan or glycan motif and a component that were in proximity to each other. A glycan-component specific reporter barcode sequence can be used to determine the presence of a glycan or glycan motif of interest in proximity to a component of interest.

A glycan specific molecule may interact with, couple to, bind or attach to a glycan in a preferential manner; the methods are not limited by the mechanism of the interaction. It is recognized that a glycan specific molecule may preferentially bind a glycan or a glycan motif. Glycan specific molecules include, but are not limited to, a glycan specific lectin, a glycan specific antibody, a synthetic nucleotide sugar, a nucleotide sugar, a synthetic sugar and an inactivated glycan specific transferase. It is recognized that a glycan specific molecule should maintain the interaction with the glycan. Thus, an inactivated glycan specific transferase would be preferable to an activated glycan specific transferase which would release the glycan of interest. In various aspects, the methods may involve providing a glycan specific transferase which may attach the glycan specific molecule to the glycan. The methods may be used with any glycan or glycan motif of interest.

In some embodiments, the component specific molecule is selected from the group consisting of antibodies, lectins, synthetic nucleotide sugars, nucleotide sugars, and synthetic sugars. In one embodiment, the component of interest is a protein, glycan, sugar, nucleotide sugar or synthetic nucleotide sugar. In some embodiment, the glycan or the glycan motif is selected from the group consisting of O-GlcNAc residues, LacNac-containing glycans, Fuc-α1,2-Gal containing glycans, Galβ1-3GalNAc containing glycans, GlcNAc-O-R, Neu5Acα2-3 Gal, and GalT1Y289L.In some embodiments, the method described herein further comprises determining the sequence of the first barcoded nucleic acid molecule or derivatives thereof to identify (i) the partition-specific barcode sequence or complement thereof and (ii) the glycan-component reporter barcode sequence or complement thereof. In another embodiment, the method comprises using the identified partition-specific barcode sequence or complement thereof and the identified glycan-component reporter barcode sequence or complement thereof to identify the glycan and the glycosylated pattern of the protein.

Components of interest are described elsewhere herein. A component of interest may be selected from the group consisting of, but not limited to a protein, glycan, sugar, nucleotide sugar, synthetic sugar, synthetic nucleotide sugar, and glycolipid. Any component specific molecule known in the art appropriate for use with a component of interest may be used in the methods. A component specific molecule may be selected from the group including, but not limited to, antibodies, lectins, synthetic nucleotide sugars, nucleotide sugars, and synthetic sugars.

Another aspect of the present disclosure provides a composition for detecting a protein-specific glycosylation pattern in a single cell comprising: (a) a plurality of glycan-motif specific molecules, each comprising a reporter oligonucleotide conjugated to a glycan-motif specific reporter barcode sequence; (b) a plurality of component specific molecules, each comprising an oligonucleotide conjugated to a component specific barcode sequence; and (c) a splint oligonucleotide. In some embodiments, the glycan-motif specific molecule binds to a glycan on a glycoprotein. In some embodiments, the component specific molecule binds to a glycoprotein.

In one embodiments, the component specific molecule is selected from the group consisting of antibodies, lectins, synthetic nucleotide sugars, nucleotide sugars, and synthetic sugars. In one embodiment, the component of interest is a protein, glycan, sugar, nucleotide sugar or synthetic nucleotide sugar. In some embodiments, the splint oligonucleotide is selected from the group consisting of a triazole and a nucleotide barcode splint. In one embodiment, the glycan specific molecule is selected from the group consisting of: (a) a glycan specific lectin; (b) a glycan specific antibody; (c) a synthetic nucleotide sugar; (d) a synthetic sugar; and (e) an inactivated glycan specific transferase. In one embodiment, the component specific molecule is selected from the group consisting of antibodies, lectins, synthetic nucleotide sugars, nucleotide sugars, and synthetic sugars.

In some embodiments, each of the plurality of glycan specific transferases is selected from the group consisting of a β1-4 galatosyltransferase, a glycosyltransferase, a sialyltransferase, an α1-3-fucosyl transferase; a human blood group A antigen glycosyltransferase (BgtA); a WbwK fucosyltransferase; an α1-2-fucosyl transferase; a β1-4 N-acetyl-galactosylaminotransferase; a β-galactoside α2-6 sialyltransferase 1; a β-galactoside α2-3 sialyltransferase 1; an ST3Gal1; an ST6Gal1; and a CgtA. In some embodiments, each of the plurality of the glycan specific transferases is specific for GlcNAc-O-R; LacNAc; Fucα1-2Gal; Galβ1-3GalNAc; Neu5Acα2-3Gal; N-glycans; or O-glycans. In some embodiments, each of the plurality of the flag molecules is selected from the group consisting of UDP-GalNAc; GDP-fucose; UDP-GalNAc; and CMP-Sia. In some embodiments, the glycan or the glycan motif is selected from the group consisting of O-GlcNAc residues, LacNac-containing glycans, Fuc-α1,2-Gal containing glycans, Galβ1-3GalNAc containing glycans, GlcNAc-O-R, Neu5Acα2-3 Gal, and GalT1Y289L.

In some embodiments, the reaction pair is selected from the group of reaction pairs consisting of an azide and an alkyne, an azide and a phosphine, an aldehyde and aminooxy, an aldehyde and a hydrazine, an aldehyde and a hydrazide, a ketone and an aminooxy, a hydrazine and a ketone, cyclopropene and a tetrazine, norbornene and tetrazine, trans-cyclooctene and tetrazine, an alkyne and a tetrazine, a nitrone and an alkene, a nitrone and alkyne, diazo and alkyne, and isonitrile and tetrazine. In some embodiments, the second reactive molecule of a reaction pair is capable of coupling to the first reactive molecule of the reaction pair.

In some embodiments, the composition further comprises a plurality of cell beads comprising a cell and a reporter oligonucleotide sequence comprising a reporter barcode sequence that identifies a glycan motif. The cell bead may further comprise additional reagents selected from primers, reverse transcriptase enzymes, polymerases, nucleotides, proteases, transposons, endonucleases, switch oligonucleotides, or any combination thereof.

In another aspect, the present disclosure provides a composition comprising a plurality of nucleic acid barcode molecules. Each of the plurality of nucleic acid barcode molecules comprises: a partition-specific barcode sequence; unique molecular identifier (UMI) sequenc; and a capture sequence. In one embodiment, the UMI is a functional sequence.

### D. Alternative Methods of Glycan Profiling

Also provided herein is a method of determining the presence of one or more glycans in a sample, comprising (a) incubating the sample with a glycan-specific reporter molecule comprising a glycan-specific binding moiety and a reporter oligonucleotide comprising a reporter barcode sequence, (b) partitioning the sample into a plurality of partitions such that a partition comprises (i) a single cell or single cell lysate from the sample and (ii) a plurality of nucleic acid barcode molecules comprising a partition-specific barcode sequence, and (c) using the reporter oligonucleotide and a nucleic acid barcode molecule of the plurality of nucleic acid barcode molecules to generate a first barcoded nucleic acid molecule comprising the partition-specific barcode sequence or complement thereof and the reporter barcode sequence or complement thereof.

In some embodiments, the glycan-specific binding moiety selectively binds a target glycan. In some embodiments, the reporter barcode sequence or reverse complement thereof is used to identify the target glycan. In some embodiments, the glycan-specific binding moiety selectively binds a target glycan motif. In some embodiments, the reporter barcode sequence or reverse complement thereof is used to identify the target glycan motif. In some embodiments, the glycan-specific binding moiety selectively binds a target glycan class. In some embodiments, the reporter barcode sequence or reverse complement thereof is used to identify the target glycan class.

In some embodiments, the glycan-specific binding moiety comprises an antibody that specifically binds to a target glycan, glycan motif, or glycan class, or an antigen-binding fragment thereof. In some embodiments, the antibody is a monoclonal antibody. Non-limiting examples of antigen-binding fragments include: (i) Fab fragments; (ii) F(ab')2 fragments; (iii) Fd fragments; (iv) Fv fragments; (v) single-chain Fv (scFv) molecules; (vi) dAb fragments; and (vii) minimal recognition units consisting of the amino acid residues that mimic the hypervariable region of an antibody (e.g., an isolated complementarity determining region (CDR) such as a CDR3 peptide), or a constrained FR3-CDR3-FR4 peptide. Other engineered molecules, such as domain-specific antibodies, single domain antibodies, chimeric antibodies, CDR-grafted antibodies, diabodies, triabodies, tetrabodies, minibodies, nanobodies (e.g., monovalent nanobodies, bivalent nanobodies, etc.), small modular immunopharmaceuticals (SMIPs), and shark variable IgNAR domains, are also encompassed within the expression "antigen-binding fragment," as used herein.

In some embodiments, the glycan-specific binding moiety is a glycan-binding protein. In some embodiments, the glycan-binding protein is selected from the group consisting of: ConA, GNA, MAL, SSA, MAH, WGA, LTL, PHA-E, GSL-II, LCA, UEA-I, AOL, AAL, LEL, DSA, ECA, PSA, TJA-I, MAL-I, SNA, PHAL, RCA120, NPA, HHL, ACG, TxLCI, BPL, TJA-II, EEL, ABA, STL, UDA, PWM, Jacalin, PNA, WFA, ACA, MPA, HPA, VVA, DBA, SBA, Calsepa, PTL-I, GSL-IA4, and GSL-IB4, or a glycan-binding fragment thereof.

In some embodiments, a glycan-specific binding moiety can be a lectin or an enzyme. For example, lectins are proteins which recognize carbohydrate domains and mainly bind to carbohydrate sugar groups. Unlike glycan binding proteins, lectins as a group do not include antibodies. Lectins bind both soluble carbohydrates and other carbohydrate moieties complexed with glycoproteins or glycolipids. As such, lectins can cause agglutination or precipitation of glycoconjugates and polysaccharides in mammals. Lectins can also mediate the attachment and binding of bacteria, viruses and fungi to their intended targets. Lectins have many functions, such as cell adhesion regulation, regulation of glycoprotein synthesis, regulation of blood protein levels, binding of glycoproteins, serve as liver cell receptors to remove certain glycoproteins from the blood stream. Further, lectins play an important part in the immune response such as their ability to mediate immune system defenses against microorganisms, their potential importance in modulations inflammatory and other immune responses. Additionally, concanavalin A, a lectin from a bean plant, has been used extensively to understand how proteins recognize carbohydrates and molecular interactions thereof. As such, their use as a glycan-specific binding moiety would be advantageous.

### E. Glycan Profiling with Multi-Assays

A glycan profiling method disclosed herein may be implemented with one or more additional assays or analyses in a multi-analyte analysis. Other analyses may include, but are not limited to transcript analysis, cell surface feature analysis, cell protein analysis, and analysis of cellular gene-editing processes and associated nucleic acid molecules. Suitable methods for multi-analyte analysis in a partition are described in U.S. Patent App. No. 15/720,085, U.S. Patent No. 10,480,029, and PCT/US2017/068320.

The combination of a glycan or glycan motif detection method of the present application with one or more additional assays or analyses may be used to evaluate glycans and gene expression, changes in the glycome related to gene expression and the relationship between the glycome and gene expression. It is understood that gene expression is intended to encompass of gene expression at any level including but not limited to at the transcript level (mRNA), at the protein level and at the metabolite level. The methods allow for simultaneous analysis of the glycome and the transcriptome, analysis of a glycan or glycan motif and the transcriptome, and a glycan or glycan motif and a targeted transcript. The analysis may be used to evaluate changes to the glycome throughout a tissue or collection of cells in conjunction with changes to the transcriptome. The methods allow for analysis of the glycome and the proteome, analysis of a glycan or glycan motif and the proteome, and the glycan or glycan motif and at least one protein or polypeptide of interest. The analysis may be used to evaluate changes to the glycome throughout a tissue or collection of cells in conjunction with changes to the proteome. Proteins of interest include, but are not limited to, cell surface proteins, extracellular membrane proteins and intracellular proteins. The methods allow for simultaneous analysis of the glycome and the metabolome, analysis of a glycan or glycan motif and the metabolome, and a glycan or glycan motif and at least one metabolite. The analysis may be used to evaluate changes to the glycome throughout a tissue or collection of cells in conjunction with changes to the metabolome.

A partition may comprise a plurality of additional nucleic acid barcode molecules comprising the partition-specific barcode sequence and a capture sequence capable of binding to a second analyte of a cell or cell lysate. The second analyte may be any component of the cell, including but not limited to a DNA analyte, RNA analyte, a protein analyte, a cell feature disclosed herein, a cell surface feature disclosed herein, and a metabolite. In some instances, the second analyte is not a glycan or glycan motif. For example, in cases the second analyte is an RNA analyte, e.g., an mRNA analyte, an additional nucleic acid barcode molecule may comprise a polyT sequence that binds to a polyA sequence of mRNA.

In some embodiments, a partition comprises a plurality of additional nucleic acid barcode molecules comprising the partition-specific barcode sequence and a capture sequence capable of binding to a sequence of an additional reporter molecule that specifically binds to the second analyte. For example, if the second analyte is a nucleic acid analyte, e.g., a targeted RNA analyte, the additional reporter molecule may be a targeted probe molecule that specifically binds to the target RNA analyte. See, e.g., PCT/US2019/019309,. The additional reporter molecule may be configured to couple to a target protein. For example, the additional reporter molecule may comprise a labelling agent that specifically binds to the target protein. The additional reporter molecule may be configured to couple to a metabolite. For example, the additional reporter molecule may comprise a labelling agent, e.g., a riboswitch that specifically binds to a target metabolite.

The additional reporter molecule may comprise an additional reporter oligonucleotide comprising a different reporter barcode sequence that identifies the second analyte. Reporter oligonucleotides are described herein.

In multi-analyte analysis a panel of reporter molecules each comprising an analyte specific barcode sequence may be utilized. In multi-analyte analysis, a panel used for analysis may comprise one or more reporter molecules, including glycan-specific reporter molecules disclosed herein, and additional analyte specific reporter molecules; one or more reporter molecules and an analyte-specific molecule, or two or more analyte-specific molecules. For example, a panel for multi-analyte analysis may comprise a first reporter molecule comprising a reporter oligonucleotide comprising a first glycan motif-specific reporter barcode sequence, a second reporter molecule comprising a reporter oligonucleotide comprising a second glycan motif-specific reporter barcode sequence, a third reporter molecule comprising a reporter oligonucleotide comprising a third glycan motif-specific reporter barcode sequence, a fourth reporter molecule comprising a reporter oligonucleotide comprising a fourth glycan-motif specific reporter barcode sequence, a fifth reporter molecule comprising a reporter oligonucleotide comprising a fifth glycan-motif specific reporter barcode sequence, sixth reporter molecule comprising a reporter oligonucleotide comprising a sixth glycan-motif specific reporter barcode sequence, and a seventh reporter molecule comprising a reporter oligonucleotide comprising a seventh glycan-motif specific reporter barcode sequence. A reporter molecule may comprise a second reactive molecule of a reaction pair. In situations with two or more analyte-specific molecules, the two or more analyte-specific molecules may include, but are not limited to a glycan specific molecule and a component specific molecule.

A reporter molecule used to identify a second analyte may comprise a reporter oligonucleotide comprising an analyte specific barcode reporter sequence. Thus, a reporter oligonucleotide may comprise a protein identifying sequence or a metabolite identifying sequence. A reporter oligonucleotide may further comprise a capture handle. By "capture handle" is intended a sequence complementary to a capture sequence of a nucleic acid barcode molecule.

Riboswitches provide a means to evaluate interaction with a metabolite. A riboswitch can refer to a regulatory segment of a nucleic acid molecule (e.g., a ribonucleic acid molecule (RNA)), or a messenger RNA, etc.) that can bind a species (including a small molecules, metabolites, etc.), resulting in a change in production of proteins encoded by nucleic acid molecules in the cell. Additional details regarding riboswitches are provided in Ruff & Strobel, RNA, 2014 November; 20(11): 1775-88 and Butler et al. Chem. Biol. 2011 Mar. 25; 18(3):293-298. A particular riboswitch may be linked to a reporter oligonucleotide. Such a riboswitch identifier sequence can be determined and used to detect metabolites. Moreover, when the species binds to its respective riboswitch, the riboswitch can change its secondary structure and/or tertiary structure such that one or more sequences of the riboswitch, inaccessible in a species-free state, become accessible in a species-bound state. Such a sequence can be used as a capture sequence that can bind a nucleic acid barcode molecule and, via one or more reactions), add a complementary sequence corresponding to the riboswitch (including the riboswitch identifier sequence) to the nucleic acid barcode molecule. Methods for use of riboswitches for metabolite analysis are described in PCT/US2019/043782.

Any nucleic acid barcode molecule may comprise a capture sequence. A capture sequence may comprise a template switch oligonucleotide (TSO) sequence. A capture sequence may comprise a polyT sequence. A capture sequence may comprise a sequence complementary to a capture handle sequence of a reporter oligonucleotide.

Another aspect of the disclosure provides a composition for characterizing a plurality of analytes. The composition comprises a partition comprising a plurality of barcode molecules and the plurality of analytes. The plurality of barcode molecules can comprise at least 1,000 barcode molecules. In addition, (i) a first individual barcode molecule of the plurality of barcode molecules can comprise a first nucleic acid barcode sequence that is capable of coupling to a first analyte of the plurality of analytes; and (ii) a second individual barcode molecule of the plurality of barcoded molecules can comprise a second nucleic acid barcode sequence that is capable of coupling to a second analyte of the plurality of analytes, where the first analyte and the second analyte are different types of analytes.

In some embodiments, the first analyte is a nucleic acid molecule, such as genomic deoxyribonucleic acid (gDNA) or is messenger RNA (mRNA). In some embodiments, the first analyte is a labelling agent capable of coupling to a cell surface feature of a cell. In some embodiments, the first individual barcode molecule or the second individual barcode molecule is capable of coupling to the labelling agent via a third nucleic acid molecule coupled to the labelling agent. In some embodiments, the cell surface feature is a receptor, an antigen, or a protein. In some embodiments, the labelling agent is an antibody, or an epitope binding fragment thereof. In some embodiments, the partition comprises the cell or one or more components of the cell. In some embodiments, the partition comprises a single cell. In some embodiments, the first nucleic acid molecule or the second nucleic molecule comprises a third barcode sequence. In some embodiments, the third barcode sequence is derived from a third nucleic acid molecule. In some embodiments, the third nucleic acid molecule is coupled to a labelling agent capable of binding to a cell surface feature of a cell.

In some embodiments, the first analyte and second analyte are different types nucleic acid molecules. In some embodiments, the first analyte is a ribonucleic acid molecule and the second analyte is a deoxyribonucleic acid molecule. In some embodiments, (i) the first individual barcode molecule comprises a first priming sequence capable of hybridizing to the first analyte; or (ii) the second individual barcode molecule comprises a second priming sequence capable of hybridizing to the second analyte. In some embodiments, the first barcode molecule or the second barcode molecule comprises a unique molecular identification (UMI) sequence.

In some embodiments, the first analyte is a nucleic acid molecule and the second analyte is a labelling agent capable of coupling to a cell surface feature. In some embodiments, the first analyte is a ribonucleic acid molecule. In some embodiments, (i) the first individual barcode molecule comprises a first priming sequence capable of hybridizing to the first analyte; or (ii) the second individual barcode molecule comprises a second priming sequence capable of hybridizing to a third nucleic acid molecule coupled to the labelling agent. In some embodiments, the labelling agent is an antibody, or an epitope binding fragment thereof. In some embodiments, the cell surface feature is a receptor, an antigen, or a protein.

Another aspect of the present disclosure provides a use of any method disclosed herein for screening, diagnosing, or staging a subject at risk of, or suffering from, a disease or condition related to abnormal glycosylation patterns or motifs. In some embodiments, the disease or condition is selected from the group consisting of neurodegenerative diseases or conditions, cancer, congenital disorders of glycosylation, and inflammatory conditions.

### F. Kits

Another aspect of the present disclosure provides a kit for determining the presence of one or more glycans in a sample or for detecting a protein-specific glycosylation pattern in a single cell. In some embodiments, the kit comprises any of the compositions for determining the presence of one or more glycans in a sample as described herein, and/or any of the compositions for detecting a protein-specific glycosylation pattern in a single cell as described herein; and/or instructions for performing any of the methods for determining the presence of one or more glycans in a sample or for detecting a protein-specific glycosylation pattern in a single cell as described herein.

In another aspect, the present disclosure provides a kit, comprising (i) a plurality of flag molecules, each comprising a nucleotide sugar and a first reactive molecule of a reaction pair; (ii) a plurality of glycan specific transferases; (iii) a plurality of reporter molecules, each comprising a second reactive molecule of a reaction pair and a reporter oligonucleotide comprising a glycan motif-specific reporter barcode sequence, and (iv) instructions for performing any of the methods for determining the presence of one or more glycans in a sample as described herein.

In another aspect, the present disclosure provides (i) a plurality of flag molecules, each comprising a synthetic sugar and a first reactive molecule of a reaction pair, wherein the flag molecule is a flag substrate for one or more glycosyltransferases of the one or more living cells, (ii) a plurality of reporter molecules, each comprising a second reactive molecule of the reaction pair and a reporter oligonucleotide comprising a glycan motif-specific reporter barcode sequence, and (iv) instructions for performing any of the methods for determining the presence of one or more glycans in a sample as described herein.

Yet in another aspect, the present disclosure provides (i) a plurality of glycan-motif specific molecules, each comprising a reporter oligonucleotide conjugated to a glycan-motif specific reporter barcode sequence, wherein the glycan-motif specific molecule binds to a glycan on a glycoprotein, (ii) a plurality of component specific molecules, each comprising an oligonucleotide conjugated to a component specific barcode sequence, wherein the component specific molecule binds to a glycoprotein; and optionally (iii) a splint oligonucleotide, and (iv) instructions for performing any of the methods for detecting a protein-specific glycosylation pattern in a single cell as described herein.

Another aspect of the disclosure provides methods for screening, diagnosing, or staging a subject at risk of, or suffering from, a disease or condition related to abnormal glycosylation patterns or motifs using the methods and compositions disclosed herein. In some embodiments, the disease or condition is selected from the group consisting of neurodegenerative diseases or conditions, cancer, congenital disorders of glycosylation, and inflammatory conditions.

### III. SINGLE CELL AND SINGLE BIOLOGICAL PARTICLE ANALYSIS

### A. Systems and Methods for Sample Compartmentalization

In one aspect the present disclosure provides a system for determining the presence of one or more glycans in a sample or for detecting a protein-specific glycosylation pattern in a single cell, comprising: (a) a first channel in fluid communication with a first source comprising a plurality of cells labeled with a glycan or a glycan-specific molecule using the method disclosed herein; (b) a second channel in fluid communication with a second source comprising a plurality of nucleic acid barcode molecules comprising a partition-specific barcode sequence; and (c) a junction that brings a first phase comprising said plurality of cells from said first channel and said plurality of nucleic acid barcode molecules from said second channel in contact with a second phase that is immiscible with said first phase, to yield a plurality of droplets comprising said plurality of cells and said plurality of nucleic acid barcode molecules. In some embodiments, a droplet of said plurality of droplets comprises said cell and said barcode bead. In some embodiments, the cell is encapsulated in a cell bead, coated on a bead cell, embedded in a bead cell, or any combination thereof.

In some embodiments, the system further comprises a third channel in fluid communication with a third source comprising additional reagents, wherein said first phase comprises said additional reagents. In one embodiment, the system further comprises a fourth channel in fluid communication with a fourth source comprising additional reagents. In one embodiment, said first phase comprises said additional reagents. In some embodiments, said additional reagents are reagents for nucleic acid amplification reagents that can degrade or dissolve cells, cell beads and/or barcode beads. In another embodiment, said additional reagents are reagents for nucleic acid amplification reagents that degrade linkages between barcodes and barcode beads, or any combination thereof.

Another aspect of the present disclosure provides a system, comprising: (i) a plurality of flag molecules, each comprising a nucleotide sugar and a first reactive molecule of a reaction pair, (ii) a plurality of glycan specific transferases, and (iii) a plurality of reporter molecules, each comprising a second reactive molecule of a reaction pair and a reporter oligonucleotide comprising a glycan motif-specific reporter barcode sequence; and (iv) a plurality of nucleic acid barcode molecules. In some embodiments, a first nucleic acid barcode molecule of the plurality of nucleic acid barcode molecules comprises a partition barcode sequence. In some embodiment, the plurality of nucleic acid barcode molecules is attached to a bead. In one embodiment, the partition barcode sequence identifies the bead. In some embodiments, the system further comprises a plurality of partitions. In one embodiment, the plurality of partitions comprises a plurality of droplets and/or a plurality of wells. In some embodiments, the system further comprises an apparatus comprising a microfluidic channel structure configured to generate a plurality of partitions. In one embodiment, the apparatus comprises (i) a first channel in fluid communication with a first source comprising a plurality of cells labeled with a glycan or a glycan-specific molecule using the methods for determining the presence of one or more glycans in a sample as described herein; (ii) a second channel in fluid communication with a second source comprising the plurality of nucleic acid barcode molecules, and (iii) a junction that brings a first phase comprising said plurality of cells from the first channel and the plurality of nucleic acid barcode molecules from said second channel in contact with a second phase that is immiscible with said first phase, to yield a plurality of droplets comprising the plurality of cells and said plurality of nucleic acid barcode molecules. In some embodiments, a droplet of said plurality of droplets comprises said cell and said barcode bead.

In another aspect, the present disclosure provides a system, comprising: (i) a plurality of flag molecules, each comprising a synthetic sugar and a first reactive molecule of a reaction pair, wherein the flag molecule is a flag substrate for one or more glycosyltransferases of the one or more living cells; (ii) a plurality of reporter molecules, each comprising a second reactive molecule of the reaction pair and a reporter oligonucleotide comprising a glycan motif-specific reporter barcode sequence; (iii) a plurality of nucleic acid barcode molecules, wherein a first nucleic acid barcode molecule of the plurality of nucleic acid barcode molecules comprises a partition barcode sequence. In some embodiments, the plurality of nucleic acid barcode molecules is attached to a bead, and wherein the partition barcode sequence identifies the bead. In some embodiments, the system further comprising a plurality of partitions. In one embodiment, the plurality of partitions comprises a plurality of droplets and/or a plurality of wells, and/or the system further comprises an apparatus comprising a microfluidic channel structure configured to generate a plurality of partitions. In one embodiment, the apparatus comprises (i) a first channel in fluid communication with a first source comprising a plurality of cells labeled with a glycan or a glycan-specific molecule using the methods for determining the presence of one or more glycans in a sample as described herein, (ii) a second channel in fluid communication with a second source comprising the plurality of nucleic acid barcode molecules, and (iii) a junction that brings a first phase comprising said plurality of cells from the first channel and the plurality of nucleic acid barcode molecules from said second channel in contact with a second phase that is immiscible with said first phase, to yield a plurality of droplets comprising the plurality of cells and said plurality of nucleic acid barcode molecules. In some embodiments, a droplet of said plurality of droplets comprises said cell and said barcode bead.

In another aspect, the present disclosure provides a system, comprising: (i) a plurality of glycan-motif specific molecules, each comprising a reporter oligonucleotide conjugated to a glycan-motif specific reporter barcode sequence (ii) a plurality of component specific molecules, each comprising an oligonucleotide conjugated to a component specific barcode sequence; and (iii) a plurality of nucleic acid barcode molecules. In some embodiments, the system further comprises a splint oligonucleotide. In some embodiments, the glycan-motif specific molecule binds to a glycan on a glycoprotein. In some embodiments, the component specific molecule binds to a glycoprotein. In some embodiments, a first nucleic acid barcode molecule of the plurality of nucleic acid barcode molecules comprises a partition barcode sequence.

In one embodiment, the plurality of nucleic acid barcode molecules is attached to a bead. In one embodiment, the partition barcode sequence identifies the bead, and/or the system further comprising a plurality of partitions. In some embodiments, the plurality of partitions comprises a plurality of droplets and/or a plurality of wells, and/or the system further comprises an apparatus comprising a microfluidic channel structure configured to generate a plurality of partitions. In one embodiment, the apparatus comprises (i) a first channel in fluid communication with a first source comprising a plurality of cells labeled with a glycan or a glycan-specific molecule using the methods for detecting a protein-specific glycosylation pattern in a single cell as described herein, (ii) a second channel in fluid communication with a second source comprising the plurality of nucleic acid barcode molecules, and (iii) a junction that brings a first phase comprising said plurality of cells from the first channel and the plurality of nucleic acid barcode molecules from said second channel in contact with a second phase that is immiscible with said first phase, to yield a plurality of droplets comprising the plurality of cells and said plurality of nucleic acid barcode molecules. In some embodiments, a droplet of said plurality of droplets comprises said cell and said barcode bead.

In an aspect, the systems and methods described herein provide for the compartmentalization, depositing, or partitioning of one or more particles (e.g., biological particles, macromolecular constituents of biological particles, beads, reagents, etc.) into discrete compartments or partitions (referred to interchangeably herein as partitions), where each partition maintains separation of its own contents from the contents of other partitions. The partition can be a droplet in an emulsion or a well. A partition may comprise one or more other partitions.

A partition may include one or more particles. A partition may include one or more types of particles. For example, a partition of the present disclosure may comprise one or more biological particles and/or macromolecular constituents thereof. A partition may comprise one or more beads. A partition may comprise one or more gel beads. A partition may comprise one or more cell beads. A partition may include a single gel bead, a single cell bead, or both a single cell bead and single gel bead. A partition may include one or more reagents. Alternatively, a partition may be unoccupied. For example, a partition may not comprise a bead.

Unique identifiers, such as barcodes, may be injected into the droplets previous to, subsequent to, or concurrently with droplet generation, such as via a bead, as described elsewhere herein.

The methods and systems of the present disclosure may comprise methods and systems for generating one or more partitions such as droplets. The droplets may comprise a plurality of droplets in an emulsion. In some examples, the droplets may comprise droplets in a colloid. In some cases, the emulsion may comprise a microemulsion or a nanoemulsion. In some examples, the droplets may be generated with aid of a microfluidic device and/or by subjecting a mixture of immiscible phases to agitation (e.g., in a container). In some cases, a combination of the mentioned methods may be used for droplet and/or emulsion formation.

The partitions described herein may comprise small volumes, for example, less than about 10 microliters (mL), 5mL, 1mL, 900 picoliters (pL), 800 pL, 700 pL, 600 pL, 500 pL, 400pL, 300 pL, 200 pL, 100pL, 50 pL, 20 pL, 10 pL, 1 pL, 500 nanoliters (nL), 100 nL, 50 nL, or less.

For example, in the case of droplet based partitions, the droplets may have overall volumes that are less than about 1000 pL, 900 pL, 800 pL, 700 pL, 600 pL, 500 pL, 400pL, 300 pL, 200 pL, 100pL, 50 pL, 20 pL, 10 pL, 1 pL, or less. Where co-partitioned with beads, it will be appreciated that the sample fluid volume, e.g., including co-partitioned biological particles and/or beads, within the partitions may be less than about 90% of the above described volumes, less than about 80%, less than about 70%, less than about 60%, less than about 50%, less than about 40%, less than about 30%, less than about 20%, or less than about 10% of the above described volumes.

As is described elsewhere herein, partitioning species may generate a population or plurality of partitions. In such cases, any suitable number of partitions can be generated or otherwise provided. For example, at least about 1,000 partitions, at least about 5,000 partitions, at least about 10,000 partitions, at least about 50,000 partitions, at least about 100,000 partitions, at least about 500,000 partitions, at least about 1,000,000 partitions, at least about 5,000,000 partitions at least about 10,000,000 partitions, at least about 50,000,000 partitions, at least about 100,000,000 partitions, at least about 500,000,000 partitions, at least about 1,000,000,000 partitions, or more partitions can be generated or otherwise provided. Moreover, the plurality of partitions may comprise both unoccupied partitions (e.g., empty partitions) and occupied partitions.

Droplets can be formed by creating an emulsion by mixing and/or agitating immiscible phases. Mixing or agitation may comprise various agitation techniques, such as vortexing, pipetting, tube flicking, or other agitation techniques. In some cases, mixing or agitation may be performed without using a microfluidic device. In some examples, the droplets may be formed by exposing a mixture to ultrasound or sonication. Systems and methods for droplet and/or emulsion generation by agitation are described in International Application No. PCT/US20/17785.

### B. Microfluidic systems

Microfluidic devices or platforms comprising microfluidic channel networks (e.g., on a chip) can be utilized to generate partitions such as droplets and/or emulsions as described herein. Methods and systems for generating partitions such as droplets, methods of encapsulating biological particles and/or beads in partitions, methods of increasing the throughput of droplet generation, and various geometries, architectures, and configurations of microfluidic devices and channels are described in U.S. Patent Publication Nos. 2019/0367997 and 2019/0064173.

In some examples, individual particles can be partitioned to discrete partitions by introducing a flowing stream of particles in an aqueous fluid into a flowing stream or reservoir of a non-aqueous fluid, such that droplets may be generated at the junction of the two streams/reservoir, such as at the junction of a microfluidic device provided elsewhere herein.

The methods of the present disclosure may comprise generating partitions and/or encapsulating particles, such as biological particles, in some cases, individual biological particles such as single cells. In some examples, reagents may be encapsulated and/or partitioned (e.g., co-partitioned with biological particles) in the partitions. Various mechanisms may be employed in the partitioning of individual particles. An example may comprise porous membranes through which aqueous mixtures of cells may be extruded into fluids (e.g., non-aqueous fluids).

The partitions can be flowable within fluid streams. The partitions may comprise, for example, micro-vesicles that have an outer barrier surrounding an inner fluid center or core. In some cases, the partitions may comprise a porous matrix that is capable of entraining and/or retaining materials within its matrix. The partitions can be droplets of a first phase within a second phase, wherein the first and second phases are immiscible. For example, the partitions can be droplets of aqueous fluid within a non-aqueous continuous phase (e.g., oil phase). In another example, the partitions can be droplets of a non-aqueous fluid within an aqueous phase. In some examples, the partitions may be provided in a water-in-oil emulsion or oil-in-water emulsion. A variety of different vessels are described in, for example, U.S. Patent Application Publication No. 2014/0155295. Emulsion systems for creating stable droplets in non-aqueous or oil continuous phases are described in, for example, U.S. Patent Application Publication No. 2010/0105112.

Fluid properties (e.g., fluid flow rates, fluid viscosities, etc.), particle properties (e.g., volume fraction, particle size, particle concentration, etc.), microfluidic architectures (e.g., channel geometry, etc.), and other parameters may be adjusted to control the occupancy of the resulting partitions (e.g., number of biological particles per partition, number of beads per partition, etc.). For example, partition occupancy can be controlled by providing the aqueous stream at a certain concentration and/or flow rate of particles. To generate single biological particle partitions, the relative flow rates of the immiscible fluids can be selected such that, on average, the partitions may contain less than one biological particle per partition in order to ensure that those partitions that are occupied are primarily singly occupied. In some cases, partitions among a plurality of partitions may contain at most one biological particle (e.g., cell). In some embodiments, the various parameters (e.g., fluid properties, particle properties, microfluidic architectures, etc.) may be selected or adjusted such that a majority of partitions are occupied, for example, allowing for only a small percentage of unoccupied partitions. The flows and channel architectures can be controlled as to ensure a given number of singly occupied partitions, less than a certain level of unoccupied partitions and/or less than a certain level of multiply occupied partitions.

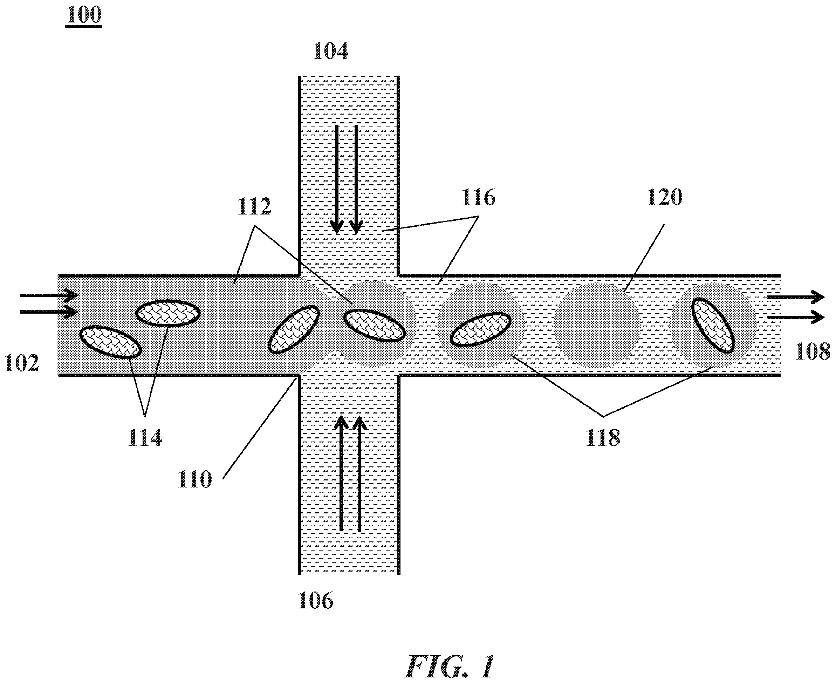

**FIG. 1** shows an example of a microfluidic channel structure **100** for partitioning individual biological particles. The channel structure **100** can include channel segments **102, 104, 106** and **108** communicating at a channel junction **110.** In operation, a first aqueous fluid **112** that includes suspended biological particles (e.g., cells) **114** may be transported along channel segment **102** into junction **110,** while a second fluid **116** that is immiscible with the aqueous fluid **112** is delivered to the junction **110** from each of channel segments **104** and **106** to create discrete droplets **118, 120** of the first aqueous fluid **112** flowing into channel segment **108,** and flowing away from junction **110.** The channel segment **108** may be fluidically coupled to an outlet reservoir where the discrete droplets can be stored and/or harvested. A discrete droplet generated may include an individual biological particle **114** (such as droplets **118**). A discrete droplet generated may include more than one individual biological particle **114** (not shown in **FIG. 1**). A discrete droplet may contain no biological particle **114** (such as droplet **120**). Each discrete partition may maintain separation of its own contents (e.g., individual biological particle **114**) from the contents of other partitions.

The second fluid **116** can comprise an oil, such as a fluorinated oil, that includes a fluorosurfactant for stabilizing the resulting droplets, for example, inhibiting subsequent coalescence of the resulting droplets **118, 120.** Examples of particularly useful partitioning fluids and fluorosurfactants are described, for example, in U.S. Patent Application Publication No. 2010/0105112.

As will be appreciated, the channel segments described herein may be coupled to any of a variety of different fluid sources or receiving components, including reservoirs, tubing, manifolds, or fluidic components of other systems. As will be appreciated, the microfluidic channel structure **100** may have other geometries. For example, a microfluidic channel structure can have more than one channel junction. For example, a microfluidic channel structure can have 2, 3, 4, or 5 channel segments each carrying particles (e.g., biological particles, cell beads, and/or gel beads) that meet at a channel junction. Fluid may be directed to flow along one or more channels or reservoirs via one or more fluid flow units. A fluid flow unit can comprise compressors (e.g., providing positive pressure), pumps (e.g., providing negative pressure), actuators, and the like to control flow of the fluid. Fluid may also or otherwise be controlled via applied pressure differentials, centrifugal force, electrokinetic pumping, vacuum, capillary or gravity flow, or the like.

The generated droplets may comprise two subsets of droplets: (1) occupied droplets **118,** containing one or more biological particles **114,** and (2) unoccupied droplets **120,** not containing any biological particles **114.** Occupied droplets **118** may comprise singly occupied droplets (having one biological particle) and multiply occupied droplets (having more than one biological particle). As described elsewhere herein, in some cases, the majority of occupied partitions can include no more than one biological particle per occupied partition and some of the generated partitions can be unoccupied (of any biological particle). In some cases, though, some of the occupied partitions may include more than one biological particle. In some cases, the partitioning process may be controlled such that fewer than about 25% of the occupied partitions contain more than one biological particle, and in many cases, fewer than about 20% of the occupied partitions have more than one biological particle, while in some cases, fewer than about 10% or even fewer than about 5% of the occupied partitions include more than one biological particle per partition.

In some cases, it may be desirable to minimize the creation of excessive numbers of empty partitions, such as to reduce costs and/or increase efficiency. While this minimization may be achieved by providing a sufficient number of biological particles (e.g., biological particles **114)** at the partitioning junction **110,** such as to ensure that at least one biological particle is encapsulated in a partition, the Poissonian distribution may expectedly increase the number of partitions that include multiple biological particles. As such, where singly occupied partitions are to be obtained, at most about 95%, 90%, 85%, 80%, 75%, 70%, 65%, 60%, 55%, 50%, 45%, 40%, 35%, 30%, 25%, 20%, 15%, 10%, 5% or less of the generated partitions can be unoccupied.

In some cases, flows can be controlled so as to present a non-Poissonian distribution of single-occupied partitions while providing lower levels of unoccupied partitions (e.g., no more than about 50%, about 25%, or about 10% unoccupied). The above noted ranges of unoccupied partitions can be achieved while still providing any of the single occupancy rates described above. As will be appreciated, the above-described occupancy rates are also applicable to partitions that include both biological particles and additional reagents, such as beads (e.g., gel beads) carrying nucleic acid barcode molecules (e.g., oligonucleotides).

In some examples, a partition of the plurality of partitions may comprise a single biological particle or biological particle (e.g., a single cell or a single nucleus of a cell). In some examples, a partition of the plurality of partitions may comprise multiple biological particles or biological particles. Such partitions may be referred to as multiply occupied partitions, and may comprise, for example, two, three, four or more cells and/or beads comprising nucleic acid barcode molecules (e.g., oligonucleotides) within a single partition. Accordingly, as noted above, the flow characteristics of the biological particle and/or bead containing fluids and partitioning fluids may be controlled to provide for such multiply occupied partitions. In particular, the flow parameters may be controlled to provide a given occupancy rate at greater than about 50% of the partitions, greater than about 75%, and in some cases greater than about 80%, 90%, 95%, or higher. Microfluidic systems for partitioning are further described in U.S. Patent Application Pub. No. US 2015/0376609.

### C. Controlled Partitioning

In some aspects, provided are systems and methods for controlled partitioning. Droplet size may be controlled by adjusting certain geometric features in channel architecture (e.g., microfluidics channel architecture). For example, an expansion angle, width, and/or length of a channel may be adjusted to control droplet size.

In some aspects, provided are systems and methods for controlled partitioning. Droplet size may be controlled by adjusting certain geometric features in channel architecture (e.g., microfluidics channel architecture). For example, an expansion angle, width, and/or length of a channel may be adjusted to control droplet size.

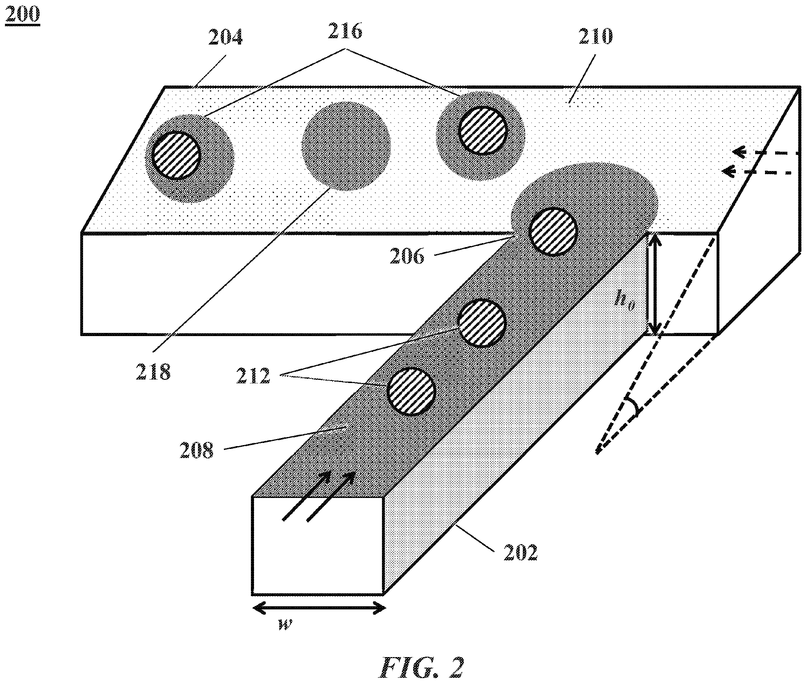

**FIG. 2** shows an example of a microfluidic channel structure for the controlled partitioning of beads into discrete droplets. A channel structure **200** can include a channel segment **202** communicating at a channel junction **206** (or intersection) with a reservoir **204.** The reservoir **204** can be a chamber. Any reference to "reservoir," as used herein, can also refer to a "chamber." In operation, an aqueous fluid **208** that includes suspended beads **212** may be transported along the channel segment **202** into the junction **206** to meet a second fluid **210** that is immiscible with the aqueous fluid **208** in the reservoir **204** to create droplets **216, 218** of the aqueous fluid **208** flowing into the reservoir **204.** At the junction **206** where the aqueous fluid **208** and the second fluid **210** meet, droplets can form based on factors such as the hydrodynamic forces at the junction **206,** flow rates of the two fluids **208, 210,** fluid properties, and certain geometric parameters (e.g., *w*, *h₀, α*, etc.) of the channel structure **200.** A plurality of droplets can be collected in the reservoir **204** by continuously injecting the aqueous fluid **208** from the channel segment **202** through the junction **206.**

In some instances, the aqueous fluid **208** can have a substantially uniform concentration or frequency of beads **212.** The beads **212** can be introduced into the channel segment **202** from a separate channel (not shown in **FIG. 2**). The frequency of beads **212** in the channel segment **202** may be controlled by controlling the frequency in which the beads **212** are introduced into the channel segment **202** and/or the relative flow rates of the fluids in the channel segment **202** and the separate channel. In some instances, the beads can be introduced into the channel segment **202** from a plurality of different channels, and the frequency controlled accordingly. In some instances, the aqueous fluid **208** in the channel segment **202** can comprise biological particles. In some instances, the aqueous fluid **208** can have a substantially uniform concentration or frequency of biological particles. As with the beads, the biological particles can be introduced into the channel segment **202** from a separate channel. The frequency or concentration of the biological particles in the aqueous fluid **208** in the channel segment **202** may be controlled by controlling the frequency in which the biological particles are introduced into the channel segment **202** and/or the relative flow rates of the fluids in the channel segment **202** and the separate channel. In some instances, the biological particles can be introduced into the channel segment **202** from a plurality of different channels, and the frequency controlled accordingly. In some instances, a first separate channel can introduce beads and a second separate channel can introduce biological particles into the channel segment **202.** The first separate channel introducing the beads may be upstream or downstream of the second separate channel introducing the biological particles. The second fluid **210** can comprise an oil, such as a fluorinated oil, that includes a fluorosurfactant for stabilizing the resulting droplets, for example, inhibiting subsequent coalescence of the resulting droplets.

In some instances, the second fluid **210** may not be subjected to and/or directed to any flow in or out of the reservoir **204.** For example, the second fluid **210** may be substantially stationary in the reservoir **204.** In some instances, the second fluid **210** may be subjected to flow within the reservoir **204,** but not in or out of the reservoir **204,** such as via application of pressure to the reservoir **204** and/or as affected by the incoming flow of the aqueous fluid **208** at the junction **206.** Alternatively, the second fluid **210** may be subjected and/or directed to flow in or out of the reservoir **204.** For example, the reservoir **204** can be a channel directing the second fluid **210** from upstream to downstream, transporting the generated droplets. Systems and methods for controlled partitioning are described further in PCT/US2018/047551.

### D. Cell Beads

In another aspect, in addition to or as an alternative to droplet-based partitioning, a biological particle (e.g., cell, or components or macromolecular constituents derived from a cell) may be encapsulated within a particulate material to form a cell bead.

A cell bead can contain biological particle (e.g., a cell) or macromolecular constituents (e.g., RNA, DNA, proteins, etc.) of a biological particle. A cell bead may include a single cell or multiple cells, or a derivative of the single cell or multiple cells. For example after lysing and washing the cells, inhibitory components from cell lysates can be washed away and the macromolecular constituents can be bound as cell beads. Systems and methods disclosed herein can be applicable to both cell beads (and/or droplets or other partitions) containing biological particles and cell beads (and/or droplets or other partitions) containing macromolecular constituents of biological particles. Cell beads may be or include a cell, cell derivative, cellular material and/or material derived from the cell in, within, or encased in a matrix, such as a polymeric matrix. In some cases, a cell bead may comprise a live cell. In some instances, the live cell may be capable of being cultured when enclosed in a gel or polymer matrix, or of being cultured when comprising a gel or polymer matrix. In some instances, the polymer or gel may be diffusively permeable to certain components and diffusively impermeable to other components (e.g., macromolecular constituents). *See* for example U.S Patent No. 10428325.

Cell beads can provide certain potential advantages of being more storable and more portable than droplet-based partitioned biological particles. Furthermore, in some cases, it may be desirable to allow biological particles to incubate for a select period of time before analysis, such as in order to characterize changes in such biological particles over time, either in the presence or absence of different stimuli (or reagents). Suitable polymers or gels may include one or more of disulfide cross-linked polyacrylamide, agarose, alginate, polyvinyl alcohol, polyethylene glycol (PEG)-diacrylate, PEG-acrylate, PEG-thiol, PEG-azide, PEG-alkyne, other acrylates, chitosan, hyaluronic acid, collagen, fibrin, gelatin, or elastin. The polymer or gel may comprise any other polymer or gel.

Encapsulation of biological particles may be performed by a variety of processes. Such processes may combine an aqueous fluid containing the biological particles with a polymeric precursor material that may be capable of being formed into a gel or other solid or semi-solid matrix upon application of a particular stimulus to the polymer precursor. The conditions sufficient to polymerize or gel the precursors may comprise any conditions sufficient to polymerize or gel the precursors. Such stimuli can include, for example, thermal stimuli (e.g., either heating or cooling), photo-stimuli (e.g., through photo-curing), chemical stimuli (e.g., through crosslinking, polymerization initiation of the precursor (e.g., through added initiators)), electromagnetic radiation, mechanical stimuli, or any combination thereof.

In some cases, air knife droplet or aerosol generators may be used to dispense droplets of precursor fluids into gelling solutions in order to form microcapsules that include individual biological particles or small groups of biological particles. Likewise, membrane-based encapsulation systems may be used to generate microcapsules comprising encapsulated biological particles as described herein. Microfluidic systems of the present disclosure, such as that shown in **FIG. 1****,** may be readily used in encapsulating biological particles (e.g., cells) as described herein. Exemplary methods for encapsulating biological particles (e.g., cells) are also further described in U.S. Patent Application Pub. No. US 2015/0376609 and PCT/US2018/016019. In particular, and with reference to **FIG. 1****,** the aqueous fluid **112** comprising (i) the biological particles **114** and (ii) the polymer precursor material (not shown) is flowed into channel junction **110,** where it is partitioned into droplets **118, 120** through the flow of non-aqueous fluid **116.** In the case of encapsulation methods, non-aqueous fluid **116** may also include an initiator (not shown) to cause polymerization and/or crosslinking of the polymer precursor to form the microcapsule that includes the entrained biological particles. Examples of polymer precursor/initiator pairs include those described in U.S. Patent Application Publication No. 2014/0378345.

In some cases, encapsulated biological particles can be selectively releasable from the microcapsule, such as through passage of time or upon application of a particular stimulus, that degrades the microcapsule sufficiently to allow the biological particles (e.g., cell), or its other contents to be released from the microcapsule, such as into a partition (e.g., droplet). Exemplary stimuli suitable for degradation of the microcapsule are described in U.S. Patent Application Publication No. 2014/0378345.

The polymer or gel may be diffusively permeable to chemical or biochemical reagents. The polymer or gel may be diffusively impermeable to macromolecular constituents of the biological particle. In this manner, the polymer or gel may act to allow the biological particle to be subjected to chemical or biochemical operations while spatially confining the macromolecular constituents to a region of the droplet defined by the polymer or gel.

The polymer or gel may be functionalized to bind to targeted analytes, such as nucleic acids, proteins, carbohydrates, lipids or other analytes. The polymer or gel may be polymerized or gelled via a passive mechanism. The polymer or gel may be stable in alkaline conditions or at elevated temperature. The polymer or gel may have mechanical properties similar to the mechanical properties of the bead. For instance, the polymer or gel may be of a similar size to the bead. The polymer or gel may have a mechanical strength (e.g. tensile strength) similar to that of the bead. The polymer or gel may be of a lower density than an oil. The polymer or gel may be of a density that is roughly similar to that of a buffer. The polymer or gel may have a tunable pore size. The pore size may be chosen to, for instance, retain denatured nucleic acids. The pore size may be chosen to maintain diffusive permeability to exogenous chemicals such as sodium hydroxide (NaOH) and/or endogenous chemicals such as inhibitors. The polymer or gel may be biocompatible. The polymer or gel may maintain or enhance cell viability. The polymer or gel may be biochemically compatible. The polymer or gel may be polymerized and/or depolymerized thermally, chemically, enzymatically, and/or optically.

The encapsulation of biological particles may constitute the partitioning of the biological particles into which other reagents are co-partitioned. Alternatively or in addition, encapsulated biological particles may be readily deposited into other partitions (e.g., droplets) as described above.

### E. Beads

Nucleic acid barcode molecules may be delivered to a partition (e.g., a droplet or well) via a solid support or carrier (e.g., a bead). In some cases, nucleic acid barcode molecules are initially associated with the solid support and then released from the solid support upon application of a stimulus, which allows the nucleic acid barcode molecules to dissociate or to be released from the solid support. In specific examples, nucleic acid barcode molecules are initially associated with the solid support (e.g., bead) and then released from the solid support upon application of a biological stimulus, a chemical stimulus, a thermal stimulus, an electrical stimulus, a magnetic stimulus, and/or a photo stimulus.

The solid support may be a bead. A solid support, e.g., a bead, may be porous, non-porous, hollow (e.g., a microcapsule), solid, semi-solid, and/or a combination thereof. Beads may be solid, semi-solid, semi-fluidic, fluidic, and/or a combination thereof. In some instances, a solid support, e.g., a bead, may be at least partially dissolvable, disruptable, and/or degradable. In some cases, a solid support, e.g., a bead, may not be degradable. In some cases, the solid support, e.g., a bead, may be a gel bead. A gel bead may be a hydrogel bead. A gel bead may be formed from molecular precursors, such as a polymeric or monomeric species. A semi-solid support, e.g., a bead, may be a liposomal bead. Solid supports, e.g., beads, may comprise metals including iron oxide, gold, and silver. In some cases, the solid support, e.g., the bead, may be a silica bead. In some cases, the solid support, e.g., a bead, can be rigid. In other cases, the solid support, e.g., a bead, may be flexible and/or compressible.

A partition may comprise one or more unique identifiers, such as barcodes. Barcodes may be previously, subsequently or concurrently delivered to the partitions that hold the compartmentalized or partitioned biological particle. For example, barcodes may be injected into droplets or deposited in microwells previous to, subsequent to, or concurrently with droplet generation or providing of reagents in the microwells, respectively. The delivery of the barcodes to a particular partition allows for the later attribution of the characteristics of the individual biological particle to the particular partition. Barcodes may be delivered, for example on a nucleic acid molecule (e.g., an oligonucleotide), to a partition via any suitable mechanism. Nucleic acid barcode molecules can be delivered to a partition via a microcapsule. A microcapsule, in some instances, can comprise a bead. Beads are described in further detail below.

In some cases, nucleic acid barcode molecules can be initially associated with the microcapsule and then released from the microcapsule. Release of the nucleic acid barcode molecules can be passive (e.g., by diffusion out of the microcapsule). In addition or alternatively, release from the microcapsule can be upon application of a stimulus which allows the barcoded nucleic acid nucleic acid molecules to dissociate or to be released from the microcapsule. Such stimulus may disrupt the microcapsule, an interaction that couples the nucleic acid barcode molecules to or within the microcapsule, or both. Such stimulus can include, for example, a thermal stimulus, photo-stimulus, chemical stimulus (e.g., change in pH or use of a reducing agent(s)), a mechanical stimulus, a radiation stimulus; a biological stimulus (e.g., enzyme), or any combination thereof.

Methods and systems for partitioning barcode carrying beads into droplets are provided herein, and in in US. Patent Publication Nos. 2019/0367997 and 2019/0064173, and International Application No. PCT/US20/17785.

A bead may be porous, non-porous, solid, semi-solid, semi-fluidic, fluidic, and/or a combination thereof. In some instances, a bead may be dissolvable, disruptable, and/or degradable. Degradable beads, as well as methods for degrading beads, are described in PCT/US2014/044398. In some cases, any combination of stimuli, e.g., stimuli described in PCT/US2014/044398 and US Patent Application Pub. No. 2015/0376609, may trigger degradation of a bead. For example, a change in pH may enable a chemical agent (e.g., DTT) to become an effective reducing agent.

In some cases, a bead may not be degradable. In some cases, the bead may be a gel bead. A gel bead may be a hydrogel bead. A gel bead may be formed from molecular precursors, such as a polymeric or monomeric species. A semi-solid bead may be a liposomal bead. Solid beads may comprise metals including iron oxide, gold, and silver. In some cases, the bead may be a silica bead. In some cases, the bead can be rigid. In other cases, the bead may be flexible and/or compressible. A bead may be of any suitable shape. Examples of bead shapes include, but are not limited to, spherical, non-spherical, oval, oblong, amorphous, circular, cylindrical, and variations thereof.

Beads may be of uniform size or heterogeneous size. In some cases, the diameter of a bead may be at least about 10 nanometers (nm), 100 nm, 500 nm, 1 micrometer (µm), 5µm, 10µm, 20µm, 30µm, 40µm, 50µm, 60µm, 70µm, 80µm, 90µm, 100µm, 250µm, 500µm, 1mm, or greater. In some cases, a bead may have a diameter of less than about 10 nm, 100 nm, 500 nm, 1µm, 5µm, 10µm, 20µm, 30µm, 40µm, 50µm, 60µm, 70µm, 80µm, 90µm, 100µm, 250µm, 500µm, 1mm, or less. In some cases, a bead may have a diameter in the range of about 40-75µm, 30-75µm, 20-75µm, 40-85µm, 40-95µm, 20-100µm, 10-100µm, 1-100µm, 20-250µm, or 20-500µm.

In certain aspects, beads can be provided as a population or plurality of beads having a relatively monodisperse size distribution. Where it may be desirable to provide relatively consistent amounts of reagents within partitions, maintaining relatively consistent bead characteristics, such as size, can contribute to the overall consistency. In particular, the beads described herein may have size distributions that have a coefficient of variation in their cross-sectional dimensions of less than 50%, less than 40%, less than 30%, less than 20%, and in some cases less than 15%, less than 10%, less than 5%, or less.

A bead may comprise natural and/or synthetic materials. For example, a bead can comprise a natural polymer, a synthetic polymer or both natural and synthetic polymers. *See,* e.g., PCT/US2014/044398. Beads may also be formed from materials other than polymers, including lipids, micelles, ceramics, glass-ceramics, material composites, metals, other inorganic materials, and others.

In some cases, the bead may comprise covalent or ionic bonds between polymeric precursors (e.g., monomers, oligomers, linear polymers), nucleic acid molecules (e.g., oligonucleotides), primers, and other entities. In some cases, the covalent bonds can be carboncarbon bonds, thioether bonds, or carbon-heteroatom bonds.

Activation of disulfide linkages within a bead can be controlled such that only a small number of disulfide linkages are activated. Methods of controlling activation of disulfide linkages within a bead are described in PCT/US2014/044398.

In some cases, a bead may comprise an acrydite moiety, which in certain aspects may be used to attach one or more nucleic acid molecules (e.g., barcode sequence, nucleic acid barcode molecule, barcoded oligonucleotide, primer, or other oligonucleotide) to the bead. Acrydite moieties, as well as their uses in attaching nucleic acid molecules to beads, are described in PCT/US2014/044398.

For example, precursors (e.g., monomers, cross-linkers) that are polymerized to form a bead may comprise acrydite moieties, such that when a bead is generated, the bead also comprises acrydite moieties. The acrydite moieties can be attached to a nucleic acid molecule described herein.

In some cases, precursors comprising a functional group that is reactive or capable of being activated such that it becomes reactive can be polymerized with other precursors to generate gel beads comprising the activated or activatable functional group. The functional group may then be used to attach additional species (e.g., disulfide linkers, primers, other oligonucleotides, etc.) to the gel beads. Exemplary precursors comprising functional groups are described in PCT/US2014/044398. Other non- limiting examples of labile bonds that may be coupled to a precursor or bead are described in PCT/US2014/044398. A bond may be cleavable via other nucleic acid molecule targeting enzymes, such as restriction enzymes (e.g., restriction endonucleases), as described further below.

Species may be encapsulated in beads during bead generation (e.g., during polymerization of precursors). Such species may or may not participate in polymerization. See, e.g., PCT/US2014/044398. Such species may include, for example, nucleic acid molecules (e.g., oligonucleotides), reagents for a nucleic acid amplification reaction (e.g., primers, polymerases, dNTPs, co-factors (e.g., ionic co-factors), buffers) including those described herein, reagents for enzymatic reactions (e.g., enzymes, co-factors, substrates, buffers), reagents for nucleic acid modification reactions such as polymerization, ligation, or digestion, and/or reagents for template preparation (e.g., tagmentation) for one or more sequencing platforms (e.g., Nextera^{®} for Illumina^{®}). Such species may include one or more enzymes described herein, including without limitation, polymerase, reverse transcriptase, restriction enzymes (e.g., endonuclease), transposase, ligase, proteinase K, DNAse, etc. Such species may include one or more reagents described elsewhere herein (e.g., lysis agents, inhibitors, inactivating agents, chelating agents, stimulus). Alternatively or in addition, species may be partitioned in a partition (e.g., droplet) during or subsequent to partition formation. Such species may include, without limitation, the abovementioned species that may also be encapsulated in a bead.

In some cases, beads can be non-covalently loaded with one or more reagents. The beads can be non-covalently loaded by, for instance, subjecting the beads to conditions sufficient to swell the beads, allowing sufficient time for the reagents to diffuse into the interiors of the beads, and subjecting the beads to conditions sufficient to de-swell the beads. The swelling of the beads may be accomplished, for instance, by placing the beads in a thermodynamically favorable solvent, subjecting the beads to a higher or lower temperature, subjecting the beads to a higher or lower ion concentration, and/or subjecting the beads to an electric field. The swelling of the beads may be accomplished by various swelling methods. The de-swelling of the beads may be accomplished, for instance, by transferring the beads in a thermodynamically unfavorable solvent, subjecting the beads to lower or high temperatures, subjecting the beads to a lower or higher ion concentration, and/or removing an electric field. The de-swelling of the beads may be accomplished by various de-swelling methods. Transferring the beads may cause pores in the bead to shrink. The shrinking may then hinder reagents within the beads from diffusing out of the interiors of the beads. The hindrance may be due to steric interactions between the reagents and the interiors of the beads. The transfer may be accomplished microfluidically. For instance, the transfer may be achieved by moving the beads from one co-flowing solvent stream to a different co-flowing solvent stream. The swellability and/or pore size of the beads may be adjusted by changing the polymer composition of the bead.

Any suitable number of molecular tag molecules (e.g., primer, barcoded oligonucleotide) can be associated with a bead such that, upon release from the bead, the molecular tag molecules (e.g., primer, e.g., barcoded oligonucleotide) are present in the partition at a pre-defined concentration. Such pre-defined concentration may be selected to facilitate certain reactions for generating a sequencing library, e.g., amplification, within the partition. In some cases, the pre-defined concentration of the primer can be limited by the process of producing oligonucleotide bearing beads.

### F. Nucleic acid barcode molecules

A nucleic acid barcode molecule may contain one or more barcode sequences. A plurality of nucleic acid barcode molecules may be coupled to a bead. The one or more barcode sequences may include sequences that are the same for all nucleic acid molecules coupled to a given bead and/or sequences that are different across all nucleic acid molecules coupled to the given bead. The nucleic acid molecule may be incorporated into the bead.

Nucleic acid barcode molecules can comprise one or more functional sequences for coupling to an analyte or analyte tag such as a reporter oligonucleotide. Such functional sequences can include, e.g., a template switch oligonucleotide (TSO) sequence, a primer sequence (e.g., a poly T sequence, or a nucleic acid primer sequence complementary to a target nucleic acid sequence and/or for amplifying a target nucleic acid sequence, a random primer, and a primer sequence for messenger RNA).

In some cases, the nucleic acid molecule can further comprise a unique molecular identifier (UMI). In some cases, the nucleic acid barcode molecule can comprise one or more functional sequences, for example, for attachment to a sequencing flow cell, such as, for example, a P5 sequence (or a portion thereof) for Illumina^{®} sequencing. In some cases, the nucleic acid barcode molecule or derivative thereof (e.g., oligonucleotide or polynucleotide generated from the nucleic acid molecule) can comprise another functional sequence, such as, for example, a P7 sequence (or a portion thereof) for attachment to a sequencing flow cell for Illumina sequencing. In some cases, the nucleic acid molecule can comprise an R1 primer sequence for Illumina sequencing. In some cases, the nucleic acid molecule can comprise an R2 primer sequence for Illumina sequencing. In some cases, a functional sequence can comprise a partial sequence, such as a partial barcode sequence, partial anchoring sequence, partial sequencing primer sequence (e.g., partial R1 sequence, partial R2 sequence, etc.), a partial sequence configured to attach to the flow cell of a sequencer (e.g., partial P5 sequence, partial P7 sequence, etc.), or a partial sequence of any other type of sequence described elsewhere herein. A partial sequence may contain a contiguous or continuous portion or segment, but not all, of a full sequence, for example. In some cases, a downstream procedure may extend the partial sequence, or derivative thereof, to achieve a full sequence of the partial sequence, or derivative thereof.

Examples of such nucleic acid molecules (e.g., oligonucleotides, polynucleotides, etc.) and uses thereof, as may be used with compositions, devices, methods and systems of the present disclosure, are provided in U.S. Patent Pub. Nos. 2014/0378345 and 2015/0376609.

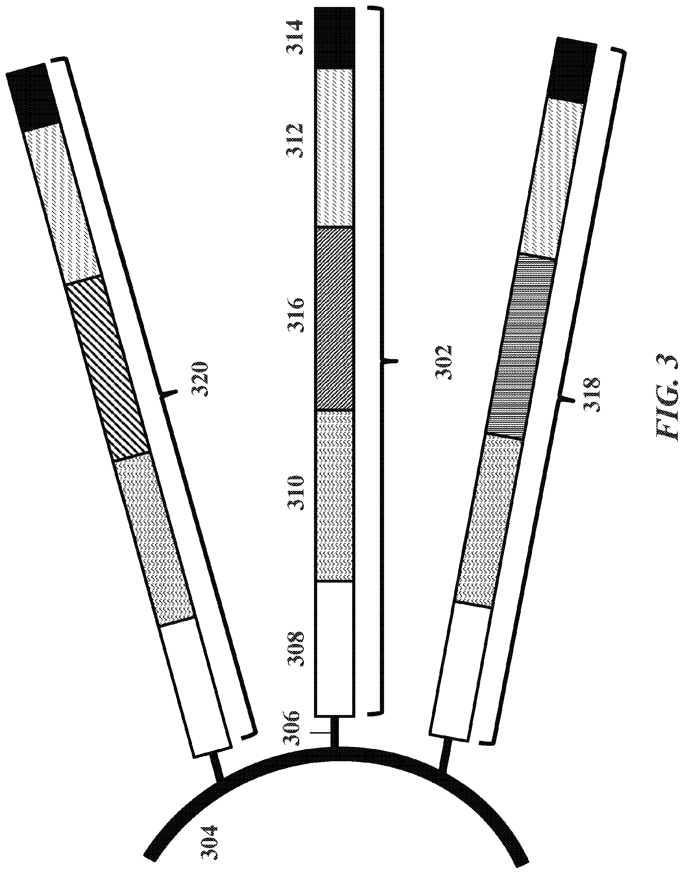

**FIG. 3** illustrates an example of a barcode carrying bead. A nucleic acid molecule **302,** such as an oligonucleotide, can be coupled to a bead **304** by a releasable linkage **306,** such as, for example, a disulfide linker. The same bead **304** may be coupled (e.g., via releasable linkage) to one or more other nucleic acid molecules **318, 320.** The nucleic acid molecule **302** may be or comprise a barcode. As noted elsewhere herein, the structure of the barcode may comprise a number of sequence elements. The nucleic acid molecule **302** may comprise a functional sequence **308** that may be used in subsequent processing. For example, the functional sequence **308** may include one or more of a sequencer specific flow cell attachment sequence (e.g., a P5 sequence for Illumina^{®} sequencing systems) and a sequencing primer sequence (e.g., a R1 primer for Illumina^{®} sequencing systems), or partial sequence(s) thereof. The nucleic acid molecule **302** may comprise a barcode sequence **310** for use in barcoding the sample (e.g., DNA, RNA, protein, etc.). In some cases, the barcode sequence **310** can be bead-specific such that the barcode sequence **310** is common to all nucleic acid molecules (e.g., including nucleic acid molecule **302**) coupled to the same bead **304.** Alternatively or in addition, the barcode sequence **310** can be partition-specific such that the barcode sequence **310** is common to all nucleic acid molecules coupled to one or more beads that are partitioned into the same partition. The nucleic acid molecule **302** may comprise sequence **312** complementary to an analyte of interest, e.g., a priming sequence. Sequence **312** can be a poly-T sequence complementary to a poly-A tail of an mRNA analyte, a targeted priming sequence, and/or a random priming sequence. The nucleic acid molecule **302** may comprise an anchoring sequence **314** to ensure that the specific priming sequence **312** hybridizes at the sequence end (e.g., of the mRNA). For example, the anchoring sequence **314** can include a random short sequence of nucleotides, such as a 1-mer, 2-mer, 3-mer or longer sequence, which can ensure that a poly-T segment is more likely to hybridize at the sequence end of the poly-A tail of the mRNA.

The nucleic acid molecule **302** may comprise a unique molecular identifying sequence **316** (e.g., unique molecular identifier (UMI)). In some cases, the unique molecular identifying sequence **316** may comprise from about 5 to about 8 nucleotides. Alternatively, the unique molecular identifying sequence **316** may compress less than about 5 or more than about 8 nucleotides. The unique molecular identifying sequence **316** may be a unique sequence that varies across individual nucleic acid barcode molecules (e.g., **302, 318, 320,** etc.) coupled to a single bead (e.g., bead **304**). In some cases, the unique molecular identifying sequence **316** may be a random sequence (e.g., such as a random N-mer sequence). For example, the UMI may provide a unique identifier of the starting analyte (e.g., mRNA) molecule that was captured, in order to allow quantitation of the number of original expressed RNA molecules. As will be appreciated, although **FIG. 3** shows three nucleic acid molecules **302, 318, 320** coupled to the surface of the bead **304,** an individual bead may be coupled to any number of individual nucleic acid molecules, for example, from one to tens to hundreds of thousands, millions, or even a billion of individual nucleic acid molecules. The respective barcodes for the individual nucleic acid molecules can comprise both common sequence segments or relatively common sequence segments (e.g., **308, 310, 312,** etc.) and variable or unique sequence segments (e.g., **316**) between different individual nucleic acid molecules coupled to the same bead.

In operation, a biological particle (e.g., cell, DNA, RNA, etc.) can be co-partitioned along with a barcode bearing bead **304.** The nucleic acid barcode molecules **302, 318, 320** can be released from the bead **304** in the partition. By way of example, in the context of analyzing sample RNA, the poly-T segment (e.g., **312**) of one of the released nucleic acid molecules (e.g., **302**) can hybridize to the poly-A tail of a mRNA molecule. Reverse transcription may result in a cDNA transcript of the mRNA, but which transcript includes each of the sequence segments **308, 310, 316** of the nucleic acid molecule **302.** Because the nucleic acid molecule **302** comprises an anchoring sequence **314,** it will more likely hybridize to and prime reverse transcription at the sequence end of the poly-A tail of the mRNA. Within any given partition, all of the cDNA transcripts of the individual mRNA molecules may include a common barcode sequence segment **310.** However, the transcripts made from the different mRNA molecules within a given partition may vary at the unique molecular identifying sequence **312** segment (e.g., UMI segment). Beneficially, even following any subsequent amplification of the contents of a given partition, the number of different UMIs can be indicative of the quantity of mRNA originating from a given partition, and thus from the biological particle (e.g., cell). As noted above, the transcripts can be amplified, cleaned up and sequenced to identify the sequence of the cDNA transcript of the mRNA, as well as to sequence the barcode segment and the UMI segment. While a poly-T primer sequence is described, other targeted or random priming sequences may also be used in priming the reverse transcription reaction. Likewise, although described as releasing the barcoded oligonucleotides into the partition, in some cases, the nucleic acid molecules bound to the bead (e.g., gel bead) may be used to hybridize and capture the mRNA on the solid phase of the bead, for example, in order to facilitate the separation of the RNA from other cell contents. In such cases, further processing may be performed, in the partitions or outside the partitions (e.g., in bulk). For instance, the RNA molecules on the beads may be subjected to reverse transcription or other nucleic acid processing, additional adapter sequences may be added to the barcoded nucleic acid molecules, or other nucleic acid reactions (e.g., amplification, nucleic acid extension) may be performed. The beads or products thereof (e.g., barcoded nucleic acid molecules) may be collected from the partitions, and/or pooled together and subsequently subjected to clean up and further characterization (e.g., sequencing).

The operations described herein may be performed at any useful or convenient step. For instance, the beads comprising nucleic acid barcode molecules may be introduced into a partition (e.g., well or droplet) prior to, during, or following introduction of a sample into the partition. The nucleic acid molecules of a sample may be subjected to barcoding, which may occur on the bead (in cases where the nucleic acid molecules remain coupled to the bead) or following release of the nucleic acid barcode molecules into the partition. In cases where analytes from the sample are captured by the nucleic acid barcode molecules in a partition (e.g., by hybridization), captured analytes from various partitions may be collected, pooled, and subjected to further processing (e.g., reverse transcription, adapter attachment, amplification, clean up, sequencing). For example, in cases wherein the nucleic acid molecules from the sample remain attached to the bead, the beads from various partitions may be collected, pooled, and subjected to further processing (e.g., reverse transcription, adapter attachment, amplification, clean up, sequencing). In other instances, the processing may occur in the partition. For example, conditions sufficient for barcoding, adapter attachment, reverse transcription, or other nucleic acid processing operations may be provided in the partition and performed prior to clean up and sequencing.

In some instances, a bead may comprise a capture sequence or binding sequence configured to bind to a corresponding capture sequence or binding sequence. In some instances, a bead may comprise a plurality of different capture sequences or binding sequences configured to bind to different respective corresponding capture sequences or binding sequences. For example, a bead may comprise a first subset of one or more capture sequences each configured to bind to a first corresponding capture sequence, a second subset of one or more capture sequences each configured to bind to a second corresponding capture sequence, a third subset of one or more capture sequences each configured to bind to a third corresponding capture sequence, and etc. A bead may comprise any number of different capture sequences. In some instances, a bead may comprise at least 2, 3, 4, 5, 6, 7, 8, 9, 10 or more different capture sequences or binding sequences configured to bind to different respective capture sequences or binding sequences, respectively. Alternatively or in addition, a bead may comprise at most about 10, 9, 8, 7, 6, 5, 4, 3, or 2 different capture sequences or binding sequences configured to bind to different respective capture sequences or binding sequences. In some instances, the different capture sequences or binding sequences may be configured to facilitate analysis of a same type of analyte. In some instances, the different capture sequences or binding sequences may be configured to facilitate analysis of different types of analytes (with the same bead). The capture sequence may be designed to attach to a corresponding capture sequence. Beneficially, such corresponding capture sequence may be introduced to, or otherwise induced in, a biological particle (e.g., cell, cell bead, etc.) for performing different assays in various formats (e.g., barcoded antibodies comprising the corresponding capture sequence, barcoded MHC dextramers comprising the corresponding capture sequence, barcoded guide RNA molecules comprising the corresponding capture sequence, etc.), such that the corresponding capture sequence may later interact with the capture sequence associated with the bead. In some instances, a capture sequence coupled to a bead (or other support) may be configured to attach to a linker molecule, such as a splint molecule, wherein the linker molecule is configured to couple the bead (or other support) to other molecules through the linker molecule, such as to one or more analytes or one or more other linker molecules.

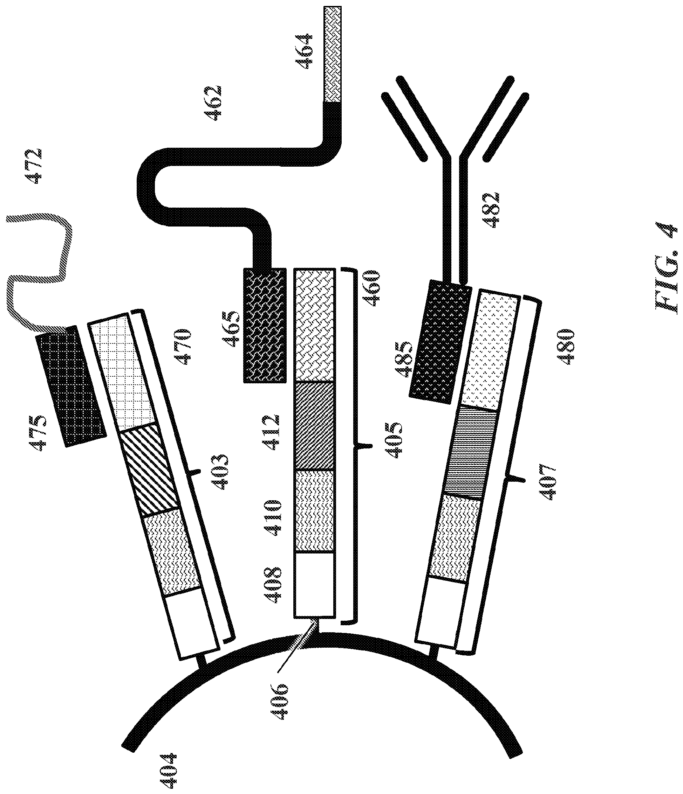

**FIG. 4** illustrates another example of a barcode carrying bead. A nucleic acid molecule **405,** such as an oligonucleotide, can be coupled to a bead **404** by a releasable linkage **406,** such as, for example, a disulfide linker. The nucleic acid molecule **405** may comprise a first capture sequence **460.** The same bead **404** may be coupled (e.g., via releasable linkage) to one or more other nucleic acid molecules **403, 407** comprising other capture sequences. The nucleic acid molecule **405** may be or comprise a barcode. As noted elsewhere herein, the structure of the barcode may comprise a number of sequence elements, such as a functional sequence **408** (e.g., flow cell attachment sequence, sequencing primer sequence, etc.), a barcode sequence **410** (e.g., bead-specific sequence common to bead, partition-specific sequence common to partition, etc.), and a unique molecular identifier **412** (e.g., unique sequence within different molecules attached to the bead), or partial sequences thereof. The capture sequence **460** may be configured to attach to a corresponding capture sequence **465.** In some instances, the corresponding capture sequence **465** may be coupled to another molecule that may be an analyte or an intermediary carrier. For example, as illustrated in **FIG. 4****,** the corresponding capture sequence **465** is coupled to a guide RNA molecule **462** comprising a target sequence **464,** wherein the target sequence **464** is configured to attach to the analyte. Another oligonucleotide molecule **407** attached to the bead **404** comprises a second capture sequence **480** which is configured to attach to a second corresponding capture sequence **485.** As illustrated in **FIG. 4****,** the second corresponding capture sequence **485** is coupled to an antibody **482.** In some cases, the antibody **482** may have binding specificity to an analyte (e.g., surface protein). Alternatively, the antibody **482** may not have binding specificity. Another oligonucleotide molecule **403** attached to the bead **404** comprises a third capture sequence **470** which is configured to attach to a second corresponding capture sequence **475.** As illustrated in **FIG. 4****,** the third corresponding capture sequence **475** is coupled to a molecule **472.** The molecule **472** may or may not be configured to target an analyte. The other oligonucleotide molecules **403, 407** may comprise the other sequences (e.g., functional sequence, barcode sequence, UMI, etc.) described with respect to oligonucleotide molecule **405.** While a single oligonucleotide molecule comprising each capture sequence is illustrated in **FIG. 4****,** it will be appreciated that, for each capture sequence, the bead may comprise a set of one or more oligonucleotide molecules each comprising the capture sequence. For example, the bead may comprise any number of sets of one or more different capture sequences. Alternatively or in addition, the bead **404** may comprise other capture sequences. Alternatively or in addition, the bead **404** may comprise fewer types of capture sequences (e.g., two capture sequences). Alternatively or in addition, the bead **404** may comprise oligonucleotide molecule(s) comprising a priming sequence, such as a specific priming sequence such as an mRNA specific priming sequence (e.g., poly-T sequence), a targeted priming sequence, and/or a random priming sequence, for example, to facilitate an assay for gene expression.

In operation, the barcoded oligonucleotides may be released (e.g., in a partition), as described elsewhere herein. Alternatively, the nucleic acid molecules bound to the bead (e.g., gel bead) may be used to hybridize and capture analytes (e.g., one or more types of analytes) on the solid phase of the bead. A bead injected or otherwise introduced into a partition may comprise releasably, cleavably, or reversibly attached barcodes. A bead injected or otherwise introduced into a partition may comprise activatable barcodes. A bead injected or otherwise introduced into a partition may be degradable, disruptable, or dissolvable beads.

Barcodes can be releasably, cleavably or reversibly attached to the beads such that barcodes can be released or be releasable through cleavage of a linkage between the barcode molecule and the bead, or released through degradation of the underlying bead itself, allowing the barcodes to be accessed or be accessible by other reagents, or both. In non-limiting examples, cleavage may be achieved through reduction of di-sulfide bonds, use of restriction enzymes, photo-activated cleavage, or cleavage via other types of stimuli (e.g., chemical, thermal, pH, enzymatic, etc.) and/or reactions, such as described elsewhere herein. Releasable barcodes may sometimes be referred to as being activatable, in that they are available for reaction once released. Thus, for example, an activatable barcode may be activated by releasing the barcode from a bead (or other suitable type of partition described herein). Other activatable configurations are also envisioned in the context of the described methods and systems.

As will be appreciated from the above disclosure, the degradation of a bead may refer to the disassociation of a bound or entrained species from a bead, both with and without structurally degrading the physical bead itself. For example, the degradation of the bead may involve cleavage of a cleavable linkage via one or more species and/or methods described elsewhere herein. In another example, entrained species may be released from beads through osmotic pressure differences due to, for example, changing chemical environments. See, e.g., PCT/US2014/044398.

A degradable bead may be introduced into a partition, such as a droplet of an emulsion or a well, such that the bead degrades within the partition and any associated species (e.g., oligonucleotides) are released within the droplet when the appropriate stimulus is applied. The free species (e.g., oligonucleotides, nucleic acid molecules) may interact with other reagents contained in the partition. See, e.g., PCT/US2014/044398.

As will be appreciated, barcodes that are releasably, cleavably or reversibly attached to the beads described herein include barcodes that are released or releasable through cleavage of a linkage between the barcode molecule and the bead, or that are released through degradation of the underlying bead itself, allowing the barcodes to be accessed or accessible by other reagents, or both.

In some cases, a species (e.g., oligonucleotide molecules comprising barcodes) that are attached to a solid support (e.g., a bead) may comprise a U-excising element that allows the species to release from the bead. In some cases, the U-excising element may comprise a singlestranded DNA (ssDNA) sequence that contains at least one uracil. The species may be attached to a solid support via the ssDNA sequence containing the at least one uracil. The species may be released by a combination of uracil-DNA glycosylase (e.g., to remove the uracil) and an endonuclease (e.g., to induce an ssDNA break). If the endonuclease generates a 5' phosphate group from the cleavage, then additional enzyme treatment may be included in downstream processing to eliminate the phosphate group, e.g., prior to ligation of additional sequencing handle elements, e.g., Illumina full P5 sequence, partial P5 sequence, full R1 sequence, and/or partial R1 sequence.

The barcodes that are releasable as described herein may sometimes be referred to as being activatable, in that they are available for reaction once released. Thus, for example, an activatable barcode may be activated by releasing the barcode from a bead (or other suitable type of partition described herein). Other activatable configurations are also envisioned in the context of the described methods and systems.

The nucleic acid barcode sequences can include from about 6 to about 20 or more nucleotides within the sequence of the nucleic acid molecules (e.g., oligonucleotides). The nucleic acid barcode sequences can include from about 6 to about 20, 30, 40, 50, 60, 70, 80, 90, 100 or more nucleotides. In some cases, the length of a barcode sequence may be about 6, 7, 8, 9, 10, 11, 12, 13, 14, 15, 16, 17, 18, 19, 20 nucleotides or longer. In some cases, the length of a barcode sequence may be at least about 6, 7, 8, 9, 10, 11, 12, 13, 14, 15, 16, 17, 18, 19, 20 nucleotides or longer. In some cases, the length of a barcode sequence may be at most about 6, 7, 8, 9, 10, 11, 12, 13, 14, 15, 16, 17, 18, 19, 20 nucleotides or shorter. These nucleotides may be completely contiguous, i.e., in a single stretch of adjacent nucleotides, or they may be separated into two or more separate subsequences that are separated by 1 or more nucleotides. In some cases, separated barcode subsequences can be from about 4 to about 16 nucleotides in length. In some cases, the barcode subsequence may be about 4, 5, 6, 7, 8, 9, 10, 11, 12, 13, 14, 15, 16 nucleotides or longer. In some cases, the barcode subsequence may be at least about 4, 5, 6, 7, 8, 9, 10, 11, 12, 13, 14, 15, 16 nucleotides or longer. In some cases, the barcode subsequence may be at most about 4, 5, 6, 7, 8, 9, 10, 11, 12, 13, 14, 15, 16 nucleotides or shorter.

The co-partitioned nucleic acid molecules can also comprise other functional sequences useful in the processing of the nucleic acids from the co-partitioned biological particles. These sequences include, e.g., targeted or random/universal amplification primer sequences for amplifying nucleic acids (e.g., mRNA, the genomic DNA) from the individual biological particles within the partitions while attaching the associated barcode sequences, sequencing primers or primer recognition sites, hybridization or probing sequences, e.g., for identification of presence of the sequences or for pulling down barcoded nucleic acids, or any of a number of other potential functional sequences. Other mechanisms of co-partitioning oligonucleotides may also be employed, including, e.g., coalescence of two or more droplets, where one droplet contains oligonucleotides, or microdispensing of oligonucleotides (e.g., attached to a bead) into partitions, e.g., droplets within microfluidic systems.

In an example, microcapsules, such as beads, are provided that each include large numbers of the above described nucleic acid barcode molecules releasably attached to the beads, where all of the nucleic acid barcode molecules attached to a particular bead will include a common nucleic acid barcode sequence, but where a large number of diverse barcode sequences are represented across the population of beads used. In some embodiments, hydrogel beads, e.g., comprising polyacrylamide polymer matrices, are used as a solid support and delivery vehicle for the nucleic acid barcode molecules into the partitions, as they are capable of carrying large numbers of nucleic acid barcode molecules, and may be configured to release those nucleic acid molecules upon exposure to a particular stimulus, as described elsewhere herein. In some cases, the population of beads provides a diverse barcode sequence library that includes at least about 1,000 different barcode sequences, at least about 5,000 different barcode sequences, at least about 10,000 different barcode sequences, at least about 50,000 different barcode sequences, at least about 100,000 different barcode sequences, at least about 1,000,000 different barcode sequences, at least about 5,000,000 different barcode sequences, or at least about 10,000,000 different barcode sequences, or more. In some cases, the population of beads provides a diverse barcode sequence library that includes about 1,000 to about 10,000 different barcode sequences, about 5,000 to about 50,000 different barcode sequences, about 10,000 to about 100,000 different barcode sequences, about 50,000 to about 1,000,000 different barcode sequences, or about 100,000 to about 10,000,000 different barcode sequences.

Additionally, each bead can be provided with large numbers of nucleic acid (e.g., oligonucleotide) molecules attached. In particular, the number of molecules of nucleic acid molecules including the barcode sequence on an individual bead can be at least about 1,000 nucleic acid molecules, at least about 5,000 nucleic acid molecules, at least about 10,000 nucleic acid molecules, at least about 50,000 nucleic acid molecules, at least about 100,000 nucleic acid molecules, at least about 500,000 nucleic acids, at least about 1,000,000 nucleic acid molecules, at least about 5,000,000 nucleic acid molecules, at least about 10,000,000 nucleic acid molecules, at least about 50,000,000 nucleic acid molecules, at least about 100,000,000 nucleic acid molecules, at least about 250,000,000 nucleic acid molecules and in some cases at least about 1 billion nucleic acid molecules, or more. Nucleic acid molecules of a given bead can include identical (or common) barcode sequences, different barcode sequences, or a combination of both. Nucleic acid molecules of a given bead can include multiple sets of nucleic acid molecules. Nucleic acid molecules of a given set can include identical barcode sequences. The identical barcode sequences can be different from barcode sequences of nucleic acid molecules of another set.

Moreover, when the population of beads is partitioned, the resulting population of partitions can also include a diverse barcode library that includes at least about 1,000 different barcode sequences, at least about 5,000 different barcode sequences, at least about 10,000 different barcode sequences, at least at least about 50,000 different barcode sequences, at least about 100,000 different barcode sequences, at least about 1,000,000 different barcode sequences, at least about 5,000,000 different barcode sequences, or at least about 10,000,000 different barcode sequences. Additionally, each partition of the population can include at least about 1,000 nucleic acid molecules, at least about 5,000 nucleic acid molecules, at least about 10,000 nucleic acid molecules, at least about 50,000 nucleic acid molecules, at least about 100,000 nucleic acid molecules, at least about 500,000 nucleic acids, at least about 1,000,000 nucleic acid molecules, at least about 5,000,000 nucleic acid molecules, at least about 10,000,000 nucleic acid molecules, at least about 50,000,000 nucleic acid molecules, at least about 100,000,000 nucleic acid molecules, at least about 250,000,000 nucleic acid molecules and in some cases at least about 1 billion nucleic acid molecules.

In some cases, it may be desirable to incorporate multiple different barcodes within a given partition, either attached to a single or multiple beads within the partition. For example, in some cases, a mixed, but known set of barcode sequences may provide greater assurance of identification in the subsequent processing, e.g., by providing a stronger address or attribution of the barcodes to a given partition, as a duplicate or independent confirmation of the output from a given partition.

The nucleic acid molecules (e.g., oligonucleotides) are releasable from the beads upon the application of a particular stimulus to the beads. In some cases, the stimulus may be a photo-stimulus, e.g., through cleavage of a photo-labile linkage that releases the nucleic acid molecules. In other cases, a thermal stimulus may be used, where elevation of the temperature of the beads environment will result in cleavage of a linkage or other release of the nucleic acid molecules from the beads. In still other cases, a chemical stimulus can be used that cleaves a linkage of the nucleic acid molecules to the beads, or otherwise results in release of the nucleic acid molecules from the beads. In one case, such compositions include the polyacrylamide matrices described above for encapsulation of biological particles, and may be degraded for release of the attached nucleic acid molecules through exposure to a reducing agent, such as DTT.

### G. Reagents

In accordance with certain aspects, biological particles and/or biological particles may be partitioned along with lysis reagents in order to release the contents of the biological particles within the partition. In such cases, the lysis agents can be contacted with the biological particle suspension concurrently with, or immediately prior to, the introduction of the biological particles into the partitioning junction/droplet generation zone (e.g., junction 210), such as through an additional channel or channels upstream of the channel junction. In accordance with other aspects, additionally or alternatively, biological particles may be partitioned along with other reagents, as will be described further below.

The methods and systems of the present disclosure may comprise microfluidic devices and methods of use thereof, which may be used for co-partitioning biological particles with reagents. Such systems and methods are described in U.S. Patent Publication No. US/20190367997. Beneficially, when lysis reagents and biological particles are co-partitioned, the lysis reagents can facilitate the release of the contents of the biological particles within the partition. The contents released in a partition may remain discrete from the contents of other partitions.

As will be appreciated, the channel segments of the microfluidic devices described elsewhere herein may be coupled to any of a variety of different fluid sources or receiving components, including reservoirs, tubing, manifolds, or fluidic components of other systems. As will be appreciated, the microfluidic channel structures may have various geometries and/or configurations. For example, a microfluidic channel structure can have more than two channel junctions. For example, a microfluidic channel structure can have 2, 3, 4, 5 channel segments or more each carrying the same or different types of beads, reagents, and/or biological particles that meet at a channel junction. Fluid flow in each channel segment may be controlled to control the partitioning of the different elements into droplets. Fluid may be directed flow along one or more channels or reservoirs via one or more fluid flow units. A fluid flow unit can comprise compressors (e.g., providing positive pressure), pumps (e.g., providing negative pressure), actuators, and the like to control flow of the fluid. Fluid may also or otherwise be controlled via applied pressure differentials, centrifugal force, electrokinetic pumping, vacuum, capillary or gravity flow, or the like.

Examples of lysis agents include bioactive reagents, such as lysis enzymes that are used for lysis of different cell types, e.g., gram positive or negative bacteria, plants, yeast, mammalian, etc., such as lysozymes, achromopeptidase, lysostaphin, labiase, kitalase, lyticase, and a variety of other lysis enzymes available from, e.g., Sigma-Aldrich, Inc. (St Louis, MO), as well as other commercially available lysis enzymes. Other lysis agents may additionally or alternatively be co-partitioned with the biological particles to cause the release of the biological particle's contents into the partitions. For example, in some cases, surfactant-based lysis solutions may be used to lyse cells, although these may be less desirable for emulsion based systems where the surfactants can interfere with stable emulsions. In some cases, lysis solutions may include non-ionic surfactants such as, for example, TritonX-100 and Tween 20. In some cases, lysis solutions may include ionic surfactants such as, for example, sarcosyl and sodium dodecyl sulfate (SDS). Electroporation, thermal, acoustic or mechanical cellular disruption may also be used in certain cases, e.g., non-emulsion-based partitioning such as encapsulation of biological particles that may be in addition to or in place of droplet partitioning, where any pore size of the encapsulate is sufficiently small to retain nucleic acid fragments of a given size, following cellular disruption.

Alternatively or in addition to the lysis agents co-partitioned with the biological particles or biological particles described above, other reagents can also be co-partitioned with the biological particles, including, for example, DNase and RNase inactivating agents or inhibitors, such as proteinase K, chelating agents, such as EDTA, and other reagents employed in removing or otherwise reducing negative activity or impact of different cell lysate components on subsequent processing of nucleic acids. In addition, in the case of encapsulated biological particles (e.g., a cell or a nucleus in a polymer matrix), the biological particles may be exposed to an appropriate stimulus to release the biological particles or their contents from its encapsulating material. For example, in some cases, a chemical stimulus may be co-partitioned along with an encapsulated biological particle (e.g., cell bead) to allow for the degradation of the cell bead and release of the cell or its contents into the larger partition. In some cases, this stimulus may be the same as the stimulus described elsewhere herein for release of nucleic acid molecules (e.g., oligonucleotides) from their respective bead. In alternative examples, this may be a different and non-overlapping stimulus, in order to allow an encapsulated biological particle to be released into a partition at a different time from the release of nucleic acid molecules into the same partition. For a description of methods, compositions, and systems for encapsulating cells (also referred to as a "cell bead"), see, e.g., U.S. Pat. 10,428,326 and U.S. Pat. Pub. 20190100632.

Additional reagents may also be co-partitioned with the biological particle, such as endonucleases to fragment a biological particle's DNA, DNA polymerase enzymes and dNTPs used to amplify the biological particle's nucleic acid fragments and to attach the barcode molecular tags to the amplified fragments. Other enzymes may be co-partitioned, including without limitation, polymerase, transposase, ligase, proteinase K, DNAse, etc. Additional reagents may also include reverse transcriptase enzymes, including enzymes with terminal transferase activity, primers and oligonucleotides, and switch oligonucleotides (also referred to herein as "switch oligos" or "template switching oligonucleotides") which can be used for template switching.

In some cases, template switching can be used to increase the length of a cDNA. In some cases, template switching can be used to append a predefined nucleic acid sequence to the cDNA. Template switching is further described in PCT/US2017/068320. Template switching oligonucleotides may comprise a hybridization region and a template region. Template switching oligonucleotides are further described in PCT/US2017/068320.

Any of the reagents described in this disclosure may be encapsulated in, or otherwise coupled to, a droplet, or bead, with any chemicals, particles, and elements suitable for sample processing reactions involving biomolecules, such as, but not limited to, nucleic acid molecules and proteins. For example, a bead or droplet used in a sample preparation reaction for DNA sequencing may comprise one or more of the following reagents: enzymes, restriction enzymes (e.g., multiple cutters), ligase, polymerase, fluorophores, oligonucleotide barcodes, adapters, buffers, nucleotides (e.g., dNTPs, ddNTPs) and the like. Additional examples of reagents include, but are not limited to: buffers, acidic solution, basic solution, temperaturesensitive enzymes, pH-sensitive enzymes, light-sensitive enzymes, metals, metal ions, magnesium chloride, sodium chloride, manganese, aqueous buffer, mild buffer, ionic buffer, inhibitor, enzyme, protein, polynucleotide, antibodies, saccharides, lipid, oil, salt, ion, detergents, ionic detergents, non-ionic detergents, and oligonucleotides.

Once the contents of the cells are released into their respective partitions, the macromolecular components (e.g., macromolecular constituents of biological particles, such as RNA, DNA, or proteins) contained therein may be further processed within the partitions. In accordance with the methods and systems described herein, the macromolecular component contents of individual biological particles can be provided with unique identifiers such that, upon characterization of those macromolecular components they may be attributed as having been derived from the same biological particle or particles. The ability to attribute characteristics to individual biological particles or groups of biological particles is provided by the assignment of unique identifiers specifically to an individual biological particle or groups of biological particles. Unique identifiers, e.g., in the form of nucleic acid barcodes can be assigned or associated with individual biological particles or populations of biological particles, in order to tag or label the biological particle's macromolecular components (and as a result, its characteristics) with the unique identifiers. These unique identifiers can then be used to attribute the biological particle's components and characteristics to an individual biological particle or group of biological particles. In some aspects, this is performed by co-partitioning the individual biological particle or groups of biological particles with the unique identifiers, such as described above (with reference to **FIGS. 1** or **2**).

In some cases, additional beads can be used to deliver additional reagents to a partition. In such cases, it may be advantageous to introduce different beads into a common channel or droplet generation junction, from different bead sources (e.g., containing different associated reagents) through different channel inlets into such common channel or droplet generation junction. In such cases, the flow and frequency of the different beads into the channel or junction may be controlled to provide for a certain ratio of beads from each source, while ensuring a given pairing or combination of such beads into a partition with a given number of biological particles (e.g., one biological particle and one bead per partition).

### H. Wells

As described herein, one or more processes may be performed in a partition, which may be a well. The well may be a well of a plurality of wells of a substrate, such as a microwell of a microwell array or plate, or the well may be a microwell or microchamber of a device (e.g., microfluidic device) comprising a substrate. The well may be a well of a well array or plate, or the well may be a well or chamber of a device (e.g., fluidic device). Accordingly, the wells or microwells may assume an "open" configuration, in which the wells or microwells are exposed to the environment (e.g., contain an open surface) and are accessible on one planar face of the substrate, or the wells or microwells may assume a "closed" or "sealed" configuration, in which the microwells are not accessible on a planar face of the substrate. In some instances, the wells or microwells may be configured to toggle between "open" and "closed" configurations. For instance, an "open" microwell or set of microwells may be "closed" or "sealed" using a membrane (e.g., semi-permeable membrane), an oil (e.g., fluorinated oil to cover an aqueous solution), or a lid, as described elsewhere herein.

The well may have a volume of less than 1 milliliter (mL). For instance, the well may be configured to hold a volume of at most 1000 microliters (µL), at most 100 µL, at most 10 µL, at most 1 µL, at most 100 nanoliters (nL), at most 10 nL, at most 1 nL, at most 100 picoliters (pL), at most 10 (pL), or less. The well may be configured to hold a volume of about 1000 µL, about 100 µL, about 10 µL, about 1 µL, about 100 nL, about 10 nL, about 1 nL, about 100 pL, about 10 pL, etc. The well may be configured to hold a volume of at least 10 pL, at least 100 pL, at least 1 nL, at least 10 nL, at least 100 nL, at least 1 µL, at least 10 µL, at least 100 µL, at least 1000 µL, or more. The well may be configured to hold a volume in a range of volumes listed herein, for example, from about 5 nL to about 20 nL, from about 1 nL to about 100 nL, from about 500 pL to about 100 µL, etc. The well may be of a plurality of wells that have varying volumes and may be configured to hold a volume appropriate to accommodate any of the partition volumes described herein.

In some instances, a microwell array or plate comprises a single variety of microwells. In some instances, a microwell array or plate comprises a variety of microwells. For instance, the microwell array or plate may comprise one or more types of microwells within a single microwell array or plate. The types of microwells may have different dimensions (e.g., length, width, diameter, depth, cross-sectional area, etc.), shapes (e.g., circular, triangular, square, rectangular, pentagonal, hexagonal, heptagonal, octagonal, nonagonal, decagonal, etc.), aspect ratios, or other physical characteristics. The microwell array or plate may comprise any number of different types of microwells. For example, the microwell array or plate may comprise 1, 2, 3, 4, 5, 6, 7, 8, 9, 10, 20, 30, 40, 50, 60, 70, 80, 90, 100, 200, 300, 400, 500, 600, 700, 800, 900, 1000 or more different types of microwells. A well may have any dimension (e.g., length, width, diameter, depth, cross-sectional area, volume, etc.), shape (e.g., circular, triangular, square, rectangular, pentagonal, hexagonal, heptagonal, octagonal, nonagonal, decagonal, other polygonal, etc.), aspect ratios, or other physical characteristics described herein with respect to any well.

In certain instances, the microwell array or plate comprises different types of microwells that are located adjacent to one another within the array or plate. For instance, a microwell with one set of dimensions may be located adjacent to and in contact with another microwell with a different set of dimensions. Similarly, microwells of different geometries may be placed adjacent to or in contact with one another. The adjacent microwells may be configured to hold different articles; for example, one microwell may be used to contain a cell, cell bead, or other sample (e.g., cellular components, nucleic acid molecules, etc.) while the adjacent microwell may be used to contain a droplet, bead, or other reagent. In some cases, the adjacent microwells may be configured to merge the contents held within, e.g., upon application of a stimulus, or spontaneously, upon contact of the articles in each microwell.

As is described elsewhere herein, a plurality of partitions may be used in the systems, compositions, and methods described herein. For example, any suitable number of partitions (e.g., wells or droplets) can be generated or otherwise provided. For example, in the case when wells are used, at least about 1,000 wells, at least about 5,000 wells, at least about 10,000 wells, at least about 50,000 wells, at least about 100,000 wells, at least about 500,000 wells, at least about 1,000,000 wells, at least about 5,000,000 wells at least about 10,000,000 wells, at least about 50,000,000 wells, at least about 100,000,000 wells, at least about 500,000,000 wells, at least about 1,000,000,000 wells, or more wells can be generated or otherwise provided. Moreover, the plurality of wells may comprise both unoccupied wells (e.g., empty wells) and occupied wells.

A well may comprise any of the reagents described herein, or combinations thereof. These reagents may include, for example, barcode molecules, enzymes, adapters, and combinations thereof. The reagents may be physically separated from a sample (e.g., a cell, cell bead, or cellular components, e.g., proteins, nucleic acid molecules, etc.) that is placed in the well. This physical separation may be accomplished by containing the reagents within, or coupling to, a bead that is placed within a well. The physical separation may also be accomplished by dispensing the reagents in the well and overlaying the reagents with a layer that is, for example, dissolvable, meltable, or permeable prior to introducing the polynucleotide sample into the well. This layer may be, for example, an oil, wax, membrane (e.g., semi-permeable membrane), or the like. The well may be sealed at any point, for example, after addition of the bead, after addition of the reagents, or after addition of either of these components. The sealing of the well may be useful for a variety of purposes, including preventing escape of beads or loaded reagents from the well, permitting select delivery of certain reagents (e.g., via the use of a semi-permeable membrane), for storage of the well prior to or following further processing, etc. Once sealed, the well may be subjected to conditions for further processing of a cell (or cells) in the well. For instance, reagents in the well may allow further processing of the cell, e.g., cell lysis, as further described herein. Alternatively, the well (or wells such as those of a well-based array) comprising the cell (or cells) may be subjected to freeze-thaw cycling to process the cell (or cells), e.g., cell lysis. The well containing the cell may be subjected to freezing temperatures (e.g., 0°C, below 0°C, -5°C, -10°C, -15°C, -20°C, - 25°C, -30°C, -35°C, -40°C, -45°C, -50°C, -55°C, -60°C, -65°C, -70°C, -80°C, or -85°C). Freezing may be performed in a suitable manner, e.g., sub-zero freezer or a dry ice/ethanol bath. Following an initial freezing, the well (or wells) comprising the cell (or cells) may be subjected to freeze-thaw cycles to lyse the cell (or cells). In one embodiment, the initially frozen well (or wells) are thawed to a temperature above freezing (e.g., 4°C or above, 8°C or above, 12°C or above, 16°C or above, 20°C or above, room temperature, or 25°C or above). In another embodiment, the freezing is performed for less than 10 minutes (e.g., 5 minutes or 7 minutes) followed by thawing at room temperature for less than 10 minutes (e.g., 5 minutes or 7 minutes). This freeze-thaw cycle may be repeated a number of times, e.g., 2, 3, 4 or more times, to obtain lysis of the cell (or cells) in the well (or wells). In one embodiment, the freezing, thawing and/or freeze/thaw cycling is performed in the absence of a lysis buffer. Additional disclosure related to freeze-thaw cycling is provided in WO2019165181A1.

A well may comprise free reagents and/or reagents encapsulated in, or otherwise coupled to or associated with, beads, or droplets.

The wells may be provided as a part of a kit. For example, a kit may comprise instructions for use, a microwell array or device, and reagents (e.g., beads). The kit may comprise any useful reagents for performing the processes described herein, e.g., nucleic acid reactions, barcoding of nucleic acid molecules, sample processing (e.g., for cell lysis, fixation, and/or permeabilization).

In some cases, a well comprises a bead, or droplet that comprises a set of reagents that has a similar attribute (e.g., a set of enzymes, a set of minerals, a set of oligonucleotides, a mixture of different barcode molecules, a mixture of identical barcode molecules). In other cases, a bead, or droplet comprises a heterogeneous mixture of reagents. In some cases, the heterogeneous mixture of reagents can comprise all components necessary to perform a reaction. In some cases, such mixture can comprise all components necessary to perform a reaction, except for 1, 2, 3, 4, 5, or more components necessary to perform a reaction. In some cases, such additional components are contained within, or otherwise coupled to, a different droplet, or bead, or within a solution within a partition (e.g., microwell) of the system.

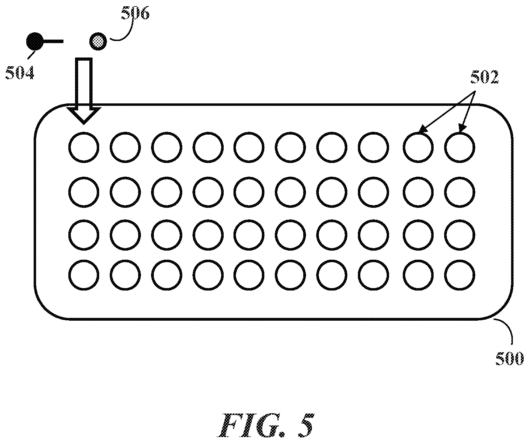

**FIG. 5** schematically illustrates an example of a microwell array. The array can be contained within a substrate **500.** The substrate **500** comprises a plurality of wells **502.** The wells **502** may be of any size or shape, and the spacing between the wells, the number of wells per substrate, as well as the density of the wells on the substrate **500** can be modified, depending on the particular application. In one such example application, a sample molecule **506,** which may comprise a cell or cellular components (e.g., nucleic acid molecules) is co-partitioned with a bead **504,** which may comprise a nucleic acid barcode molecule coupled thereto. The wells **502** may be loaded using gravity or other loading technique (e.g., centrifugation, liquid handler, acoustic loading, optoelectronic, etc.). In some instances, at least one of the wells **502** contains a single sample molecule **506** (e.g., cell) and a single bead **504.**

Reagents may be loaded into a well either sequentially or concurrently. In some cases, reagents are introduced to the device either before or after a particular operation. In some cases, reagents (which may be provided, in certain instances, in droplets or beads) are introduced sequentially such that different reactions or operations occur at different steps. The reagents (or droplets, or beads) may also be loaded at operations interspersed with a reaction or operation step. For example, droplets or beads comprising reagents for fragmenting polynucleotides (e.g., restriction enzymes) and/or other enzymes (e.g., transposases, ligases, polymerases, etc.) may be loaded into the well or plurality of wells, followed by loading of droplets, or beads comprising reagents for attaching nucleic acid barcode molecules to a sample nucleic acid molecule. Reagents may be provided concurrently or sequentially with a sample, e.g., a cell or cellular components (e.g., organelles, proteins, nucleic acid molecules, carbohydrates, lipids, etc.). Accordingly, use of wells may be useful in performing multi-step operations or reactions.

As described elsewhere herein, the nucleic acid barcode molecules and other reagents may be contained within a bead, or droplet. These beads, or droplets may be loaded into a partition (e.g., a microwell) before, after, or concurrently with the loading of a cell, such that each cell is contacted with a different bead, or droplet. This technique may be used to attach a unique nucleic acid barcode molecule to nucleic acid molecules obtained from each cell. Alternatively or in addition to, the sample nucleic acid molecules may be attached to a support. For instance, the partition (e.g., microwell) may comprise a bead which has coupled thereto a plurality of nucleic acid barcode molecules. The sample nucleic acid molecules, or derivatives thereof, may couple or attach to the nucleic acid barcode molecules on the support. The resulting barcoded nucleic acid molecules may then be removed from the partition, and in some instances, pooled and sequenced. In such cases, the nucleic acid barcode sequences may be used to trace the origin of the sample nucleic acid molecule. For example, polynucleotides with identical barcodes may be determined to originate from the same cell or partition, while polynucleotides with different barcodes may be determined to originate from different cells or partitions.

The samples or reagents may be loaded in the wells or microwells using a variety of approaches. The samples (e.g., a cell, cell bead, or cellular component) or reagents (as described herein) may be loaded into the well or microwell using an external force, e.g., gravitational force, electrical force, magnetic force, or using mechanisms to drive the sample or reagents into the well, e.g., via pressure-driven flow, centrifugation, optoelectronics, acoustic loading, electrokinetic pumping, vacuum, capillary flow, etc. In certain cases, a fluid handling system may be used to load the samples or reagents into the well. The loading of the samples or reagents may follow a Poissonian distribution or a non-Poissonian distribution, e.g., super Poisson or sub-Poisson. The geometry, spacing between wells, density, and size of the microwells may be modified to accommodate a useful sample or reagent distribution; for instance, the size and spacing of the microwells may be adjusted such that the sample or reagents may be distributed in a super-Poissonian fashion.

In one particular non-limiting example, the microwell array or plate comprises pairs of microwells, in which each pair of microwells is configured to hold a droplet (e.g., comprising a single cell) and a single bead (such as those described herein, which may, in some instances, also be encapsulated in a droplet). The droplet and the bead (or droplet containing the bead) may be loaded simultaneously or sequentially, and the droplet and the bead may be merged, e.g., upon contact of the droplet and the bead, or upon application of a stimulus (e.g., external force, agitation, heat, light, magnetic or electric force, etc.). In some cases, the loading of the droplet and the bead is super-Poissonian. In other examples of pairs of microwells, the wells are configured to hold two droplets comprising different reagents and/or samples, which are merged upon contact or upon application of a stimulus. In such instances, the droplet of one microwell of the pair can comprise reagents that may react with an agent in the droplet of the other microwell of the pair. For instance, one droplet can comprise reagents that are configured to release the nucleic acid barcode molecules of a bead contained in another droplet, located in the adjacent microwell. Upon merging of the droplets, the nucleic acid barcode molecules may be released from the bead into the partition (e.g., the microwell or microwell pair that are in contact), and further processing may be performed (e.g., barcoding, nucleic acid reactions, etc.). In cases where intact or live cells are loaded in the microwells, one of the droplets may comprise lysis reagents for lysing the cell upon droplet merging.

A droplet or bead may be partitioned into a well. The droplets may be selected or subjected to pre-processing prior to loading into a well. For instance, the droplets may comprise cells, and only certain droplets, such as those containing a single cell (or at least one cell), may be selected for use in loading of the wells. Such a pre-selection process may be useful in efficient loading of single cells, such as to obtain a non-Poissonian distribution, or to pre-filter cells for a selected characteristic prior to further partitioning in the wells. Additionally, the technique may be useful in obtaining or preventing cell doublet or multiplet formation prior to or during loading of the microwell.

In some instances, the wells can comprise nucleic acid barcode molecules attached thereto. The nucleic acid barcode molecules may be attached to a surface of the well (e.g., a wall of the well). The nucleic acid barcode molecules may be attached to a droplet or bead that has been partitioned into the well. The nucleic acid barcode molecule (e.g., a partition barcode sequence) of one well may differ from the nucleic acid barcode molecule of another well, which can permit identification of the contents contained with a single partition or well. In some cases, the nucleic acid barcode molecule can comprise a spatial barcode sequence that can identify a spatial coordinate of a well, such as within the well array or well plate. In some cases, the nucleic acid barcode molecule can comprise a unique molecular identifier for individual molecule identification. In some instances, the nucleic acid barcode molecules may be configured to attach to or capture a nucleic acid molecule within a sample or cell distributed in the well. For example, the nucleic acid barcode molecules may comprise a capture sequence that may be used to capture or hybridize to a nucleic acid molecule (e.g., RNA, DNA) within the sample. In some instances, the nucleic acid barcode molecules may be releasable from the microwell. In some instances, the nucleic acid barcode molecules may be releasable from the bead or droplet. For instance, the nucleic acid barcode molecules may comprise a chemical cross-linker which may be cleaved upon application of a stimulus (e.g., photo-, magnetic, chemical, biological, stimulus). The released nucleic acid barcode molecules, which may be hybridized or configured to hybridize to a sample nucleic acid molecule, may be collected and pooled for further processing, which can include nucleic acid processing (e.g., amplification, extension, reverse transcription, etc.) and/or characterization (e.g., sequencing). In some instances nucleic acid barcode molecules attached to a bead or droplet in a well may be hybridized to sample nucleic acid molecules, and the bead with the sample nucleic acid molecules hybridized thereto may be collected and pooled for further processing, which can include nucleic acid processing (e.g., amplification, extension, reverse transcription, etc.) and/or characterization (e.g., sequencing). In such cases, the unique partition barcode sequences may be used to identify the cell or partition from which a nucleic acid molecule originated.

Characterization of samples within a well may be performed. Such characterization can include, in non-limiting examples, imaging of the sample (e.g., cell, cell bead, or cellular components) or derivatives thereof. Characterization techniques such as microscopy or imaging may be useful in measuring sample profiles in fixed spatial locations. For instance, when cells are partitioned, optionally with beads, imaging of each microwell and the contents contained therein may provide useful information on cell doublet formation (e.g., frequency, spatial locations, etc.), cell-bead pair efficiency, cell viability, cell size, cell morphology, expression level of a biomarker (e.g., a surface marker, a fluorescently labeled molecule therein, etc.), cell or bead loading rate, number of cell-bead pairs, etc. In some instances, imaging may be used to characterize live cells in the wells, including, but not limited to: dynamic live-cell tracking, cell-cell interactions (when two or more cells are co-partitioned), cell proliferation, etc. Alternatively or in addition to, imaging may be used to characterize a quantity of amplification products in the well.

In operation, a well may be loaded with a sample and reagents, simultaneously or sequentially. When cells or cell beads are loaded, the well may be subjected to washing, e.g., to remove excess cells from the well, microwell array, or plate. Similarly, washing may be performed to remove excess beads or other reagents from the well, microwell array, or plate. In the instances where live cells are used, the cells may be lysed in the individual partitions to release the intracellular components or cellular analytes. Alternatively, the cells may be fixed or permeabilized in the individual partitions. The intracellular components or cellular analytes may couple to a support, e.g., on a surface of the microwell, on a solid support (e.g., bead), or they may be collected for further downstream processing. For instance, after cell lysis, the intracellular components or cellular analytes may be transferred to individual droplets or other partitions for barcoding. Alternatively, or in addition to, the intracellular components or cellular analytes (e.g., nucleic acid molecules) may couple to a bead comprising a nucleic acid barcode molecule; subsequently, the bead may be collected and further processed, e.g., subjected to nucleic acid reaction such as reverse transcription, amplification, or extension, and the nucleic acid molecules thereon may be further characterized, e.g., via sequencing. Alternatively, or in addition to, the intracellular components or cellular analytes may be barcoded in the well (e.g., using a bead comprising nucleic acid barcode molecules that are releasable or on a surface of the microwell comprising nucleic acid barcode molecules). The barcoded nucleic acid molecules or analytes may be further processed in the well, or the barcoded nucleic acid molecules or analytes may be collected from the individual partitions and subjected to further processing outside the partition. Further processing can include nucleic acid processing (e.g., performing an amplification, extension) or characterization (e.g., fluorescence monitoring of amplified molecules, sequencing). At any convenient or useful step, the well (or microwell array or plate) may be sealed (e.g., using an oil, membrane, wax, etc.), which enables storage of the assay or selective introduction of additional reagents.

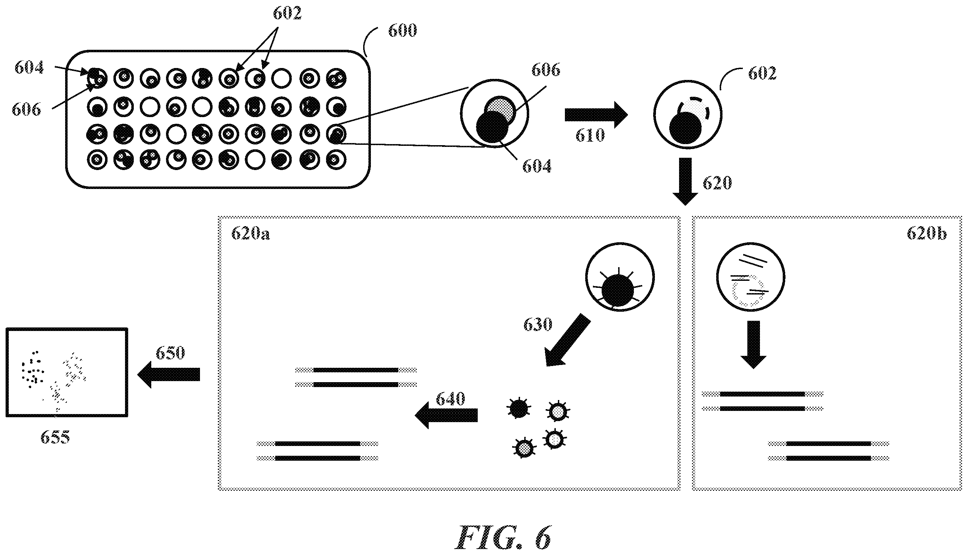

**FIG. 6** schematically shows an example workflow for processing nucleic acid molecules within a sample. A substrate **600** comprising a plurality of microwells 602 may be provided. A sample **606** which may comprise a cell, cell bead, cellular components or analytes (e.g., proteins and/or nucleic acid molecules) can be co-partitioned, in a plurality of microwells **602,** with a plurality of beads **604** comprising nucleic acid barcode molecules. During process **610,** the sample **606** may be processed within the partition. For instance, in the case of live cells, the cell may be subjected to conditions sufficient to lyse the cells and release the analytes contained therein. In process **620,** the bead **604** may be further processed. By way of example, processes **620a** and **620b** schematically illustrate different workflows, depending on the properties of the bead **604.**

In **620a,** the bead comprises nucleic acid barcode molecules that are attached thereto, and sample nucleic acid molecules (e.g., RNA, DNA) may attach, e.g., via hybridization of ligation, to the nucleic acid barcode molecules. Such attachment may occur on the bead. In process **630,** the beads **604** from multiple wells **602** may be collected and pooled. Further processing may be performed in process **640.** For example, one or more nucleic acid reactions may be performed, such as reverse transcription, nucleic acid extension, amplification, ligation, transposition, etc. In some instances, adapter sequences are ligated to the nucleic acid molecules, or derivatives thereof, as described elsewhere herein. For instance, sequencing primer sequences may be appended to each end of the nucleic acid molecule. In process **650,** further characterization, such as sequencing may be performed to generate sequencing reads. The sequencing reads may yield information on individual cells or populations of cells, which may be represented visually or graphically, e.g., in a plot 655.

In **620b,** the bead comprises nucleic acid barcode molecules that are releasably attached thereto, as described below. The bead may degrade or otherwise release the nucleic acid barcode molecules into the well **602;** the nucleic acid barcode molecules may then be used to barcode nucleic acid molecules within the well **602.** Further processing may be performed either inside the partition or outside the partition. For example, one or more nucleic acid reactions may be performed, such as reverse transcription, nucleic acid extension, amplification, ligation, transposition, etc. In some instances, adapter sequences are ligated to the nucleic acid molecules, or derivatives thereof, as described elsewhere herein. For instance, sequencing primer sequences may be appended to each end of the nucleic acid molecule. In process **650,** further characterization, such as sequencing may be performed to generate sequencing reads. The sequencing reads may yield information on individual cells or populations of cells, which may be represented visually or graphically, e.g., in a plot **655.**

### I. Multi-assay & 5' Barcodes

The present disclosures provides methods and systems for multiplexing, and otherwise increasing throughput in, analysis. For example, a single or integrated process workflow may permit the processing, identification, and/or analysis of more or multiple analytes, more or multiple types of analytes, and/or more or multiple types of analyte characterizations. For example, in the methods and systems described herein, one or more labelling agents (also referred to herein as reporter molecules) capable of binding to or otherwise coupling to one or more cell features may be used to characterize biological particles and/or cell features. In some instances, cell features include cell surface features. Cell surface features may include, but are not limited to, a receptor, an antigen, a surface protein, a transmembrane protein, a cluster of differentiation protein, a protein channel, a protein pump, a carrier protein, a phospholipid, a glycoprotein, a glycolipid, a cell-cell interaction protein complex, an antigen-presenting complex, a major histocompatibility complex, an engineered T-cell receptor, a T-cell receptor, a B-cell receptor, a chimeric antigen receptor, a gap junction, an adherens junction, or any combination thereof. In some instances, cell features may include intracellular analytes, such as proteins, protein modifications (e.g., phosphorylation status or other post-translational modifications), nuclear proteins, nuclear membrane proteins, or any combination thereof. A labelling agent may include, but is not limited to, a protein, a peptide, an antibody (or an epitope binding fragment thereof), a lipophilic moiety (such as cholesterol), a cell surface receptor binding molecule, a receptor ligand, a small molecule, a bi-specific antibody, a bi-specific T-cell engager, a T-cell receptor engager, a B-cell receptor engager, a pro-body, an aptamer, a monobody, an affimer, a darpin, and a protein scaffold, or any combination thereof. The labelling agents can include (e.g., are attached to) a reporter oligonucleotide that is indicative of the cell feature to which the binding group binds. For example, the reporter oligonucleotide may comprise a barcode sequence that permits identification of the labelling agent. For example, a labelling agent that is specific to one type of cell feature (e.g., a first cell surface feature) may have a first reporter oligonucleotide coupled thereto, while a labelling agent that is specific to a different cell feature (e.g., a second cell surface feature) may have a different reporter oligonucleotide coupled thereto. For a description of exemplary labelling agents, reporter oligonucleotides, and methods of use, see, e.g., U.S. Pat. 10,550,429; U.S. Pat. Pub. 20190177800; and U.S. Pat. Pub. 20190367969.

In a particular example, a library of potential cell feature labelling agents may be provided, where the respective cell feature labelling agents are associated with nucleic acid reporter molecules, such that a different reporter oligonucleotide sequence is associated with each labelling agent capable of binding to a specific cell feature. In some aspects, different members of the library may be characterized by the presence of a different oligonucleotide sequence label. For example, an antibody capable of binding to a first protein may have associated with it a first reporter oligonucleotide sequence, while an antibody capable of binding to a second protein may have a different reporter oligonucleotide sequence associated with it. The presence of the particular oligonucleotide sequence may be indicative of the presence of a particular antibody or cell feature which may be recognized or bound by the particular antibody.

Labelling agents capable of binding to or otherwise coupling to one or more biological particles may be used to characterize a biological particle as belonging to a particular set of biological particles. For example, labeling agents may be used to label a sample of cells or a group of cells. In this way, a group of cells may be labeled as different from another group of cells. In an example, a first group of cells may originate from a first sample and a second group of cells may originate from a second sample. Labelling agents may allow the first group and second group to have a different labeling agent (or reporter oligonucleotide associated with the labeling agent). This may, for example, facilitate multiplexing, where cells of the first group and cells of the second group may be labeled separately and then pooled together for downstream analysis. The downstream detection of a label may indicate analytes as belonging to a particular group.

For example, a reporter oligonucleotide may be linked to an antibody or an epitope binding fragment thereof, and labeling a biological particle may comprise subjecting the antibody-linked barcode molecule or the epitope binding fragment-linked barcode molecule to conditions suitable for binding the antibody to a molecule present on a surface of the biological particle. The binding affinity between the antibody or the epitope binding fragment thereof and the molecule present on the surface may be within a desired range to ensure that the antibody or the epitope binding fragment thereof remains bound to the molecule. For example, the binding affinity may be within a desired range to ensure that the antibody or the epitope binding fragment thereof remains bound to the molecule during various sample processing steps, such as partitioning and/or nucleic acid amplification or extension. A dissociation constant (Kd) between the antibody or an epitope binding fragment thereof and the molecule to which it binds may be less than about 100 µM, 90 µM, 80 µM, 70 µM, 60 µM, 50 µM, 40 µM, 30 µM, 20 µM, 10 µM, 9 µM, 8 µM, 7 µM, 6 µM, 5 µM, 4 µM, 3 µM, 2 µM, 1 µM, 900 nM, 800 nM, 700 nM, 600 nM, 500 nM, 400 nM, 300 nM, 200 nM, 100 nM, 90 nM, 80 nM, 70 nM, 60 nM, 50 nM, 40 nM, 30 nM, 20 nM, 10 nM, 9 nM, 8 nM, 7 nM, 6 nM, 5 nM, 4 nM, 3 nM, 2 nM, 1 nM, 900 pM, 800 pM, 700 pM, 600 pM, 500 pM, 400 pM, 300 pM, 200 pM, 100 pM, 90 pM, 80 pM, 70 pM, 60 pM, 50 pM, 40 pM, 30 pM, 20 pM, 10 pM, 9 pM, 8 pM, 7 pM, 6 pM, 5 pM, 4 pM, 3 pM, 2 pM, or 1 pM. For example, the dissociation constant may be less than about 10 µM.

In another example, a reporter oligonucleotide may be coupled to a cell-penetrating peptide (CPP), and labeling cells may comprise delivering the CPP coupled reporter oligonucleotide into an analyte carrier. Labeling biological particles may comprise delivering the CPP conjugated oligonucleotide into a cell and/or cell bead by the cell-penetrating peptide. A cell-penetrating peptide that can be used in the methods provided herein can comprise at least one non-functional cysteine residue, which may be either free or derivatized to form a disulfide link with an oligonucleotide that has been modified for such linkage. Non-limiting examples of cell-penetrating peptides that can be used in embodiments herein include penetratin, transportan, plsl, TAT(48-60), pVEC, MTS, and MAP. Cell-penetrating peptides useful in the methods provided herein can have the capability of inducing cell penetration for at least about 30%, 40%, 50%, 60%, 70%, 80%, 90%, 95%, 96%, 97%, 98%, 99%, or 100% of cells of a cell population. The cell-penetrating peptide may be an arginine-rich peptide transporter. The cell-penetrating peptide may be Penetratin or the Tat peptide.

In another example, a reporter oligonucleotide may be coupled to a fluorophore or dye, and labeling cells may comprise subjecting the fluorophore-linked barcode molecule to conditions suitable for binding the fluorophore to the surface of the biological particle. In some instances, fluorophores can interact strongly with lipid bilayers and labeling biological particles may comprise subjecting the fluorophore-linked barcode molecule to conditions such that the fluorophore binds to or is inserted into a membrane of the biological particle. In some cases, the fluorophore is a water-soluble, organic fluorophore. In some instances, the fluorophore is Alexa 532 maleimide, tetramethylrhodamine-5-maleimide (TMR maleimide), BODIPY-TMR maleimide, Sulfo-Cy3 maleimide, Alexa 546 carboxylic acid/succinimidyl ester, Atto 550 maleimide, Cy3 carboxylic acid/succinimidyl ester, Cy3B carboxylic acid/succinimidyl ester, Atto 565 biotin, Sulforhodamine B, Alexa 594 maleimide, Texas Red maleimide, Alexa 633 maleimide, Abberior STAR 635P azide, Atto 647N maleimide, Atto 647 SE, or Sulfo-Cy5 maleimide. See, e.g., Hughes L D, et al. PLoS One. 2014 Feb. 4; 9(2):e87649 for a description of organic fluorophores.

A reporter oligonucleotide may be coupled to a lipophilic molecule, and labeling biological particles may comprise delivering the nucleic acid barcode molecule to a membrane of the biological particle or a nuclear membrane by the lipophilic molecule. Lipophilic molecules can associate with and/or insert into lipid membranes such as cell membranes and nuclear membranes. In some cases, the insertion can be reversible. In some cases, the association between the lipophilic molecule and biological particle may be such that the biological particle retains the lipophilic molecule (e.g., and associated components, such as nucleic acid barcode molecules, thereof) during subsequent processing (e.g., partitioning, cell permeabilization, amplification, pooling, etc.). The reporter nucleotide may enter into the intracellular space and/or a cell nucleus.

A reporter oligonucleotide may be part of a nucleic acid molecule comprising any number of functional sequences, as described elsewhere herein, such as a target capture sequence, a random primer sequence, and the like, and coupled to another nucleic acid molecule that is, or is derived from, the analyte.

Prior to partitioning, the cells may be incubated with the library of labelling agents, that may be labelling agents to a broad panel of different cell features, e.g., receptors, proteins, etc., and which include their associated reporter oligonucleotides. Unbound labelling agents may be washed from the cells, and the cells may then be co-partitioned (e.g., into droplets or wells ) along with nucleic acid barcode molecules comprising a partition-specific barcode sequence(e.g., attached to a support, such as a bead or gel bead) as described elsewhere herein. As a result, the partitions may include the cell or cells, as well as the bound labelling agents and their known, associated reporter oligonucleotides.

In other instances, e.g., to facilitate sample multiplexing, a labelling agent that is specific to a particular cell feature may have a first plurality of the labelling agent (e.g., an antibody or lipophilic moiety) coupled to a first reporter oligonucleotide and a second plurality of the labelling agent coupled to a second reporter oligonucleotide. For example, the first plurality of the labeling agent and second plurality of the labeling agent may interact with different cells, cell populations or samples, allowing a particular report oligonucleotide to indicate a particular cell population (or cell or sample) and cell feature. In this way, different samples or groups can be independently processed and subsequently combined together for pooled analysis (e.g., partition-based barcoding as described elsewhere herein). See, e.g., U.S. Pat. Pub. 20190323088.

As described elsewhere herein, libraries of labelling agents may be associated with a particular cell feature as well as be used to identify analytes as originating from a particular biological particle, population, or sample. The biological particles may be incubated with a plurality of libraries and a given biological particle may comprise multiple labelling agents. For example, a cell may comprise coupled thereto a lipophilic labeling agent and an antibody. The lipophilic labeling agent may indicate that the cell is a member of a particular cell sample, whereas the antibody may indicate that the cell comprises a particular analyte. In this manner, the reporter oligonucleotides and labelling agents may allow multi-analyte, multiplexed analyses to be performed.

In some instances, these reporter oligonucleotides may comprise nucleic acid barcode sequences that permit identification of the labelling agent which the reporter oligonucleotide is coupled to. The use of oligonucleotides as the reporter may provide advantages of being able to generate significant diversity in terms of sequence, while also being readily attachable to most biomolecules, e.g., antibodies, etc., as well as being readily detected, e.g., using sequencing or array technologies.

Attachment (coupling) of the reporter oligonucleotides to the labelling agents may be achieved through any of a variety of direct or indirect, covalent or non-covalent associations or attachments. For example, oligonucleotides may be covalently attached to a portion of a labelling agent (such a protein, e.g., an antibody or antibody fragment) using chemical conjugation techniques (e.g., Lightning-Link^{®} antibody labelling kits available from Innova Biosciences), as well as other non-covalent attachment mechanisms, e.g., using biotinylated antibodies and oligonucleotides (or beads that include one or more biotinylated linker, coupled to oligonucleotides) with an avidin or streptavidin linker. Antibody and oligonucleotide biotinylation techniques are available. See, e.g., Fang, et al., "Fluoride-Cleavable Biotinylation Phosphoramidite for 5'-end-Labelling and Affinity Purification of Synthetic Oligonucleotides," Nucleic Acids Res. Jan. 15, 2003; 31(2):708-715. Likewise, protein and peptide biotinylation techniques have been developed and are readily available. See, e.g., U.S. Pat. No. 6,265,552.

Furthermore, click reaction chemistry such as a Methyltetrazine-PEG5-NHS Ester reaction, a TCO-PEG4-NHS Ester reaction, or the like, may be used to couple reporter oligonucleotides to labelling agents. Commercially available kits, such as those from Thunderlink and Abcam, and techniques common in the art may be used to couple reporter oligonucleotides to labelling agents as appropriate. In another example, a labelling agent is indirectly (e.g., via hybridization) coupled to a reporter oligonucleotide comprising a barcode sequence that identifies the label agent. For instance, the labelling agent may be directly coupled (e.g., covalently bound) to a hybridization oligonucleotide that comprises a sequence that hybridizes with a sequence of the reporter oligonucleotide. Hybridization of the hybridization oligonucleotide to the reporter oligonucleotide couples the labelling agent to the reporter oligonucleotide. In some embodiments, the reporter oligonucleotides are releasable from the labelling agent, such as upon application of a stimulus. For example, the reporter oligonucleotide may be attached to the labeling agent through a labile bond (e.g., chemically labile, photolabile, thermally labile, etc.) as generally described for releasing molecules from supports elsewhere herein. In some instances, the reporter oligonucleotides described herein may include one or more functional sequences that can be used in subsequent processing, such as an adapter sequence, a unique molecular identifier (UMI) sequence, a sequencer specific flow cell attachment sequence (such as an P5, P7, or partial P5 or P7 sequence), a primer or primer binding sequence, a sequencing primer or primer biding sequence (such as an R1, R2, or partial R1 or R2 sequence).

In some cases, the labelling agent can comprise a reporter oligonucleotide and a label. A label can be fluorophore, a radioisotope, a molecule capable of a colorimetric reaction, a magnetic particle, or any other suitable molecule or compound capable of detection. The label can be conjugated to a labelling agent (or reporter oligonucleotide) either directly or indirectly (e.g., the label can be conjugated to a molecule that can bind to the labelling agent or reporter oligonucleotide). In some cases, a label is conjugated to an oligonucleotide that is complementary to a sequence of the reporter oligonucleotide, and the oligonucleotide may be allowed to hybridize to the reporter oligonucleotide.

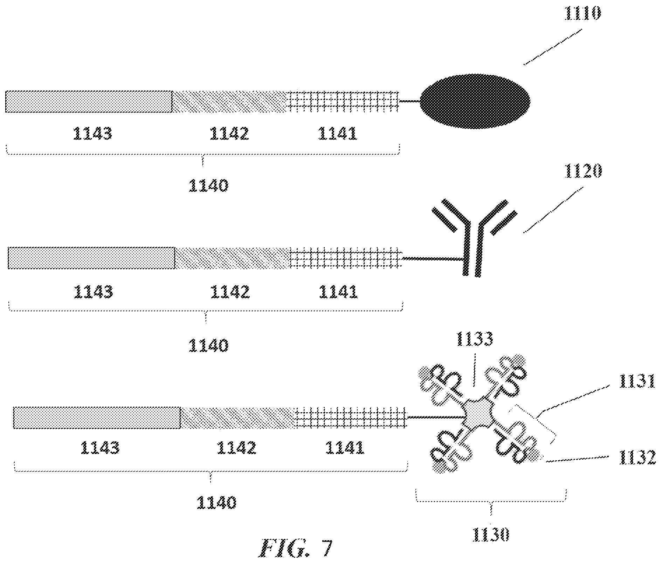

**FIG. 7** describes exemplary labelling agents (**1110, 1120**, and **1130**) comprising reporter oligonucleotides **(1140)** attached thereto. Labelling agent **1110** (e.g., any of the labelling agents described herein) is attached (either directly, e.g., covalently attached, or indirectly) to reporter oligonucleotide **1140.** Reporter oligonucleotide **1140** may comprise barcode sequence **1142** that identifies labelling agent **1110.** Reporter oligonucleotide **1140** may also comprise one or more functional sequences that can be used in subsequent processing, such as an adapter sequence, a unique molecular identifier (UMI) sequence, a sequencer specific flow cell attachment sequence (such as an P5, P7, or partial P5 or P7 sequence), a primer or primer binding sequence, or a sequencing primer or primer biding sequence (such as an R1, R2, or partial R1 or R2 sequence).

Referring to **FIG. 7****,** in some instances, reporter oligonucleotide **1140** conjugated to a labelling agent (e.g., **1110, 1120, 1130**) comprises a functional sequence **1141** (e.g., a primer sequence), a barcode sequence that identifies the labelling agent (e.g., **1110, 1120, 1130**), and functional sequence **1143.** Functional sequence **1143** can be a reporter capture handle sequence configured to hybridize to a complementary sequence, such as a complementary sequence present on a nucleic acid barcode molecule **1190** (not shown), such as those described elsewhere herein. In some instances, nucleic acid barcode molecule **1190** is attached to a support (e.g., a bead, such as a gel bead), such as those described elsewhere herein. For example, nucleic acid barcode molecule **1190** may be attached to the support via a releasable linkage (e.g., comprising a labile bond), such as those described elsewhere herein. In some instances, reporter oligonucleotide **1140** comprises one or more additional functional sequences, such as those described above.

In some instances, the labelling agent **1110** is a protein or polypeptide (e.g., an antigen or prospective antigen) comprising reporter oligonucleotide **1140.** Reporter oligonucleotide **1140** comprises barcode sequence **1142** that identifies polypeptide **1110** and can be used to infer the presence of an analyte, e.g., a binding partner of polypeptide **1110** (i.e., a molecule or compound to which polypeptide **1110** can bind). In some instances, the labelling agent **1110** is a lipophilic moiety (e.g., cholesterol) comprising reporter oligonucleotide **1140,** where the lipophilic moiety is selected such that labelling agent **1110** integrates into a membrane of a cell or nucleus. Reporter oligonucleotide **1140** comprises barcode sequence **1142** that identifies lipophilic moiety **1110** which in some instances is used to tag cells (e.g., groups of cells, cell samples, etc.) and may be used for multiplex analyses as described elsewhere herein. In some instances, the labelling agent is an antibody **1120** (or an epitope binding fragment thereof) comprising reporter oligonucleotide **1140.** Reporter oligonucleotide **1140** comprises barcode sequence **1142** that identifies antibody **1120** and can be used to infer the presence of, e.g., a target of antibody **1120** (i.e., a molecule or compound to which antibody **1120** binds). In other embodiments, labelling agent **1130** comprises an MHC molecule **1131** comprising peptide **1132** and reporter oligonucleotide **1140** that identifies peptide **1132.** In some instances, the MHC molecule is coupled to a support **1133.** In some instances, support **1133** may be a polypeptide, such as streptavidin, or a polysaccharide, such as dextran. In some instances, reporter oligonucleotide **1140** may be directly or indirectly coupled to MHC labelling agent **1130** in any suitable manner. For example, reporter oligonucleotide **1140** may be coupled to MHC molecule **1131,** support **1133,** or peptide **1132.** In some embodiments, labelling agent **1130** comprises a plurality of MHC molecules, (e.g. is an MHC multimer, which may be coupled to a support (e.g., **1133**)). There are many possible configurations of Class I and/or Class II MHC multimers that can be utilized with the compositions, methods, and systems disclosed herein, e.g., MHC tetramers, MHC pentamers (MHC assembled via a coiled-coil domain, e.g., Pro5^{®} MHC Class I Pentamers, (ProImmune, Ltd.), MHC octamers, MHC dodecamers, MHC decorated dextran molecules (e.g., MHC Dextramer^{®} (Immudex)), etc. For a description of exemplary labelling agents, including antibody and MHC-based labelling agents, reporter oligonucleotides, and methods of use, see, e.g., U.S. Pat. 10,550,429 and U.S. Pat. Pub. 20190367969.

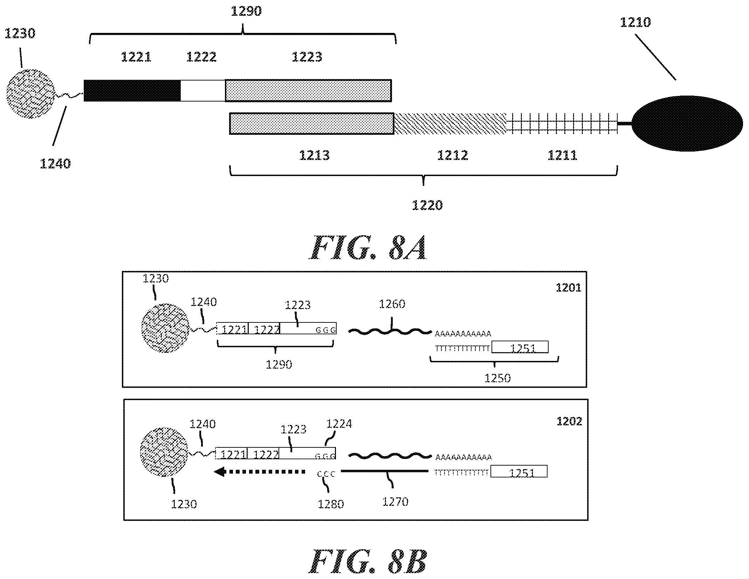

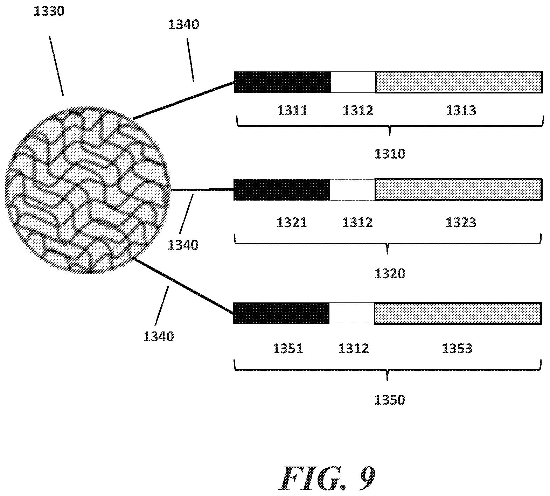

**FIG. 9** illustrates another example of a barcode carrying bead. In some embodiments, analysis of multiple analytes (e.g., RNA and one or more analytes using labelling agents described herein) may comprise nucleic acid barcode molecules as generally depicted in **FIG. 9****.** In some embodiments, nucleic acid barcode molecules **1310** and **1320** are attached to support **1330** via a releasable linkage **1340** (e.g., comprising a labile bond) as described elsewhere herein. Nucleic acid barcode molecule **1310** may comprise adapter sequence **1311,** barcode sequence **1312** and capture sequence 1313. Nucleic acid barcode molecule **1320** may comprise adapter sequence **1321,** barcode sequence **1312,** and capture sequence 1323, wherein capture sequence 1323 comprises a different sequence than capture sequence 1313. In some instances, adapter **1311** and adapter **1321** comprise the same sequence. In some instances, adapter **1311** and adapter **1321** comprise different sequences. Although support **1330** is shown comprising nucleic acid barcode molecules **1310** and **1320,** any suitable number of barcode molecules comprising common barcode sequence **1312** are contemplated herein. For example, in some embodiments, support **1330** further comprises nucleic acid barcode molecule **1350.** Nucleic acid barcode molecule **1350** may comprise adapter sequence **1351,** barcode sequence **1312** and capture sequence 1353, wherein capture sequence 1353 comprises a different sequence than capture sequence 1313 and **1323.** In some instances, nucleic acid barcode molecules (e.g., **1310, 1320, and 1350**) comprise one or more additional functional sequences, such as a UMI or other sequences described herein. The nucleic acid barcode molecules **1310, 1320** or **1350** may interact with analytes as described elsewhere herein, for example, as depicted in **FIGs. 8A-C**.

Referring to **FIG. 8A****,** in an instance where cells are labelled with labeling agents, "capture sequence" or "reporter capture sequence" **1223** may be complementary to an adapter sequence of a reporter oligonucleotide. Cells may be contacted with one or more reporter oligonucleotide **1220** conjugated labelling agents **1210** (e.g., lectin, sugar, nucleotide sugar, synthetic sugar, polypeptide, antibody, or others described elsewhere herein). In some cases, the cells may be further processed prior to barcoding. For example, such processing steps may include one or more washing and/or cell sorting steps. In some instances, a cell that is bound to labelling agent **1210** which is conjugated to oligonucleotide **1220** and support **1230** (e.g., a bead, such as a gel bead) comprising nucleic acid barcode molecule **1290** is partitioned into a partition amongst a plurality of partitions (e.g., a droplet of a droplet emulsion or a well of a microwell array). In some instances, the partition comprises at most a single cell bound to labelling agent **1210.** In some instances, reporter oligonucleotide **1220** conjugated to labelling agent **1210** (e.g., antibody, lectin, polypeptide, synthetic sugar, and nucleotide sugar.) comprises a first functional sequence **1211** (such as but not limited to an adapter sequence or a primer sequence)), a reporter barcode sequence **1212** that identifies the labelling agent **1210** (e.g., the polypeptide, antibody, or peptide of a pMHC molecule or complex), and an capture handle sequence **1213.** Capture handle sequence **1213** may be configured to hybridize to a complementary sequence, such as a capture sequence **1223** present on a nucleic acid barcode molecule **1290** (e.g. partition-specific barcode molecule). In some instances, oligonucleotide **1220** comprises one or more additional functional sequences, such as those described elsewhere herein.

Barcoded nucleic may be generated (e.g., via a nucleic acid reaction, such as nucleic acid extension or ligation) from the constructs described in **FIGs. 8A-C**. For example, capture handle sequence **1213** may then be hybridized to complementary sequence, such as capture sequence **1223** to generate (e.g., via a nucleic acid reaction, such as nucleic acid extension or ligation) a barcoded nucleic acid molecule comprising cell (e.g., partition specific) barcode sequence **1222** (or a reverse complement thereof) and reporter barcode sequence **1212** (or a reverse complement thereof). In some embodiments capture handle sequence 1213 comprises a sequence complementary to a template switching oligonucleotide on the capture sequence 1223. **In** some embodiments, the nucleic acid barcode molecule **1290** (*e.g.*, partition-specific barcode molecule) further includes a UMI (not shown). Barcoded nucleic acid molecules can then be optionally processed as described elsewhere herein, e.g., to amplify the molecules and/or append sequencing platform specific sequences to the fragments. See, e.g., U.S. Pat. Pub. 2018/0105808. Barcoded nucleic acid molecules, or derivatives generated therefrom, can then be sequenced on a suitable sequencing platform.

In some instances, analysis of multiple analytes (e.g., nucleic acids and one or more analytes using labelling agents described herein) may be performed. For example, the workflow may comprise a workflow as generally depicted in any of **FIGs. 8A-C**, or a combination of workflows for an individual analyte, as described elsewhere herein. For example, by using a combination of the workflows as generally depicted in **FIGs. 8A-C**, multiple analytes can be analyzed.

In some instances, analysis of an analyte (e.g. a nucleic acid, a polypeptide, a carbohydrate, a lipid, a glycan, a glycan motif, a metabolite, a protein, etc.) comprises a workflow as generally depicted in **FIG. 8A****.** A nucleic acid barcode molecule **1290** (e.g. partition specific barcode molecule) may be co-partitioned with the one or more analytes. In some instances, nucleic acid barcode molecule **1290** is attached to a support **1230** (e.g., a bead, such as a gel bead), such as those described elsewhere herein. For example, nucleic acid barcode molecule **1290** may be attached to support **1230** via a releasable linkage **1240** (e.g., comprising a labile bond), such as those described elsewhere herein. Nucleic acid barcode molecule **1290** may comprise a functional sequence **1221** and optionally comprise other additional sequences, for example, a barcode sequence **1222** (*e.g*., common barcode, partition-specific barcode, or other functional sequences described elsewhere herein), and/or a UMI sequence (not shown). The nucleic acid barcode molecule **1290** may comprise a capture sequence **1223** that may be complementary to another nucleic acid sequence, such that it may hybridize to a particular sequence, e.g., capture handle sequence 1213.

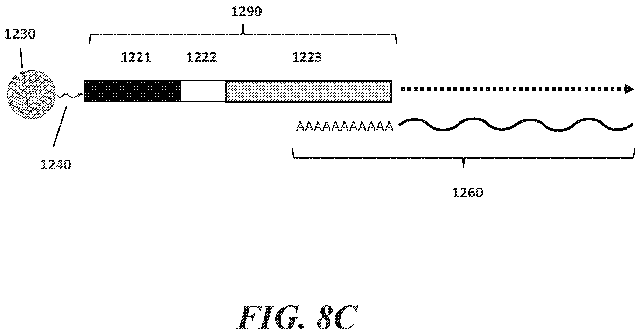

For example, capture sequence 1223 may comprise a poly-T sequence and may be used to hybridize to mRNA. Referring to **FIG. 8C****,** in some embodiments, nucleic acid barcode molecule **1290** comprises capture sequence 1223 complementary to a sequence of RNA molecule **1260** from a cell. In some instances, capture sequence 1223 comprises a sequence specific for an RNA molecule. Capture sequence 1223 may comprise a known or targeted sequence or a random sequence. In some instances, a nucleic acid extension reaction may be performed, thereby generating a barcoded nucleic acid product comprising capture sequence **1223,** the functional sequence **1221,** barcode sequence **1222,** any other functional sequence, and a sequence corresponding to the RNA molecule **1260.**

In another example, capture sequence **1223** may be complementary to an overhang sequence or an adapter sequence that has been appended to an analyte. For example, referring to **FIG. 8B****,** panel **1201,** in some embodiments, primer **1250** comprises a sequence complementary to a sequence of nucleic acid molecule **1260** (such as an RNA encoding for a BCR sequence) from a biological particle. In some instances, primer **1250** comprises one or more sequences **1251** that are not complementary to RNA molecule **1260.** Sequence **1251** may be a functional sequence as described elsewhere herein, for example, an adapter sequence, a sequencing primer sequence, or a sequence the facilitates coupling to a flow cell of a sequencer. In some instances, primer **1250** comprises a poly-T sequence. In some instances, primer **1250** comprises a sequence complementary to a target sequence in an RNA molecule. In some instances, primer **1250** comprises a sequence complementary to a region of an immune molecule, such as the constant region of a TCR or BCR sequence. Primer **1250** is hybridized to nucleic acid molecule **1260** and complementary molecule **1270** is generated (*see* Panel **1202**). For example, complementary molecule **1270** may be cDNA generated in a reverse transcription reaction. In some instances, an additional sequence may be appended to complementary molecule **1270.** For example, the reverse transcriptase enzyme may be selected such that several non-templated bases **1280** (e.g., a poly-C sequence) are appended to the cDNA. In another example, a terminal transferase may also be used to append the additional sequence. Nucleic acid barcode molecule **1290** comprises a sequence **1224** complementary to the non-templated bases, and the reverse transcriptase performs a template switching reaction onto nucleic acid barcode molecule **1290** to generate a barcoded nucleic acid molecule comprising cell (e.g., partition specific) barcode sequence **1222** (or a reverse complement thereof) and a sequence of complementary molecule **1270** (or a portion thereof). In some instances, sequence **1223** comprises a sequence complementary to a region of an immune molecule, such as the constant region of a TCR or BCR sequence. Sequence **1223** is hybridized to nucleic acid molecule **1260** and a complementary molecule **1270** is generated. For example complementary molecule **1270** may be generated in a reverse transcription reaction generating a barcoded nucleic acid molecule comprising cell (e.g., partition specific) barcode sequence **1222** (or a reverse complement thereof) and a sequence of complementary molecule **1270** (or a portion thereof). Additional methods and compositions suitable for barcoding cDNA generated from mRNA transcripts including those encoding V(D)J regions of an immune cell receptor and/or barcoding methods and composition including a template switch oligonucleotide are described in International Patent Application WO2018/075693, U.S. Patent Publication No. 2018/0105808, U.S. Patent Publication No. 2015/0376609, filed June 26, 2015, and U.S. Patent Publication No. 2019/0367969. Sequence 1223 may comprise a template switching oligonucleotide.

Any suitable agent may degrade beads. Suitable agents may include, but are not limited to, changes in temperature, changes in pH, reduction, oxidation and exposure to water or other aqueous solutions.

### J. Combinatorial barcoding

In some instances, barcoding of a nucleic acid molecule may be done using a combinatorial approach. In such instances, one or more nucleic acid molecules (which may be comprised in a cell, e.g., a fixed cell, or cell bead) may be partitioned (e.g., in a first set of partitions, e.g., wells or droplets) with one or more first nucleic acid barcode molecules (optionally coupled to a bead). The first nucleic acid barcode molecules or derivative thereof (e.g., complement, reverse complement) may then be attached to the one or more nucleic acid molecules, thereby generating first barcoded nucleic acid molecules, e.g., using the processes described herein. The first nucleic acid barcode molecules may be partitioned to the first set of partitions such that a nucleic acid barcode molecule, of the first nucleic acid barcode molecules, that is in a partition comprises a barcode sequence that is unique to the partition among the first set of partitions. Each partition may comprise a unique barcode sequence. For example, a set of first nucleic acid barcode molecules partitioned to a first partition in the first set of partitions may each comprise a common barcode sequence that is unique to the first partition among the first set of partitions, and a second set of first nucleic acid barcode molecules partitioned to a second partition in the first set of partitions may each comprise another common barcode sequence that is unique to the second partition among the first set of partitions. Such barcode sequence (unique to the partition) may be useful in determining the cell or partition from which the one or more nucleic acid molecules (or derivatives thereof) originated.

The first barcoded nucleic acid molecules from multiple partitions of the first set of partitions may be pooled and re-partitioned (e.g., in a second set of partitions, e.g., one or more wells or droplets) with one or more second nucleic acid barcode molecules. The second nucleic acid barcode molecules or derivative thereof may then be attached to the first barcoded nucleic acid molecules, thereby generating second barcoded nucleic acid molecules. As with the first nucleic acid barcode molecules during the first round of partitioning, the second nucleic acid barcode molecules may be partitioned to the second set of partitions such that a nucleic acid barcode molecule, of the second nucleic acid barcode molecules, that is in a partition comprises a barcode sequence that is unique to the partition among the second set of partitions. Such barcode sequence may also be useful in determining the cell or partition from which the one or more nucleic acid molecules or first barcoded nucleic acid molecules originated. The second barcoded nucleic acid molecules may thus comprise two barcode sequences (e.g., from the first nucleic acid barcode molecules and the second nucleic acid barcode molecules).

Additional barcode sequences may be attached to the second barcoded nucleic acid molecules by repeating the processes any number of times (e.g., in a split-and-pool approach), thereby combinatorically synthesizing unique barcode sequences to barcode the one or more nucleic acid molecules. For example, combinatorial barcoding may comprise at least 1, 2, 3, 4, 5, 6, 7, 8, 9, 10 or more operations of splitting (e.g., partitioning) and/or pooling (e.g., from the partitions). Additional examples of combinatorial barcoding may also be found in International Patent Publication Nos. WO2019/165318.

Beneficially, the combinatorial barcode approach may be useful for generating greater barcode diversity, and synthesizing unique barcode sequences on nucleic acid molecules derived from a cell or partition. For example, combinatorial barcoding comprising three operations, each with 100 partitions, may yield up to 106 unique barcode combinations. In some instances, the combinatorial barcode approach may be helpful in determining whether a partition contained only one cell or more than one cell. For instance, the sequences of the first nucleic acid barcode molecule and the second nucleic acid barcode molecule may be used to determine whether a partition comprised more than one cell. For instance, if two nucleic acid molecules comprise different first barcode sequences but the same second barcode sequences, it may be inferred that the second set of partitions comprised two or more cells.

In some instances, combinatorial barcoding may be achieved in the same compartment. For instance, a unique nucleic acid molecule comprising one or more nucleic acid bases may be attached to a nucleic acid molecule (e.g., a sample or target nucleic acid molecule) in successive operations within a partition (e.g., droplet or well) to generate a first barcoded nucleic acid molecule. A second unique nucleic acid molecule comprising one or more nucleic acid bases may be attached to the first barcoded nucleic acid molecule molecule, thereby generating a second barcoded nucleic acid molecule. In some instances, all the reagents for barcoding and generating combinatorially barcoded molecules may be provided in a single reaction mixture, or the reagents may be provided sequentially.

In some instances, cell beads comprising nucleic acid molecules may be barcoded. Methods and systems for barcoding cell beads are further described in PCT/US2018/067356 and U.S. Pat. Pub. No. 2019/0330694.

### IV. DEFINITIONS

Unless defined otherwise, all technical and scientific terms used herein have the meaning commonly understood by a person skilled in the art to which this invention belongs.

Technical and scientific terms used herein have the meanings commonly understood by one of ordinary skill in the art, unless otherwise defined. Any suitable materials and/or methodologies known to those of ordinary skill in the art can be utilized in carrying out the methods described herein.

"A", "an", and "the", as used herein, can include plural referents unless expressly and unequivocally limited to one referent.

As used herein, the term "comprising" is intended to mean that the compounds, compositions and methods include the recited elements, but not exclude others. "Consisting essentially of" when used to define compounds, compositions and methods, shall mean excluding other elements of any essential significance to the combination. Thus, a composition consisting essentially of the elements as defined herein would not exclude trace contaminants, e.g., from the isolation and purification method and pharmaceutically acceptable carriers, preservatives, and the like. "Consisting of" shall mean excluding more than trace elements of other ingredients. Embodiments defined by each of these transition terms are within the scope of this technology.

All numerical designations, e.g., mass, temperature, time, and concentration, including ranges, are approximations which are varied (+) or (-) by increments of 1, 5, or 10%. It is to be understood, although not always explicitly stated that all numerical designations are preceded by the term "about."

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number. If the degree of approximation is not otherwise clear from the context, "about" means either within plus or minus 10% of the provided value, or rounded to the nearest significant figure, in all cases inclusive of the provided value. In some embodiments, the term "about" indicates the designated value ± up to 10%, up to ± 5%, or up to ± 1%.

Use of ordinal terms such as "first", "second", "third", etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements. Similarly, the use of these terms in the specification does not by itself connote any required priority, precedence, or order.

By "glycan" is intended any sugar or chain of sugars attached to a biomolecule such as a protein, lipid or nucleic acid.

The term "class of glycan" refers to broad subsets of glycans defined by their structure such as N-linked, O-linked or C-linked glycans. The glycan classes may further be categorized in subsets. Glycans and glycan motifs include, but are not limited to, O-GlcNAc residues, LacNac-containing glycans, Fuc-α1,2-Gal containing glycans, Galβ1-3GalNAc containing glycans, GlcNAc-OR, Neu5Acα2-3 Gal and GalT1Y289L.

"Glycan motif" refers to a specific glycan substructure within a larger glycan molecule (although some glycans can be a single sugar). Glycan motifs are characterized by the type of sugar monomer (such as, but not limited to, glucose or galactose) and the connectivity of the glycosidic bonds. See for example Gal *β*1-3GalNAc. Gal *β*1-3GalNAc refers to 2 types of unique sugar monomers, galactose and N-acetyl galactosamine, that are connected through a beta 1-3 linkage. Beta refers to the conformation (alpha or beta) and 1-3 refers to the specific carbon atom that the oxygen atom is bridging on each sugar.

Determining the presence of a glycan motif may allow determination of the glycan class or the glycan in which the glycan motif occurs. The β-linked GlcNAc motif on a Ser or Thr residue is synthesized by the enzyme O-GlcNAc transferase (OGT). The β-linked GlcNAc motif can be rapidly removed in a substoichiometric reaction. The glycome status can change rapidly as for example with O-GlcNAcylation.

Glycan classes are known in the art. Glycan classes include, but are not limited to, O-linked glycans, N-linked glycans, mucin type O-linked glycans, O-linked glycans core I and O-linked glycans core 2 classes.

Different classes of protein glycosylation include N-linked, O-linked and C-linked modifications. N-linked glycans are attached to the side chain nitrogen atoms of asparagine (Asn, N) residues found primarily within a N-X-S/T consensus sequence where X is any amino acid except proline. Many N-linked glycans are found on proteins translated into the endoplasmic reticulum where a universal glycan core structure, beginning with a β-linked N-acetylglycosamine transferred onto nascent proteins. N-linked glycan subsets include, but are not limited to, high-mannose, hybrid and complex structures. O-linked glycans do not require a universal core structure. O-linked glycans are attached to the side-chain oxygen atom of serine (Ser), threonine (Thr), tyrosine (Tyr) or hydroxylysine residues. Each type or subset of O-glycosylation is defined by the identity of the peptide-proximal glycan structure. The most common O-linked subset are mucin-type glycans, which are initiated by an α-linked N-acetylgalactosamine (GalNAc) residue attached to Ser or Thr residues. Other O-linked glycan subsets may also be initiated with Ser or Thr bound glucose, xylose, mannose or fucose residues. Another O-linked subset is O-GlcNAc. O-linked glycan complexity may be further increased by additional glycans added by glycosyltransferases to yield structures such as tetrasaccharide sialyl Lewis X. Alternatively, O-linked glycans may remain relatively simple such as a β-linked GlcNAc motif on a Ser or Thr residue.

The term "sample" as used herein generally refers to a biological sample. The biological sample may comprise any number of macromolecules, for example, cellular macromolecules. The sample may be a cell sample. The sample may be a cell line or cell culture sample. The sample may comprise one or more cells. The cell or cells may be fixed or live. The sample may include one or more cell lysates. A sample may be obtained from a subject. The sample may include one or more microbes. A sample may comprise a nucleic acid, a protein, a carbohydrate, a lipid or combinations thereof. A biological sample may be derived from another sample. The sample may be a tissue sample, such as a biopsy, core biopsy, needle aspirate or fine needle aspirate. The sample may be a fluid sample, such as but not limited to a blood sample, urine sample, saliva sample, plasma sample or serum sample. A sample may be a skin sample. A sample may be a cheek swab. The sample may be a cell-free sample or cell free. Extracellular samples may be obtained from bodily samples selected from the group consisting of blood, plasma, serum, urine, saliva, mucosal excretions, sputum, sweat, stool and tears. The sample may be selected from the group consisting of tissues, cells, fixed cells, live cells and cell lysates.

"Incubating" is intended to encompass maintaining or providing conditions favorable for or conducive to a desired reaction such as but not limited to, providing a reaction component or reagent. It is recognized that conditions favorable for or conducive to one reaction may differ from conditions favorable for another reaction. Suitable conditions for different reactions and reaction types are known in the art. By "flag molecule" is intended a molecule comprising a nucleotide sugar and a first reactive molecule of a reaction pair. The first reactive molecule is attached, coupled, linked, or connected to the nucleotide sugar. Nucleotide sugars comprise a monosaccharide portion and either a nucleoside diphosphate or nucleoside monophosphate; nucleotide sugars act as glycosyl donors in glycosylation reactions. Nucleotide sugars can include but are not limited to uridine diphosphate sugars, guanine diphosphate sugars, cytosine diphosphate sugars, cytosine monophosphate sugars, adenosine diphosphate sugars, and thymidine diphosphate sugars. Uridine diphosphate sugars include but are not limited to UDP-α-D-Glc, UDP α-D-Gal, UDP α-D-GalNAc, UDP α-D-GlcNAc, UDP α-D-GlcA, UDP α-D-GlcUA and UDP α-D-Xyl. Guanine diphosphate sugars include but are not limited to GDP-α-D-Man and GDP-β-L-Fuc. Cytosine monophosphate sugars include but are not limited to CMP-β-D-Neu5Ac. Cytosine diphosphate sugars include but are not limited to CDP-D-Ribitol and CDP-Glu. Adenosine diphosphate sugars include but are not limited to ADP-ribose and ADP-glu.

By "synthetic nucleotide sugar" is intended any synthesized nucleotide sugar produced outside of a cell or organism and any non-native nucleotide sugars. A non-native nucleotide sugar is a nucleotide sugar isolated from a cell or organism and supplied to or provided to a cell or organism that does not normally produce or contain that nucleotide sugar. It is recognized that a synthesized nucleotide sugar encompasses a nucleotide sugar isolated from a cell or organism and further chemically altered. Synthetic nucleotide sugars can include, but are not limited to, GDP-Glc, N-acetylmannosamine and N-acetylgalactosamine. A synthetic nucleotide sugar may be acetylated. A synthetic sugar may be incorporated into a glycan selected from the group consisting of sialic acid and mucin type O-linked glycans. A synthetic sugar may be glycan class-specific. A synthetic sugar may be glycan motif specific.

A "reaction pair" comprises a first reactive molecule and a second reactive molecule capable of coupling to each other. In various aspects the second reactive molecule selectively couples with the first reactive molecule of the reaction pair. Suitable reaction pairs may include, but are not limited to, an azide and an alkyne, an azide and a phosphine, an aldehyde and aminooxy, an aldehyde and a hydrazine, an aldehyde and a hydrazide, a ketone and an aminooxy, a hydrazine and a ketone, cyclopropene and a tetrazine, norbornene and tetrazine, trans-cyclooctene and tetrazine, an alkyne and a tetrazine, a nitrone and an alkene, a nitrone and alkyne, diazo and alkyne, and isonitrile and tetrazine. It is recognized that a component of a reaction pair may function as either the first reactive molecule or the second reactive molecule of the reaction pair. For example, and without limitation, a first flag molecule may comprise a nucleotide sugar and an azide as the first reactive molecule of a reaction pair with an alkyne as the second reactive molecule of a reaction pair. Alternatively, a first flag molecule may comprise a nucleotide sugar and an alkyne as the first reactive molecule of a reaction pair with an azide as the second reactive molecule of a reaction pair. Methods may utilize multiple reaction pairs to identify multiple glycans, glycan motifs or glycan classes or any combination thereof. It is further recognized that the first reactive molecule of a first reaction pair may be the second reactive molecule of a second, third, fourth or additional reaction pair; similarly the second reactive molecule of a first reaction pair may be the first reactive molecular of a different reaction pair. For example, a first flag molecule may comprise a nucleotide sugar and an azide as the first reactive molecule of a first reaction pair with an alkyne as the second reactive molecule of a reaction pair in a reporter molecule, and a second flag molecule may comprise a nucleotide sugar and an alkyne as the first reactive molecule of a second reaction pair with an azide as the second reactive molecule of a second reaction pair. It is also recognized that some reaction pairs may be preferred for use with other reaction pairs and that some reaction pairs may be preferred to not use with other reaction pairs. Those skilled in the art would select reaction pairs suitable for use with each other.

As used herein, the term "capable of coupling" is intended to encompass "capable of interacting", "capable of binding", "capable of attaching", "capable of forming an attachment", "capable of linking" and "capable of reacting". "Capable of coupling" is not intended to be limited by mechanism. In some embodiments, the first reactive molecule and the second reactive molecule of a reaction pair are selectively reactive with each other. In an embodiment, the coupling or binding of a first reactive molecule and a second reactive molecule of a reaction pair is a covalent interaction. In various embodiments, the interaction of a first reactive molecule and a second reactive molecule of a reaction pair is stable.

As used herein, the term "stable" means that the interaction or linkage is maintained until a stimulus is provided to disrupt the interaction. Any suitable stimulus may be used to disrupt the interaction including, but not limited to, chemical exposure and physical change. Chemical stimuli may include, but are not limited to, TCEP. Typically, the interaction is maintained in standard single-cell work flow conditions. In various aspects, a disrupting stimulus may be provided after partitioning of the sample occurs.

In methods involving live cells, the reaction between molecules of a reaction pair is preferably non-toxic or biorthogonal. By "biorthogonal reaction" is intended a chemical reaction that neither interacts with nor interferes with a biological system. The participating functional groups or reactive molecules are inert to biological moieties, are selectively reactive with each other under biocompatible conditions, and, for *in vivo* applications, are nontoxic to cells and organisms. In various aspects, one reactive molecule or group is small and therefore minimally perturbing of a biomolecule into which it has been introduced either chemically or biosynthetically. See Sletten & Bertozzi, Accts Chem Res. 44(9): 666-676 (2011).

"Glycosyltransferases" are enzymes that catalyze the transfer of a glycosyl residue from a donor to an acceptor substrate with defined connectivity. The acceptor substrate may be selected from the group including, but not limited to, a peptide, peptide sequence, glycan and glycan motif. Glycosyltransferases include, but are not limited to, nucleotide-phosphate using enzymes, dolichol phosphate using enzymes, oligosaccharide or polysaccharide using enzymes and nucleoside phosphorylases. Glycosyltransferases include but are not limited to "glycan specific transferases". By "glycan specific transferase" is intended a glycosyltransferase that preferentially catalyzes addition of a particular sugar at a known position to a substrate with defined connectivity.

Glycan specific transferases can include, but are not limited to, glycosyltransferase, sialyltransferase, α1,3-fucosyl transferase; BGTA; WbwK fucosyltransferase; α1,2-fucosyl transferase; β1-4 N-acetyl-galactosylaminotransferase; β-galactoside α2,6 sialyltransferase 1; and β-galactoside α2,3 sialyltransferase 1.

In various aspects of the methods, multiple glycosyltransferases may be used. In such a case, the different glycosyltransferases would be referred to as a first glycosyltransferase, a second glycosyltransferase, a third glycosyltransferase, a fourth glycosyltransferase, a fifth glycosyltransferase, or an additional glycosyltransferase. Various methods may involve the use of a first glycan specific transferase, a second glycan specific transferase, a third glycan specific transferase, a fourth glycan specific transferase, a fifth glycan specific transferase, or an additional glycan specific transferase. It is recognized that glycan specific transferases and the nucleotide sugar should be paired by preferential usage. It is recognized that one skilled in the art may select the glycosyltransferase and a preferred nucleoside sugar substrate.

The term "barcode" as used herein generally refers to a label or identifier that conveys or is capable of conveying information about a component of interest, an analyte, a reporter, or a partition. A barcode can be directly or indirectly attached to a reactive molecule. A barcode can be part of an analyte. A barcode can be independent of an analyte. A barcode can be a tag attached to an analyte (for example, a nucleic acid molecule) or a combination of the tag in addition to a reactive molecule. A barcode may be unique. A barcode may share identifying sequence regions for related molecules such as a shared origin, a shared partition or other similarity. Barcodes can have a variety of different formats. For example, barcodes may include, polynucleotide barcodes, random nucleic acid and/or amino acid sequences and synthetic nucleic acid and/or amino acid sequences. A barcode can be attached to an analyte in a reversible or irreversible manner. A barcode can be added to, for example, a fragment of a deoxyribonucleic acid (DNA) or ribonucleic acid (RNA) in a sample before, during, and/or after sequencing of the sample. Barcodes can allow for identification of individual sequence reads.

The term "subject" as used herein generally refers to an organism such as an animal, plant, microorganism, microbe, bacteria, fungi, archaea, virus, invertebrate, mammal, avian, vertebrate, rodent, primate, simian, human, farm animal, sports animal or pet. A subject can be healthy or asymptomatic, an individual at risk for or suspected of having a disease, or an individual in need of therapy or suspected of needing therapy.

Generally, reporter molecules comprise a target specific portion that directly or indirectly interacts with a target and a reporter oligonucleotide comprising a target identifying sequence. Thus, a glycan-specific reporter molecule may comprise a glycan-specific binding moiety and a reporter oligonucleotide comprising the glycan-identifying sequence. In some embodiments, a glycan-specific reporter molecule indirectly interacts with the target glycan via a flag molecule that is incorporated into a glycan motif. In particular embodiments, the flag molecule comprises a first reactive molecule of a reaction pair and the glycan-specific reporter molecule comprises the second reactive molecule of the reaction pair and a reporter molecule comprising a glycan-identifying sequence. A "component specific reporter molecule" comprises a target specific portion that directly interacts with a component of interest and a reporter oligonucleotide comprising a component identifying sequence.

An "unincorporated reporter molecule" is a reporter molecule that is not bound to its respective analyte, for example not bound directly to the target analyte, not bound to the first reactive molecule of a reaction pair, or is bound to a first reactive molecule of a reaction pair that was not incorporated into a glycan. The terms "unincorporated reporter molecule" and "unbound reporter molecule" may be used interchangeably. Removing unincorporated reporter molecules involves removing at least one reporter molecule wherein the second reactive molecule has not coupled to a first reactive molecule or wherein the second reactive molecule has coupled to a first reactive molecule but the first reactive molecule was not incorporated into a glycan. Methods of removing at least one unincorporated reporter molecule can include, but are not limited to, washing with reaction buffer and washing with an aqueous buffer. An aqueous buffer may include but is not limited to a PBS buffer comprising bovine serum albumin (BSA) and PBS plus 0.1%-1% BSA. Washing may be performed once or multiple times, such as for example three washing cycles. Unincorporated reporter molecules external to cells or absorbed by cells may be removed by any method known in the art. It is recognized that a washing cycle to remove unincorporated reporter molecules absorbed by the cell may involve longer washing cycles, additional washing cycles, or washing with a different buffer.

An "unincorporated flag molecule" is a flag molecule that has either not entered a cell, has entered a cell but has not been processed into a flag substrate, or has entered a cell but has not been a substrate for a glycosyltransferase in a living cell.

The term "partition" as used herein, generally, refers to a space or volume that may be suitable to contain one or more species or conduct one or more reactions. A partition may be a physical component, such as a droplet or well. The partition may isolate space or volume from another space or volume. The contents of a partition may remain discrete from the contents of other partitions. The droplet may be a first phase (e.g. aqueous phase) in a second phase (e.g. oil) immiscible with the first phase. The droplet may be a first phase in a second phase that does not phase separate from the first phase, such as for example, a capsule or liposome in an aqueous phase. A partition may comprise one or more other (inner) partitions. In some cases, a partition may be a virtual component that can be defined and identified by an index across multiple and/or remote physical compartments. For example, a physical compartment may comprise a plurality of virtual components. It is recognized that one or more inner partitions may be caused to be disrupted, releasing the contents of the inner partition to the larger partition. The timing of the disruption or release may be adjusted as desired.

The term "partitioning" as used herein is intended to encompass parting, dividing, depositing, separating, or compartmentalizing into one or more partitions. Systems and methods for partitioning of one or more particles (such as, but not limited to, biological particles, macromolecular constituents of biological particles, beads, reagents, etc.) into discrete compartments or partitions (referred to interchangeably here as partitions), wherein each partition maintains separation of its own content from the contents of other partitions are known in the art. See for example US 2020/0032335. The partition can be a droplet in an emulsion. A partition may comprise one or more other partitions.

A "plurality of nucleic acid barcode molecules" may comprise at least about 500 nucleic acid barcode molecules, at least about 1,000 nucleic acid barcode molecules, at least about 5,000 nucleic acid barcode molecules, at least about 10,000 nucleic acid barcode molecules, at least about 50,000 nucleic acid barcode molecules, at least about 100,000 nucleic acid barcode molecules, at least about 500,000 nucleic acid barcode molecules, at least about 1,000,000 barcode molecules, at least about 5,000,000 nucleic acid barcode molecules, at least about 10,000,000 nucleic acid barcode molecules, at least about 100,000,000 nucleic acid barcode molecules, at least about 1,000,000,000 nucleic acid barcode molecules. In some cases, a plurality of nucleic acid barcode molecules comprise a partition-specific barcode sequence.

Each of the plurality of nucleic acid barcode molecules may include an identifier sequence separate from the partition-specific barcode sequence, where the identifier sequence is different for each nucleic acid partition-specific barcode molecule of the plurality of nucleic acid partition specific barcode molecules. In some cases, such an identifier sequence is a unique molecular identifier (UMI) as described elsewhere herein. As described elsewhere herein, UMI sequences can uniquely identify a particular nucleic acid molecule that is barcoded, which may be identifying particular nucleic acid molecules that are analyzed, counting particular nucleic acid molecules that are analyzed, etc. Furthermore, in some cases, each of the plurality of nucleic acid barcode molecules can comprise the partition specific barcode sequence and the bead can be from plurality of beads, such as a population of barcoded beads. Each of the partition specific barcode sequences can be different from partition specific barcode sequences of nucleic acid barcode molecules of other beads of the plurality of beads. Where this is the case, a population of barcoded beads, with each bead comprising a different partition specific barcode sequence can be analyzed.

A nucleic acid barcode molecule of a plurality of nucleic acid molecules may be used to generate a "barcoded nucleic acid molecule". A first "barcoded nucleic acid molecule" comprises a partition-specific barcode sequence and may further comprise one or more additional barcode sequences. In some cases, a first barcoded nucleic acid molecule comprises a reporter barcode sequence. A reporter barcode sequence may identify a first glycan motif, second glycan motif, a third glycan motif, a fourth glycan motif, a fifth glycan motif, a sixth glycan motif, a seventh glycan motif, an eighth glycan motif, a ninth glycan motif, or a tenth glycan motif. In some cases, a barcoded molecule comprises a glycan-component reporter sequence. A glycan-component reporter sequence comprises a glycan specific or glycan motif specific reporter barcode sequence, a component specific barcode sequence and may further comprise a splint oligonucleotide sequence. In some cases, a barcoded molecule comprises a different reporter barcode sequence that identifies a second analyte. A different reporter barcode sequence or an analyte-specific barcode sequence may identify a protein, a lipid, a metabolite or other second analyte.

A "glycan-motif specific reporter barcode sequence" is a barcode sequence assigned to a glycan motif. A first glycan-motif specific reporter barcode sequence is a barcode sequence that is assigned to a first glycan motif; it can be used to identify one or more instances of the first glycan motif. A second glycan-motif specific reporter barcode sequence is a barcode sequences that is assigned to a second glycan motif; it can be used to identify one or more instances of the second glycan motif. A third glycan-motif specific reporter barcode sequence is a barcode sequences that is assigned to a third glycan motif; it can be used to identify one or more instances of the third glycan motif. A fourth glycan-motif specific reporter barcode sequence is a barcode sequences that is assigned to a fourth glycan motif; it can be used to identify one or more instances of the fourth glycan motif. A fifth glycan-motif specific reporter barcode sequence is a barcode sequences that is assigned to a fifth glycan motif; it can be used to identify one or more instances of the fifth glycan motif. A sixth glycan-motif specific reporter barcode sequence is a barcode sequences that is assigned to a sixth glycan motif; it can be used to identify one or more instances of the sixth glycan motif. A seventh glycan-motif specific reporter barcode sequence is a barcode sequences that is assigned to a seventh glycan motif; it can be used to identify one or more instances of the seventh glycan motif. An eighth glycan-motif specific reporter barcode sequence is a barcode sequences that is assigned to an eighth glycan motif; it can be used to identify one or more instances of the eighth glycan motif. A ninth glycan-motif specific reporter barcode sequence is a barcode sequences that is assigned to a ninth glycan motif; it can be used to identify one or more instances of the ninth glycan motif. A tenth glycan-motif specific reporter barcode sequence is a barcode sequences that is assigned to a tenth glycan motif; it can be used to identify one or more instances of the tenth glycan motif. It is understood that a glycan motif specific reporter barcode sequence may further comprise an additional unique molecular identifying sequence that distinguishes each original instance of a glycan motif specific reporter barcode sequence.

By "derivative" of a barcoded nucleic acid molecule is intended an amplification product, the product of reverse transcription, and a complement of the barcoded nucleic acid molecule.

Methods of determining the sequence of a nucleic acid molecule may include sequencing. In some embodiments, the sequencing is high throughput sequencing. Sequencing methods include, but are not limited to, sequencing performed by a system such as a sequencing system by Illumina^{™}, Pacific Biosciences (PacBio^{™}), Oxford Nanopore^{™}, or Life Technologies (Ion Torrent^{™}). Alternatively or in addition, sequencing may be performed using nucleic acid amplification, polymerase chain reaction (PCR) (e.g. digital PCR, quantitative PCR) or real time PCR) or isothermal amplification. Some sequencing systems provide sequencing reads (also "reads" herein).

The term "bead" as used herein, generally refers to a particle. The bead may be a solid or semi-solid particle. The bead may be a gel bead. The gel bead may include a polymer matrix (e.g. matrix formed by polymerization or cross-linking). The polymer matrix may include one or more polymers (e.g. polymers having different functional groups or repeat units). Polymers in the polymer matrix may be randomly arranged such as in random copolymers. Crosslinking can be via covalent, ionic or inductive interactions or physical entanglement. The bead may be a macromolecule. The bead may be formed of nucleic acid molecules bound together. The bead may be formed via covalent or non-covalent assembly of molecules (e.g. macromolecules) such as monomers or polymers. Such polymers or monomers may be natural or synthetic. Such polymers or monomers may be or include, for example, nucleic acid molecules (e.g. DNA or RNA). The bead may be formed of a polymeric material. The bead may be magnetic or non-magnetic. The bead may be rigid. The bead may be flexible and/or compressible. The bead may be disruptable or dissolvable. A semi-solid bead may be a liposomal bead. The bead may be a solid particle (e.g. a metal-based particle including but not limited to iron oxide, gold or silver) covered with a coating comprising one or more polymers. Such coating may be disruptable or dissolvable.

By "analytic processing step" is intended a processing step deliberately performed by a human or directly or indirectly caused to be performed by a human. Analytic processing steps include but are not limited to, a process, reaction, experimental step, incubation, exposure to an agent of interest, partition lysis, cell lysis, filtration, separation, and other experimental or analytical processes. By "metabolic processing step" is intended a reaction that occurs within a cell, extracellular matrix or virus.

By "component of interest" is intended any type of biological or bioreactive molecule. A component of interest may be selected from the group consisting of, but not limited to, a protein, glycan, sugar, nucleotide sugar or synthetic nucleotide sugar. A component of interest may be located on the same cell as a glycan of interest, on a different cell, or in an extracellular milieu. The component of interest may be transiently available or constitutively available. If the component of interest is transiently available, its presence may be induced by a known stimulus or by an undetermined stimulus. It is understood that cellular processes including metabolism, communication and disease or disorder processes could alter the availability of a component of interest.

By "analyte" is intended a biological molecule. Analytes include but are not limited to a DNA analyte, an RNA analyte, an oligonucleotide, a reporter molecule, a reporter molecule configured to directly couple to a protein, a reporter molecule configured to indirectly couple to a protein, a reporter molecule configured to directly couple to a metabolite, and a reporter molecule configured to indirectly couple to a metabolite.

By "binding sequence" is intended a nucleic acid sequence capable of binding to an analyte.

By "in proximity to" is intended the molecules of interest are or were located physically close enough to each other such that when a first reporter molecule comprising a reporter barcode sequence is attached to a first molecule of interest and a second reporter molecule comprising a reporter barcode sequence is attached to a second molecule of interest, in the presence of a splint, the reporter barcode sequence on the first reporter molecule and the reporter barcode sequence on the second reporter molecule can be ligated together. In an aspect, the first molecule of interest is a glycan and the second molecule of interest is a component of interest. For example, the methods may involve a glycan-specific molecule comprising a reporter oligonucleotide comprising a glycan-specific reporter barcode sequence and a component specific molecule comprising an oligonucleotide comprising a component specific barcode sequence. The methods may involve providing a splint and performing a ligation reaction with the oligonucleotide comprising a glycan-specific reporter barcode sequence and the oligonucleotide comprising a component specific barcode to generate a glycan-component reporter sequence. It is recognized that the size of either reporter molecule, the length of either barcode sequence, the size and flexibility of any linker region (if present) and the length of the splint may impact the actual distance between two molecules considered "in proximity" to each other. It is understood the molecules of interest may be present on the same cell, on adjacent cells, in an extracellular milieu around a cell and on a cell, on or in the same organelle or cellular component, and on or in separate organelles or cellular components within the same cell.

Each glycan-specific molecule is capable of coupling, attaching, binding, or linking to a glycan of interest or to a glycan motif of interest in a preferential manner. As used herein, by "in a preferential manner" is intended that coupling, attaching, binding or linking to a molecule of interest is at least 2%, at least 5%, at least 10%, at least 15%, at least 20%, at least 25%, at least 30%, at least 40%, at least 50%, at least 60%, at least 70%, at least 80%, at least 90%, at least 100% or more than the coupling, attaching, binding or linking to another molecule. A glycan-specific molecule may couple, attach, bind, or link to a glycan or glycan motif of interest at least 2%, at least 5%, at least 10%, at least 15%, at least 20%, at least 25%, at least 30%, at least 40%, at least 50%, at least 60%, at least 70%, at least 80%, at least 90%, at least 100% or more than to a different glycan or glycan motif. Glycan specific molecules can include, but are not limited to, glycan specific lectins, glycan specific antibodies, synthetic nucleotide sugars, synthetic sugars and inactivated glycan specific transferases. Glycans and glycan motifs of interest include, but are not limited to, O-GlcNAc residues, LacNac-containing glycans, Fuc-α1,2-Gal containing glycans, Galβ1-3GalNAc-containing glycans, GlcNAc-O-R, Neu5Acα2-3 Gal and GalTIY289L.

A component specific molecule is capable of coupling, attaching, binding, or linking to a component of interest in a preferential manner. A component of interest may be, but is not limited to, a protein, glycan, glycan motif, sugar, nucleotide sugar and synthetic nucleotide sugar. Component specific molecules may include, but are not limited to, antibodies, lectins, synthetic nucleotide sugars, nucleotide sugars and synthetic sugars.

A splint is a joining molecule capable of binding two oligonucleotides. Splint molecules can include, but are not limited to a nucleic acid molecule, an oligonucleotide, triazole, and oligonucleotides comprising a nucleotide barcode sequence.

The terms "unique molecular identifier", "unique molecular identifying sequence", "UMI" and "UMI sequence" are used synonymously. Within any given partition, individual barcoded molecules may comprise a common barcode sequence such as a partition specific sequence. The barcoded molecules may further vary in a unique molecular identifying sequence segment.

The ability to attribute characteristics to individual glycans, glycan classes or glycan motifs is provided by the assessment of unique identifiers specifically to an individual glycan, glycan class or glycan motif or group of glycans, glycan class or glycan motifs. Unique identifiers in the form of nucleic acid barcodes can be assigned or associated with glycans, glycan motifs or glycan classes or populations of glycans, glycan motifs or glycan classes. These unique identifiers can then be used to attribute the glycan, glycan motifs to an individual biological particle or group of biological particles. The nucleic acid molecules are partitioned such that as between nucleic acid molecules in a given partition, the partition-specific barcode sequences contained therein are the same, but as between different partitions, the nucleic acid molecules can and do have differing barcode sequences or at least represent a large number of different barcode sequences across all of the partitions in a given analysis. In some aspects only one partition specific barcode sequence is associated with a given partition, although in some aspects, two or more different barcode sequences may be present.

### EXAMPLES

### Example 1. Single-cell profiling of glycome using chemoenzymatic glycan labeling

This example illustrates a process for chemoenzymatic labeling of glycan for single cell profiling for detecting peripheral higher order glycans that play important roles in cell signaling and disease state.

Chemoenzymatic labeling is used to detect unique class of higher order glycans that are labeled with specific linked monosaccharide building block. Chemoenzymatic labeling permits the detection of higher order glycans, such as mucin O-linked glycans, sialylated, fucosylated glycans, and cytosolic O-GlcNAcylated proteins when combined with multimodal single-cell profiling technologies. Chemoenzymatic labeling requires a monosaccharide building block bearing a chemically reactive tag. The modified monosaccharide, when taken up by cells and metabolized, is incorporated into cell-surface glycoconjugates. Example of modified monosaccharide that can be used are UDP-GalNAc; GDP-fucose; or CMP-Sialic acid. The bioorthogonal chemical tag then allows covalent conjugation with fluorescent probes for visualization, with affinity probes for enrichment and glycomic analysis, or oligonucleotide barcodes for single cell profiling.

The methods disclosed herein use small-molecule probes to detect cell-surface polysaccharides, such as Type II N-acetyllactosamine (LacNAc, Gal(pi,4)GlcNAc) to generate an activated and chemically tagged sugar; and rely on a glycan transfer enzyme (e.g., 1,3 fucosyltransferase; GalT1 (β1-4 galatosyltransferase); α1-3 FucT (α1-3 fucosyltransferase 3); BgtA (human blood group A antigen glycosyltransferase); WbwK (α1-2 fucosyltransferase);BgtA; CgtA (β1-4 N-acetylgalactosaminyltransferase); ST6Gal1 (β galactoside α2-6 sialyltransferase 1); ST3Gal1 (β galactoside α2-3 sialyltransferase 1)) to transfer the activated and chemically tagged sugar (e.g. fucose) to the polysaccharide (e.g. LacNAc residue). Additional target polysaccharides are GlcNAc-O-R; LacNAc; Fucα1-2Gal; Galβ1-3GalNAc; Neu5Acα2-3Gal; N-glycans (e.g.,LacNAc); or O-glycans (e.g., mostly core 1 and 2 glycans). The glycan transfer enzyme can be endogenous enzymes or genetically engineered enzymes with higher specificity for a particular types of glycans.

The newly labeled glycoconjugates (e.g., glycoprotein) are subsequently linked to an oligonucleotide barcode using biorthogonal chemical reaction in live cells. The biorthogonal chemical reactions include azide- or cyclooctyne-functionalized oligonucleotide barcodes coupled to biocompatible copper-catalyzed azide-alkyne cycloadditon (CuAAC) or copper-free click chemistry. Biorthogonal chemical reactions are known to those skilled in the art. *See e.g.,* Soriano del Amo et al., J Am Chem Soc. 132:16893-16899 (2010); Jewett JC et al., Chem Soc Rev. 39:1272-1279 (2010); and Zheng et al., Angew Chem Int Ed Engl. 50(18): 4113-4118 (2011). Cells comprising labeled glycoconjugates are then prepared for single-cell profiling using the methods disclosed herein and methods known in the art. Cells can be wild-type cells or cells engineered to express engineered glycosyltransferases.

A flag molecule comprising a nucleotide sugar and a first reactive molecule of a reaction pair is prepared. The flag molecule and a first glycan specific transferase are incubated with a sample. The glycan specific transferase uses the flag molecule comprising a nucleotide sugar and a first reactive molecule as a substrate and incorporates the nucleotide sugar into a glycan. The first reactive molecule of a reaction pair remains attached to the nucleotide sugar upon incorporation into the glycan. The sample is incubated with a reporter molecule comprising a second reactive molecule of a reaction pair and glycan motif-specific reporter barcode sequence. The second reactive molecule of a reaction pair couples with the first reactive molecule of a reaction pair that is attached to the nucleotide sugar in the glycan. Unbound reporter molecules are removed by washing three times with buffer.

The sample is partitioned into a plurality of beads such that each bead comprises a single cell and a plurality of nucleic acid barcode molecules comprising a partition-specific barcode sequence. The reporter oligonucleotide on the reporter molecule and a nucleic acid barcode molecule of the plurality of nucleic acid barcode molecules generates a first barcoded nucleic acid molecule. The first barcoded nucleic acid molecule comprises a partition-specific barcode sequence or complement thereof and the reporter barcode sequence or complement thereof. The sequence of the barcoded nucleic acid molecule is determined and the partition-specific barcode sequence and reporter barcode sequence are identified. The identified partition-specific barcode sequence and reporter barcode sequence are used to back-determine the presence of at least one glycan in the cell. The abundance of at least one glycan or glycan motif is also determined.

The results from this experiment will provide information regarding the single-cell profiling of numerous glycans including, but not limited to, glycans containing O-GlcNAc, type II LacNAc-containing glycans; Fucα1-2Gal-containing glycans; glycans containing terminal Galβ1-3GalNAc; Neu5Acα2-3Gal containing glycans. The bioorthogonal ligations of various the hydrazine/ketone and alkyne/azide pairs with different feature oligonucleotide barcodes will allow the simultaneous identification of various glycan using the multimodal single-cell sequencing technologies disclosed herein. To detect the GlcNAc-O-R containing glycans, cells overexpressing β1-4 galatosyltransferase (GalT1) will be used with unnatural UDP-GalNAc. To detect LacNAc containing glycans, α1-3 fucosyltransferase 3 (α1-3 FucT) will be used with unnatural GDP-fucose. To detect Fucα1-2Gal containing glycans, human blood group A antigen glycosyltransferase (BgtA) will be used with unnatural UDP-GalNAc. To detect Galβ1-3GalNAc containing glycans, α1-2 fucosyltransferase (WbwK or BgtA) will be used with unnatural GDP-fucose or UDP-GalNAc. To detect Neu5Acα2-3Gal containing glycans, β1-4 N-acetylgalactosaminyltransferase (CgtA) will be used with unnatural UDP-GalNAc. To detect N-glycans, a β galactoside α2-6 sialyltransferase 1(ST6Gal1) will be used with unnatural CMP-Sialic acid (CMP-Sia). To detect O-glycans, β galactoside α2-3 sialyltransferase 1 (ST3Gal1) will be used with unnatural CMP-Sia. The results will provide a comprehensive snapshot of the glycosylation pattern of a single cell under physiological conditions with higher specificity and sensitivity than the traditional methods known in the art.

### Example 2. Metabolic Labeling of glycan for single-cell profiling analysis

This examples illustrates the metabolic labeling of oligosaccharide to detect various glycosylated proteins or lipids, including but not limited to sialylated, fucosylated, and/or mucin-type O-linked glycans in live cells.

A sample of live cells is incubated with a flag molecule comprising a synthetic sugar and an azide under conditions that allow the flag molecule to be absorbed by a cell. The flag molecule is a flag substrate for a glycosyltransferase of the living cells. The flag molecule can be a sialic acid precursor peracetylated N-α-azidoacetylmannosamine (Ac4ManNAz); peracetylated N-azidoacetylgalactosamine (Ac4GalNAz); an azido analog of GalNAc (Ac4GalNAz), N-propanoyl mannosamine (ManNProp), N-butanoyl mannosamine (ManNBut); N-pentanoyl mannosamine (ManNPent)N-acetyl mannosamine (ManNAc) analogs; fucose analogs; sialic acid analogs, GlcNAc analogs; unnatural sialic acid analogs; unnatural mannose analogs; unnatural galactose analogs; unnatural N-acetylglucosamine analogs; or N-acetylgalactosamine analogs. Additional unnatural analogs are known to those skilled in the art, for example, Mbua et al., Proc. Natl. Acad. Sci. U. S. A. 110:10207-10212 (2013). Each of the analogs can be used at about 25 µM and the cells are incubated for up to 2 days. Following the incubation period, the cells are washed three times with buffer. A glycosyltransferase incorporates the synthetic sugar and azide (the flag substrate or unnatural analogs) into a glycan. Cells are the incubated with a reporter molecule comprising an alkyne and a reporter oligonucleotide comprising a glycan-motif specific reporter barcode sequence. The cells are washed three times with buffer to remove unbound reporter molecules. In the cell, the azide and the alkyne couple, indirectly tagging the glycan with the reporter oligonucleotide. The sample is partitioned into a plurality of cell beads, such that a plurality of cell beads comprises one cell from the sample and a plurality of nucleic acid barcode molecules comprising a partition-specific barcode sequence. Each labeled glycan will be coded with a unique glycan-motif specific reporter barcode sequence. The cell-beads are encapsulated into droplets comprising a ligase. The reporter oligonucleotide and a nucleic acid barcode molecule comprising a partition-specific barcode are ligated together. The barcoded nucleic acid molecule is sequenced to identify the partition-specific barcode sequence and the glycan motif-specific reporter barcode sequence. The identified partition-specific barcode sequence and reporter barcode sequence are used to back-determine the presence of at least one glycan in the cell. The abundance of at least one glycan or glycan motif is also determined.

It is expected that treating cells with these unnatural monosaccharide precursors will lead to general labeling of cell surface glycans containing that unnatural monosaccharide. The single cell profiling analysis will provide the glycome profile of each cell. In addition, the single cell profiling will provide the abundance of each type of glycan on the cell surface based on a sequenceable readout at the single cell level. Furthermore, it is expected that the results will identify and distinguish subsets of cells with unique glycosylation profiles.

### Example 3. Protein-specific glycosylation Analysis

This example illustrates the analysis of protein-specific glycosylation patterns using a multimodal single-cell profiling methodology.

Protein glycosylation is one of the most ubiquitous modifications of eukaryotic proteins as it is estimated that over 50% of all eukaryotic proteins are glycosylated. Two main forms of protein glycosylation pattern exists: N-linked and O-linked glycosylation. The glycosylation pattern of the protein is dependent on the tissue of origin of that protein and reflects the status of the cell. In addition, aberrant glycosylation has been associated with several illnesses including cancer development and progression. Glycosylation also affects the biological activities of numerous proteins. For example, the activity of many antibodies is dependent on the glycosylation pattern of the antibody. O-glycan alteration, such as the expression of the truncated O-glycan epitope sialyl Tn (STn) is involved in cancer development and progression. In addition, the activity of cell adhesion molecules, such as E-cadherin, is influenced by glycosylation. Alteration of a branched N-glycan structure in E-cadherin, or the addition of the β1,6GlcNAc branched glycans to E-cadherin, disturbs the normal function of the protein and promotes tumor cell development and progression. Conversely, the presence of a bisecting N-acetlyglucosamine (GlcNAc) N-glycans in E-cadherin inhibits E-cadherin-mediated cancer progression and prevents epithelial-to-mesenchymal transition process.

A sample is incubated with a glycan motif specific molecule comprising a reporter oligonucleotide comprising a glycan-motif specific reporter barcode sequence. An antibody to a component of interest is provided; the antibody is coupled to an oligonucleotide comprising a component specific barcode sequence. The sample is washed three times to remove unincorporated glycan motif specific molecules and unincorporated component specific molecules. A splint is provided to the sample. A ligation reaction is performed. The glycan motif specific reporter barcode sequence and component specific barcode sequence are ligated together to generate a glycan-component reporter sequence. The sample is partitioned into a plurality of beads. The glycan-component reporter sequence and a nucleic acid barcode molecule comprising a partition-specific barcode sequence form a barcoded molecule.

The single cell profiling provides a sensitive and a specific detection of glycosylated proteins in a single cell by providing a comprehensive snapshot of protein-specific glycosylation state in that single cell. The approach disclosed herein is better than approaches known in the art because it provides a triple recognition system for the detection of the proteins and their post-translation modifications in a single cell under physiological conditions. The method disclosed herein is an optimized version of Proximity ligation assay (PLA), which is an immunoassay developed to detect protein molecules through DNA ligation and signal amplification. When combined with single-cell profiling, this optimized PLA assay is highly specific and sensitive when compared to PLA alone. The detection of protein specific glycosylation as disclosed herein may be used to identify disease biomarkers, and will offer an unprecedented knowledge into post-translation modifications associated with specific cell types in health and disease and associated changes in protein glycosylation pattern.

As will be understood by one skilled in the art, for any and all purposes, particularly in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, *etc.* As a non-limiting example, each range discussed herein can be readily broken down into a lower third, middle third and upper third, *etc.* As will also be understood by one skilled in the art all language such as "up to," "at least," "greater than," "less than," and the like, include the number recited and refer to ranges which can be subsequently broken down into subranges as discussed above. Finally, as will be understood by one skilled in the art, a range includes each individual member. Thus, for example, a group having 1-3 cells refers to groups having 1, 2, or 3 cells. Similarly, a group having 1-5 cells refers to groups having 1, 2, 3, 4, or 5 cells, and so forth.

## Claims

1. A method of determining the presence of one or more glycans in a sample comprising the steps of:
(a) incubating the sample with (i) a first flag molecule comprising a nucleotide sugar and a first reactive molecule of a reaction pair and (ii) a first glycan specific transferase,
wherein the first flag molecule is a substrate for the first glycan specific transferase;
wherein the first flag molecule is incorporated onto a glycan-modified glycoprotein in the sample, and
wherein the sample is selected from the group consisting of a tissue, a cell, a fixed cell, a live cell, and cell lysates;
(b) admixing the sample with a first reporter molecule comprising (i) a second reactive molecule of the reaction pair and (ii) a first reporter oligonucleotide comprising a first glycan motif-specific reporter barcode sequence, wherein the second reactive molecule of the reaction pair couples to the first reactive molecule of the reaction pair, whereby the first reporter molecule is conjugated to the glycan-modified glycoprotein via the first flag molecule;
(c) removing unincorporated reporter molecules from the sample;
(d) partitioning the sample into a plurality of partitions such that a partition comprises (i) a single cell or single cell lysate from the sample and (ii) a plurality of nucleic acid barcode molecules comprising a partition-specific barcode sequence;
(e) using the first reporter oligonucleotide and a nucleic acid barcode molecule of the plurality of nucleic acid barcode molecules to generate a first glycan barcoded nucleic acid molecule comprising the partition-specific barcode sequence or a complementary sequence thereof and the first glycan motif-specific reporter barcode sequence or complementary sequence thereof;
(f) determining the sequence of the first glycan barcoded nucleic acid molecule or derivative thereof to identify (i) the partition-specific barcode sequence or complementary sequence thereof and (ii) the first glycan motif-specific reporter barcode sequence or complementary sequence thereof; and
(g) using the identified partition-specific barcode sequence or complementary sequence thereof and the identified first glycan motif-specific reporter barcode sequence or complementary sequence thereof to determine the presence and/or abundance of at least one glycan in the sample.

2. The method of claim 1, wherein:
(a) the reaction pair is selected from the group of reaction pairs consisting of an azide and an alkyne,
an azide and a phosphine,
an aldehyde and aminooxy,
an aldehyde and a hydrazine,
an aldehyde and a hydrazide,
a ketone and an aminooxy,
a hydrazine and a ketone,
cyclopropene and a tetrazine,
norbornene and tetrazine,
trans-cyclooctene and tetrazine,
an alkyne and a tetrazine,
a nitrone and an alkene,
a nitrone and alkyne,
diazo and alkyne, and
isonitrile and tetrazine; and/or
(b) the first glycan specific transferase is selected from the group consisting of a β1-4 galatosyltransferase, a glycosyltransferase, sialyltransferase, α1-3-fucosyl transferase; a human blood group A antigen glycosyltransferase (BgtA); WbwK fucosyltransferase; α1-2-fucosyl transferase; β1-4 N-acetyl-galactosylaminotransferase; β-galactoside α2-6 sialyltransferase 1; and β-galactoside α2-3 sialyltransferase 1; ST3Gal1; ST6Gal1; and CgtA; and/or
(c) the first flag molecule is selected from the group consisting of UDP-GalNAc; GDP-fucose; UDP-GalNAc; and CMP-Sia; and/or
(d) the glycan-modified glycoprotein comprises a glycan selected from the group consisting of GlcNAc-O-R; LacNAc; Fucα1-2Gal; Galβ1-3GalNAc; Neu5Acα2-3Gal; N-glycans; and O-glycans.

3. The method of claim 1 or claim 2, further comprising:
(a) incubating the sample with (i) a second flag molecule comprising a second nucleotide sugar and a first reactive molecule of a second reaction pair and (ii) a second glycan specific transferase, wherein the second flag molecule is incorporated onto a glycan-modified glycoprotein in the sample; and
(b) admixing the sample with a second reporter molecule comprising (i) a second reactive molecule of the second reaction pair, wherein the second reactive molecule couples to the first reactive molecule of the second reaction pair, and (ii) a second reporter oligonucleotide comprising a reporter barcode sequence that identifies a second glycan motif, wherein the second reporter molecule is conjugated on the glycan-modified glycoprotein via the second flag molecule.

4. The method of claim 3, further comprising:
(a) incubating the sample with (i) a third flag molecule comprising a third nucleotide sugar and a first reactive molecule of a third reaction pair and (ii) a third glycan specific transferase, wherein the third flag molecule is incorporated onto a glycan modified glycoprotein in the sample; and
(b) admixing the sample with a third reporter molecule comprising
(i) a second reactive molecule of the third reaction pair, wherein the second reactive molecule couples to the first reactive molecule of the third reaction pair, and
(ii) a third reporter oligonucleotide comprising a reporter barcode sequence that identifies a third glycan motif, wherein the third reporter molecule is conjugated on the glycan-modified glycoprotein via the third flag molecule.

5. The method of claim 4, further comprising:
(a) incubating the sample with (i) a fourth flag molecule comprising a fourth nucleotide sugar and a first reactive molecule of a fourth reaction pair and (ii) a fourth glycan specific transferase, wherein the fourth flag molecule is incorporated onto a glycan-modified glycoprotein in the sample; and
(b) admixing the sample with a fourth reporter molecule comprising (i) a second reactive molecule of a fourth reaction pair, wherein the second reactive molecule couples to the first reactive molecule of said fourth reaction pair, and (ii) a fourth reporter oligonucleotide comprising a reporter barcode sequence that identifies a fourth glycan motif, wherein the fourth reporter molecule is conjugated on the glycan-modified glycoprotein via the fourth flag molecule.

6. The method of any one of claims 1-5, wherein:
(a) the sample is a tissue; and/or
(b) a plurality of partitions receive a single cell from the sample; and/or
(c) a lysate from a single cell is encapsulated in a cell bead, coated on a cell bead, embedded in a cell bead, or any combination thereof.

7. The method of claim 3 further comprising the step of:
(a) using the second reporter oligonucleotide and the nucleic acid barcode molecule of the plurality of nucleic acid barcode molecules to generate a second glycan barcoded nucleic acid molecule comprising the partition-specific barcode sequence or complementary sequence thereof and the second glycan motif-specific reporter barcode sequence or complementary sequence thereof;
(b) determining the sequence of the second glycan barcoded nucleic acid molecules or derivatives thereof to identify (i) the partition-specific barcode sequences or complementary sequences thereof and (ii) the second glycan motif-specific reporter barcode sequence or complementary sequence thereof; and
(c) using the identified partition-specific barcode sequence or complement thereof and the identified second glycan motif-specific reporter barcode sequence or complementary sequence thereof to determine the presence and/or abundance of a first glycan and a second glycan in said sample.

8. The method of claim 4, further comprising the steps of:
(a) using the third reporter oligonucleotide and the nucleic acid barcode molecule of the plurality of nucleic acid barcode molecules to generate a third glycan barcoded nucleic acid molecule comprising the partition-specific barcode sequence or complementary sequence thereof and the third glycan motif-specific reporter barcode sequence or complementary sequence thereof;
(b) determining the sequence of the third glycan barcoded nucleic acid molecules or derivatives thereof to identify (i) the partition-specific barcode sequence or complementary sequence thereof and (ii) the third glycan motif-specific reporter barcode sequence or complementary sequence thereof; and
(c) using the identified partition-specific barcode sequence or complementary sequence thereof and the identified third glycan motif-specific reporter barcode sequence or complementary sequence thereof to determine the presence and/or abundance of a first glycan, a second glycan motif and a third glycan in the sample.

9. The method of claim 5, further comprising the steps of:
(a) using the fourth reporter oligonucleotide and the nucleic acid barcode molecule of the plurality of nucleic acid barcode molecules to generate a fourth glycan barcoded nucleic acid molecule comprising the partition-specific barcode sequence or complementary sequence thereof and the fourth glycan motif-specific reporter barcode sequence or complementary sequence thereof;
(b) determining the sequence of the fourth barcoded nucleic acid molecules or derivatives thereof to identify (i) the partition-specific barcode sequence or complementary sequence thereof and (ii) the fourth glycan motif-specific reporter barcode sequence or complementary sequence thereof; and
(c) using the identified partition-specific barcode sequence or complementary sequence thereof and the identified fourth glycan motif-specific reporter barcode sequence or complementary sequence thereof to determine the presence and/or abundance of the first glycan, the second glycan, the third glycan and the fourth glycan in said sample.

10. The method of any one of claims 1-9, wherein:
(a) the first glycan specific transferase is the β1-4 galactosyltransferase, the first flag molecule is UDP-GalNAc; and the glycan on the glycan-modified glycoprotein comprises GlcNAc-O-R;
(b) the first glycan specific transferase is the α1-3-fucosyl transferase, the first flag molecule is GDP-fucose; and the glycan on the glycan-modified glycoprotein comprises LacNAc;
(c) the first glycan specific transferase is the human blood group A antigen glycosyltransferase (BgtA), the first flag molecule is UDP-GalNAc; and the glycan on the glycan-modified glycoprotein comprises Fucα1-2Gal;
(d) the first glycan specific transferase is the α1-2-fucosyl transferase, the first flag molecule is GDP-fucose; and the glycan on the glycan-modified glycoprotein comprises Galβ1-3GalNAc;
(e) the first glycan specific transferase is β1-4 N-acetyl-galactosylaminotransferase; the first flag molecule is UDP-GalNAc; and the glycan on the glycan-modified glycoprotein comprises Neu5Acα2-3Gal;
(f) the first glycan specific transferase is β-galactoside α2-6 sialyltransferase 1; the first flag molecule is CMP-Sia; and the glycan on the glycan-modified glycoprotein is the N-glycan; or
(g) the first glycan specific transferase is specific for GlcNAc-O-R; LacNAc; Fucα1-2Gal; Galβ1-3GalNAc; Neu5Acα2-3Gal; N-glycans; or O-glycans.

11. A method of determining the presence of one or more glycans in a sample comprising one or more living cells, comprising the steps of:
(a) incubating the sample with a flag molecule comprising a synthetic sugar and a first reactive molecule of a reaction pair, wherein (i) the flag molecule is a substrate for one or more glycosyltransferases of the one or more living cells, (ii) the flag molecule is incorporated onto a glycan-modified glycoprotein of one or more living cells of the sample;
(b) admixing the sample with a reporter molecule comprising (i) a second reactive molecule of the reaction pair and (ii) a reporter oligonucleotide comprising a glycan motif-specific reporter barcode sequence, wherein the second reactive molecule of the reaction pair couples to the first reactive molecule of the reaction pair, whereby the reporter molecule is conjugated to the glycan-modified glycoprotein via the flag molecule;
(c) removing unincorporated reporter molecules;
(d) partitioning the sample and a plurality of nucleic acid barcode molecules into a plurality of partitions such that a partition of the plurality comprises a cell from the sample and a plurality of nucleic acid barcode molecules comprising a partition-specific barcode sequence;
(e) using the reporter oligonucleotide and the nucleic acid barcode molecule to generate a glycan barcoded nucleic acid molecule comprising the partition-specific barcode sequence or a complementary sequence thereof and the glycan motif-specific reporter barcode sequence or complementary sequence thereof thereof;
(f) determining the sequence of the first glycan barcoded nucleic acid molecule or derivative thereof to identify (i) the partition-specific barcode sequence or complementary sequence thereof and (ii) the first glycan motif-specific reporter barcode sequence or complementary sequence thereof; and
(g) using the identified partition-specific barcode sequence or complementary sequence thereof and the identified first glycan motif-specific reporter barcode sequence or complementary sequence thereof to determine the presence and/or abundance of at least one glycan in the sample.

12. The method of claim 11, wherein:
(a) the reaction pair is selected from the group of reaction pairs consisting of an azide and an alkyne,
an azide and a phosphine,
an aldehyde and aminooxy,
an aldehyde and a hydrazine,
an aldehyde and a hydrazide,
a ketone and an aminooxy,
a hydrazine and a ketone,
cyclopropene and a tetrazine,
norbornene and tetrazine,
trans-cyclooctene and tetrazine,
an alkyne and a tetrazine,
a nitrone and an alkene,
a nitrone and alkyne,
diazo and alkyne, and
isonitrile and tetrazine; and/or
(b) the second reactive molecule of the reaction pair is an azide and the first reactive molecule of the reaction pair is an alkyne or a phosphine, or the second reactive molecule of a reaction pair is an alkyne or a phosphine and the first reactive molecule of the reaction pair is an azide; and/or
(c) the synthetic sugar is selected from the group consisting of galactose, sialic acid, fucose, mannose, N-acetylmannosamine and N-acetylgalactosamine; and/or
(d) the synthetic sugar is incorporated into a glycan selected from the group consisting of sialytated glycans; fucosylated glycans; cytosolic O-GlcNAcylated; and mucin type O-linked glycans; and/or
(e) the synthetic sugar is glycan class specific; and/or
(f) the synthetic sugar is glycan-motif specific; and/or
(g) the synthetic sugar is acetylated; and/or
(h) the one or more glycosyltransferases are endogenous or heterologous to the one or more living cells.

## Patentansprüche

1. Verfahren zum Ermitteln des Vorliegens eines oder mehrerer Glykane in einer Probe, das die folgenden Schritte umfasst:
(a) Inkubieren der Probe mit (i) einem ersten Flag-Molekül, das einen Nukleotidzucker und ein erstes reaktives Molekül eines Reaktionspaares umfasst, und (ii) einer ersten Glykan-spezifischen Transferase,
wobei das erste Flag-Molekül ein Substrat für die erste Glykan-spezifische Transferase ist;
wobei das erste Flag-Molekül in ein Glykanmodifiziertes Glykoprotein in der Probe eingebunden ist und
wobei die Probe aus der Gruppe ausgewählt ist, die aus einem Gewebe, einer Zelle, einer fixierten Zelle, einer lebenden Zelle und Zelllysaten besteht;
(b) Beimischen zu der Probe eines ersten Reportermoleküls, das Folgendes umfasst: (i) ein zweites reaktives Molekül des Reaktionspaares und (ii) ein erstes Reporteroligonukleotid, das eine erste Glykanmotivspezifische Reporter-Barcodesequenz umfasst, wobei das zweite reaktive Molekül des Reaktionspaares an das erste reaktive Molekül des Reaktionspaares koppelt, wodurch das erste Reportermolekül über das erste Flag-Molekül an das Glykan-modifizierte Glykoprotein konjugiert wird;
(c) Entfernen von nicht eingebundenen Reportermolekülen aus der Probe;
(d) Aufteilen der Probe in eine Vielzahl von Partitionen, sodass eine Partition (i) eine einzelne Zelle oder ein einzelnes Zelllysat aus der Probe und (ii) eine Vielzahl von Nukleinsäure-Barcodemolekülen umfasst, die eine partitionsspezifische Barcodesequenz umfassen;
(e) Verwenden des ersten Reporteroligonukleotids und eines Nukleinsäure-Barcodemoleküls der Vielzahl von Nukleinsäure-Barcodemolekülen, um ein erstes Glykan-barcodiertes Nukleinsäuremolekül zu erzeugen, das die partitionsspezifische Barcodesequenz oder eine komplementäre Sequenz davon und die erste Glykanmotiv-spezifische Reporter-Barcodesequenz oder die komplementäre Sequenz davon umfasst;
(f) Ermitteln der Sequenz des ersten Glykan-barcodierten Nukleinsäuremoleküls oder eines Derivats davon, um (i) die partitionsspezifische Barcodesequenz oder die komplementäre Sequenz davon und (ii) die erste Glykanmotiv-spezifische Reporter-Barcodesequenz oder die komplementäre Sequenz davon zu identifizieren; und
(g) Verwenden der identifizierten partitionsspezifischen Barcodesequenz oder der komplementären Sequenz davon und der identifizierten ersten Glykanmotiv-spezifischen Reporter-Barcodesequenz oder der komplementären Sequenz davon, um das Vorliegen und/oder die Häufigkeit von mindestens einem Glykan in der Probe zu ermitteln.

2. Verfahren nach Anspruch 1, wobei:
(a) das Reaktionspaar ausgewählt ist aus der Gruppe von Reaktionspaaren, bestehend aus
einem Azid und einem Alkin,
einem Azid und einem Phosphin,
einem Aldehyd und Aminooxy,
einem Aldehyd und einem Hydrazin,
einem Aldehyd und einem Hydrazid,
einem Keton und einem Aminooxy,
einem Hydrazin und einem Keton,
Cyclopropen und einem Tetrazin,
Norbornen und Tetrazin,
trans-Cycloocten und Tetrazin,
einem Alkin und einem Tetrazin,
einem Nitron und einem Alken,
einem Nitron und Alkin,
Diazo und Alkin und
Isonitril und Tetrazin; und/oder
(b) die erste Glykan-spezifische Transferase ausgewählt ist aus der Gruppe bestehend aus einer β1-4-Galactosyltransferase, einer Glykosyltransferase, Sialyltransferase, α1-3-Fucosyltransferase; einer Human-Blutgruppe-A-Antigen-Glykosyltransferase (BgtA); WbwK-Fucosyltransferase; α1-2-Fucosyltransferase; β1-4-N-Acetyl-Galactosylaminotransferase; β-Galactosid-α2-6-Sialyltransferase 1; und β-Galactosid-α2-3-Sialyltransferase 1; ST3Gal1; ST6Gal1; und CgtA; und/oder
(c) das erste Flag-Molekül aus der Gruppe ausgewählt ist, bestehend aus UDP-GalNAc; GDP-Fucose; UDP-GalNAc; und CMP-Sia; und/oder
(d) das Glykan-modifizierte Glykoprotein ein Glykan umfasst, das aus der Gruppe ausgewählt ist, bestehend aus GlcNAc-O-R; LacNAc; Fucα1-2Gal; Galβ1-3GalNAc; Neu5Acα2-3Gal; N-Glykanen; und O-Glykanen.

3. Verfahren nach Anspruch 1 oder Anspruch 2, ferner umfassend:
(a) Inkubieren der Probe mit (i) einem zweiten Flag-Molekül, das einen zweiten Nukleotidzucker und ein erstes reaktives Molekül eines zweiten Reaktionspaares umfasst, und (ii) einer zweiten Glykan-spezifischen Transferase, wobei das zweite Flag-Molekül in ein Glykan-modifiziertes Glykoprotein in der Probe eingebunden ist; und
(b) Beimischen zu der Probe eines zweiten Reportermoleküls, das Folgendes umfasst: (i) ein zweites reaktives Molekül des zweiten Reaktionspaares, wobei das zweite reaktive Molekül an das erste reaktive Molekül des zweiten Reaktionspaares koppelt, und (ii) ein zweites Reporteroligonukleotid, das eine Reporter-Barcodesequenz umfasst, die ein zweites Glykanmotiv identifiziert, wobei das zweite Reportermolekül über das zweite Flag-Molekül an das Glykan-modifizierte Glykoprotein konjugiert ist.

4. Verfahren nach Anspruch 3, ferner umfassend:
(a) Inkubieren der Probe mit (i) einem dritten Flag-Molekül, das einen dritten Nukleotidzucker und ein erstes reaktives Molekül eines dritten Reaktionspaares umfasst, und (ii) einer dritten Glykan-spezifischen Transferase, wobei das dritte Flag-Molekül in ein Glykan-modifiziertes Glykoprotein in der Probe eingebunden ist; und
(b) Beimischen zu der Probe eines dritten Reportermoleküls, das Folgendes umfasst:
(i) ein zweites reaktives Molekül des dritten Reaktionspaares, wobei das zweite reaktive Molekül an das erste reaktive Molekül des dritten Reaktionspaares koppelt, und
(ii) ein drittes Reporteroligonukleotid, das eine Reporter-Barcodesequenz umfasst, die ein drittes Glykanmotiv identifiziert, wobei das dritte Reportermolekül über das dritte Flag-Molekül an das Glykan-modifizierte Glykoprotein konjugiert ist.

5. Verfahren nach Anspruch 4, ferner umfassend:
(a) Inkubieren der Probe mit (i) einem vierten Flag-Molekül, das einen vierten Nukleotidzucker und ein erstes reaktives Molekül eines vierten Reaktionspaares umfasst, und (ii) einer vierten Glykan-spezifischen Transferase, wobei das vierte Flag-Molekül in ein Glykan-modifiziertes Glykoprotein in der Probe eingebunden ist; und
(b) Beimischen zu der Probe eines vierten Reportermoleküls, das Folgendes umfasst: (i) ein zweites reaktives Molekül eines vierten Reaktionspaares, wobei das zweite reaktive Molekül an das erste reaktive Molekül des vierten Reaktionspaares koppelt, und (ii) ein viertes Reporteroligonukleotid, das eine Reporter-Barcodesequenz umfasst, die ein viertes Glykanmotiv identifiziert, wobei das vierte Reportermolekül über das vierte Flag-Molekül an das Glykan-modifizierte Glykoprotein konjugiert ist.

6. Verfahren nach einem der Ansprüche 1-5, wobei:
(a) die Probe ein Gewebe ist; und/oder
(b) eine Vielzahl von Partitionen eine einzelne Zelle von der Probe empfangen; und/oder
(c) ein Lysat aus einer einzelnen Zelle in ein Zellkügelchen eingekapselt, auf ein Zellkügelchen beschichtet, in ein Zellkügelchen eingebettet ist oder eine beliebige Kombination davon.

7. Verfahren nach Anspruch 3, ferner umfassend den Schritt:
(a) Verwenden des zweiten Reporteroligonukleotids und des Nukleinsäure-Barcodemoleküls der Vielzahl von Nukleinsäure-Barcodemolekülen, um ein zweites Glykan-barcodiertes Nukleinsäuremolekül zu erzeugen, das die partitionsspezifische Barcodesequenz oder eine komplementäre Sequenz davon und die zweite Glykanmotiv-spezifische Reporter-Barcodesequenz oder die komplementäre Sequenz davon umfasst;
(b) Ermitteln der Sequenz der zweiten Glykan-barcodierten Nukleinsäuremoleküle oder von Derivaten davon, um (i) die partitionsspezifischen Barcodesequenzen oder komplementäre Sequenzen davon und (ii) die zweite Glykanmotiv-spezifische Reporter-Barcodesequenz oder die komplementäre Sequenz davon zu identifizieren; und
(c) Verwenden der identifizierten partitionsspezifischen Barcodesequenz oder des Komplements davon und der identifizierten zweiten Glykanmotiv-spezifischen Reporter-Barcodesequenz oder der komplementären Sequenz davon, um das Vorliegen und/oder die Häufigkeit eines ersten Glykans und eines zweiten Glykans in der Probe zu ermitteln.

8. Verfahren nach Anspruch 4, ferner umfassend die Schritte:
(a) Verwenden des dritten Reporteroligonukleotids und des Nukleinsäure-Barcodemoleküls der Vielzahl von Nukleinsäure-Barcodemolekülen, um ein drittes Glykan-barcodiertes Nukleinsäuremolekül zu erzeugen, das die partitionsspezifische Barcodesequenz oder eine komplementäre Sequenz davon und die dritte Glykanmotiv-spezifische Reporter-Barcodesequenz oder die komplementäre Sequenz davon umfasst;
(b) Ermitteln der Sequenz der dritten Glykan-barcodierten Nukleinsäuremoleküle oder von Derivaten davon, um (i) die partitionsspezifische Barcodesequenz oder die komplementäre Sequenz davon und (ii) die dritte Glykanmotiv-spezifische Reporter-Barcodesequenz oder die komplementäre Sequenz davon zu identifizieren; und
(c) Verwenden der identifizierten partitionsspezifischen Barcodesequenz oder der komplementären Sequenz davon und der identifizierten dritten Glykanmotiv-spezifischen Reporter-Barcodesequenz oder der komplementären Sequenz davon, um das Vorliegen und/oder die Häufigkeit eines ersten Glykans, eines zweiten Glykanmotivs und eines dritten Glykans in der Probe zu ermitteln.

9. Verfahren nach Anspruch 5, ferner umfassend die Schritte:
(a) Verwenden des vierten Reporteroligonukleotids und des Nukleinsäure-Barcodemoleküls der Vielzahl von Nukleinsäure-Barcodemolekülen, um ein viertes Glykan-barcodiertes Nukleinsäuremolekül zu erzeugen, das die partitionsspezifische Barcodesequenz oder eine komplementäre Sequenz davon und die vierte Glykanmotiv-spezifische Reporter-Barcodesequenz oder die komplementäre Sequenz davon umfasst;
(b) Ermitteln der Sequenz der vierten barcodierten Nukleinsäuremoleküle oder von Derivaten davon, um (i) die partitionsspezifische Barcodesequenz oder die komplementäre Sequenz davon und (ii) die vierte Glykanmotiv-spezifische Reporter-Barcodesequenz oder die komplementäre Sequenz davon zu identifizieren; und
(c) Verwenden der identifizierten partitionsspezifischen Barcodesequenz oder der komplementären Sequenz davon und der identifizierten vierten Glykanmotiv-spezifischen Reporter-Barcodesequenz oder der komplementären Sequenz davon, um das Vorliegen und/oder die Häufigkeit des ersten Glykans, des zweiten Glykans, des dritten Glykans und des vierten Glykans in der Probe zu ermitteln.

10. Verfahren nach einem der Ansprüche 1-9, wobei:
(a) die erste Glykan-spezifische Transferase die β1-4-Galactosyltransferase ist, das erste Flag-Molekül UDP-GalNAc ist; und das Glykan auf dem Glykan-modifizierten Glykoprotein GlcNAc-O-R umfasst;
(b) die erste Glykan-spezifische Transferase die α1-3-Fucosyltransferase ist, das erste Flag-Molekül GDP-Fucose ist; und das Glykan auf dem Glykan-modifizierten Glykoprotein LacNAc umfasst;
(c) die erste Glykan-spezifische Transferase die Human-Blutgruppe-A-Antigen-Glycosyltransferase (BgtA) ist, das erste Flag-Molekül UDP-GalNAc ist; und das Glykan auf dem Glykan-modifizierten Glykoprotein Fucα1-2Gal umfasst;
(d) die erste Glykan-spezifische Transferase die α1-2-Fucosyltransferase ist, das erste Flag-Molekül GDP-Fucose ist; und das Glykan auf dem Glykan-modifizierten Glykoprotein Galβ1-3GalNAc umfasst;
(e) die erste Glykan-spezifische Transferase β1-4-N-Acetyl-Galactosylaminotransferase ist; das erste Flag-Molekül UDP-GalNAc ist; und das Glykan auf dem Glykan-modifizierten Glykoprotein Neu5Acα2-3Gal umfasst;
(f) die erste Glykan-spezifische Transferase β-Galactosid-α2-6-Sialyltransferase 1 ist; das erste Flag-Molekül CMP-Sia ist; und das Glykan auf dem Glykan-modifizierten Glykoprotein das N-Glykan ist; oder
(g) die erste Glykan-spezifische Transferase spezifisch ist für GlcNAc-O-R; LacNAc; Fucα1-2Gal; Galβ1-3GalNAc; Neu5Acα2-3Gal; N-Glykane; oder O-Glykane.

11. Verfahren zum Ermitteln des Vorliegens eines oder mehrerer Glykane in einer Probe, die eine oder mehrere lebende Zellen umfasst, das die folgenden Schritte umfasst:
(a) Inkubieren der Probe mit einem Flag-Molekül, das einen synthetischen Zucker und ein erstes reaktives Molekül eines Reaktionspaares umfasst, wobei (i) das Flag-Molekül ein Substrat für eine oder mehrere Glycosyltransferasen der einen oder der mehreren lebenden Zellen ist, (ii) das Flag-Molekül in ein Glykan-modifiziertes Glykoprotein einer oder mehrerer lebender Zellen der Probe eingebunden ist;
(b) Beimischen zu der Probe eines Reportermoleküls, das Folgendes umfasst: (i) ein zweites reaktives Molekül des Reaktionspaares und (ii) ein Reporteroligonukleotid, das eine Glykanmotiv-spezifische Reporter-Barcodesequenz umfasst, wobei das zweite reaktive Molekül des Reaktionspaares an das erste reaktive Molekül des Reaktionspaares koppelt, wodurch das Reportermolekül über das Flag-Molekül an das Glykan-modifizierte Glykoprotein konjugiert wird;
(c) Entfernen von nicht eingebundenen Reportermolekülen;
(d) Aufteilen der Probe und einer Vielzahl von Nukleinsäure-Barcodemolekülen in eine Vielzahl von Partitionen, sodass eine Partition der Vielzahl eine Zelle aus der Probe und eine Vielzahl von Nukleinsäure-Barcodemoleküle umfasst, die eine partitionsspezifische Barcodesequenz umfassen;
(e) Verwenden des Reporteroligonukleotids und des Nukleinsäure-Barcodemoleküls, um ein Glykan-barcodiertes Nukleinsäuremolekül zu erzeugen, das die partitionsspezifische Barcodesequenz oder eine komplementäre Sequenz davon und die Glykanmotiv-spezifische Reporter-Barcodesequenz oder die komplementäre Sequenz davon umfasst;
(f) Ermitteln der Sequenz des ersten Glykan-barcodierten Nukleinsäuremoleküls oder eines Derivats davon, um (i) die partitionsspezifische Barcodesequenz oder die komplementäre Sequenz davon und (ii) die erste Glykanmotiv-spezifische Reporter-Barcodesequenz oder die komplementäre Sequenz davon zu identifizieren; und
(g) Verwenden der identifizierten partitionsspezifischen Barcodesequenz oder der komplementären Sequenz davon und der identifizierten ersten Glykanmotiv-spezifischen Reporter-Barcodesequenz oder der komplementären Sequenz davon, um das Vorliegen und/oder die Häufigkeit von mindestens einem Glykan in der Probe zu ermitteln.

12. Verfahren nach Anspruch 11, wobei:
(a) das Reaktionspaar ausgewählt ist aus der Gruppe der Reaktionspaare, bestehend aus
einem Azid und einem Alkin,
einem Azid und einem Phosphin,
einem Aldehyd und Aminooxy,
einem Aldehyd und einem Hydrazin,
einem Aldehyd und ein Hydrazid,
einem Keton und einem Aminooxy,
einem Hydrazin und einem Keton,
Cyclopropen und einem Tetrazin,
Norbornen und Tetrazin,
trans-Cycloocten und Tetrazin,
einem Alkin und einem Tetrazin,
einem Nitron und einem Alken,
einem Nitron und Alkin,
Diazo und Alkin und
Isonitril und Tetrazin; und/oder
(b) das zweite reaktive Molekül des Reaktionspaares ein Azid ist und das erste reaktive Molekül des Reaktionspaares ein Alkin oder ein Phosphin ist oder das zweite reaktive Molekül eines Reaktionspaares ein Alkin oder ein Phosphin ist und das erste reaktive Molekül des Reaktionspaares ein Azid ist; und/oder
(c) der synthetische Zucker ausgewählt ist aus der Gruppe bestehend aus Galactose, Sialinsäure, Fucose, Mannose, N-Acetylmannosamin und N-Acetylgalactosamin; und/oder
(d) der synthetische Zucker in ein Glykan eingebunden ist, das aus der Gruppe ausgewählt ist, bestehend aus sialytierten Glykanen; fucosylierten Glykanen; zytosolischen O-GlcNAcylierten; und Mucin-Typ-O-gebundenen Glykanen; und/oder
(e) der synthetische Zucker Glykanklassen-spezifisch ist; und/oder
(f) der synthetische Zucker Glykanmotiv-spezifisch ist; und/oder
(g) der synthetische Zucker acetyliert ist; und/oder
(h) die eine oder die mehreren Glycosyltransferasen endogen oder heterolog zu der einen oder den mehreren lebenden Zellen sind.

## Revendications

1. Procédé de détermination de la présence d'un ou de plusieurs glycanes dans un échantillon, comprenant les étapes suivantes :
(a) l'incubation de l'échantillon avec (i) une première molécule drapeau comprenant un sucre nucléotide et une première molécule réactive d'une paire de réaction et (ii) une première transférase spécifique de glycane, dans lequel la première molécule drapeau est un substrat pour la première transférase spécifique de glycane ;
dans lequel la première molécule drapeau est incorporée sur une glycoprotéine modifiée par glycane dans l'échantillon, et
dans lequel l'échantillon est choisi dans le groupe constitué par un tissu, une cellule, une cellule fixée, une cellule vivante, et des lysats cellulaires ;
(b) l'ajout à l'échantillon d'une première molécule de rapporteur comprenant (i) une deuxième molécule réactive de la paire de réaction et (ii) un premier oligonucléotide rapporteur comprenant une première séquence de code à barres de rapporteur spécifique du motif glycane, dans lequel la deuxième molécule réactive de la paire de réaction se couple à la première molécule réactive de la paire de réaction, moyennant quoi la première molécule de rapporteur est conjuguée à la glycoprotéine modifiée par glycane par l'intermédiaire de la première molécule drapeau ;
(c) l'élimination des molécules de rapporteur non incorporées de l'échantillon ;
(d) le compartimentage de l'échantillon en une pluralité de compartiments de sorte qu'un compartiment comprend (i) une cellule unique ou un lysat cellulaire unique issu de l'échantillon et (ii) une pluralité de molécules de code à barres d'acide nucléique comprenant une séquence de code à barres spécifique de compartiment ;
(e) l'utilisation du premier oligonucléotide rapporteur et d'une molécule de code à barres d'acide nucléique de la pluralité de molécules de code à barres d'acide nucléique pour générer une première molécule d'acide nucléique codée par code à barres de glycane comprenant la séquence de code à barres spécifique de compartiment ou une séquence complémentaire de celle-ci et la première séquence de code à barres de rapporteur spécifique de motif glycane ou une séquence complémentaire de celle-ci ;
(f) la détermination de la séquence de la première molécule d'acide nucléique codée par code à barres de glycane ou d'un dérivé de celle-ci pour identifier (i) la séquence de code à barres spécifique de compartiment ou une séquence complémentaire de celle-ci et (ii) la première séquence de code à barres de rapporteur spécifique de motif de glycane ou une séquence complémentaire de celle-ci ; et
(g) l'utilisation de la séquence de code à barres spécifique de compartiment identifiée ou d'une séquence complémentaire de celle-ci et de la première séquence de code à barres de rapporteur spécifique de motif glycane identifiée ou d'une séquence complémentaire de celle-ci pour déterminer la présence et/ou l'abondance d'au moins un glycane dans l'échantillon.

2. Procédé selon la revendication 1, dans lequel :
(a) la paire de réaction est choisie dans le groupe de paires de réaction constitué par
un azide et un alcyne,
un azide et une phosphine,
un aldéhyde et aminooxy,
un aldéhyde et une hydrazine,
un aldéhyde et un hydrazide,
une cétone et un aminooxy,
une hydrazine et une cétone,
cyclopropène et une tétrazine,
norbornène et tétrazine,
trans-cyclooctène et tétrazine,
un alcyne et une tétrazine,
une nitrone et un alcène,
une nitrone et alcyne,
diazo et alcyne, et
isonitrile et tétrazine ; et/ou
(b) la première transférase spécifique de glycane est choisie dans le groupe constitué par une β1-4 galatosyl-transférase, une glycosyl-transférase, une sialyl-transférase, une α1-3-fucosyl-transférase ; une glycosyltransférase d'antigène du groupe sanguin humain A (BgtA) ; une fucosyl-transférase WbwK ; une α1-2-fucosyl transférase ; une β1-4 N-acétyl-galactosyl-aminotransférase ; une β-galactoside α2-6 sialyl-transférase 1 ; et une β-galactoside α2-3 sialyl-transférase 1 ; ST3Gal1 ; ST6Gal1 ; et CgtA ; et/ou
(c) la première molécule drapeau est choisie dans le groupe constitué par UDP-GalNAc ; GDP-fucose ; UDP-GalNAc ; et CMP-Sia ; et/ou
(d) la glycoprotéine modifiée par glycane comprend un glycane choisi dans le groupe constitué par GlcNAc-OR ; LacNAc ; Fuca1-2Gal ; Galβ1-3GalNAc ; Neu5Acα2-3Gal ; N-glycanes ; et O-glycanes.

3. Procédé selon la revendication 1 ou la revendication 2, comprenant en outre :
(a) l'incubation de l'échantillon avec (i) une deuxième molécule drapeau comprenant un deuxième sucre nucléotide et une première molécule réactive d'une deuxième paire de réaction et (ii) une deuxième transférase spécifique de glycane, dans lequel la deuxième molécule drapeau est incorporée sur une glycoprotéine modifiée par glycane dans l'échantillon ; et
(b) l'ajout à l'échantillon d'une deuxième molécule de rapporteur comprenant (i) une deuxième molécule réactive de la deuxième paire de réaction, dans lequel la deuxième molécule réactive se couple à la première molécule réactive de la deuxième paire de réaction, et (ii) un deuxième oligonucléotide rapporteur comprenant une séquence de code à barres de rapporteur qui identifie un deuxième motif glycane, dans lequel la deuxième molécule de rapporteur est conjuguée sur la glycoprotéine modifiée par glycane par l'intermédiaire de la deuxième molécule drapeau.

4. Procédé selon la revendication 3, comprenant en outre :
(a) l'incubation de l'échantillon avec (i) une troisième molécule drapeau comprenant un troisième sucre nucléotide et une première molécule réactive d'une troisième paire de réaction et (ii) une troisième transférase spécifique de glycane, dans lequel la troisième molécule drapeau est incorporée sur une glycoprotéine modifiée par glycane dans l'échantillon ; et
(b) l'ajout à l'échantillon d'une troisième molécule de rapporteur comprenant
(i) une deuxième molécule réactive de la troisième paire de réaction, dans lequel la deuxième molécule réactive se couple à la première molécule réactive de la troisième paire de réaction, et
(ii) un troisième oligonucléotide rapporteur comprenant une séquence de code à barres de rapporteur qui identifie un troisième motif glycane, dans lequel la troisième molécule de rapporteur est conjuguée sur la glycoprotéine modifiée par glycane par l'intermédiaire de la troisième molécule drapeau.

5. Procédé selon la revendication 4, comprenant en outre :
(a) l'incubation de l'échantillon avec (i) une quatrième molécule drapeau comprenant un quatrième sucre nucléotide et une première molécule réactive d'une quatrième paire de réaction et (ii) une quatrième transférase spécifique de glycane, dans lequel la quatrième molécule drapeau est incorporée sur une glycoprotéine modifiée par glycane dans l'échantillon ; et
(b) l'ajout à l'échantillon d'une quatrième molécule de rapporteur comprenant (i) une deuxième molécule réactive de la quatrième paire de réaction, dans lequel la deuxième molécule réactive se couple à la première molécule réactive de ladite quatrième paire de réaction, et (ii) un quatrième oligonucléotide rapporteur comprenant une séquence de code à barres de rapporteur qui identifie un quatrième motif glycane, dans lequel la quatrième molécule de rapporteur est conjuguée sur la glycoprotéine modifiée par glycane par l'intermédiaire de la quatrième molécule drapeau.

6. Procédé selon l'une quelconque des revendications 1 à 5, dans lequel :
(a) l'échantillon est un tissu ; et/ou
(b) une pluralité de compartiments reçoivent une cellule unique issue de l'échantillon ; et/ou
(c) un lysat issu d'une cellule unique est encapsulé dans une bille cellulaire, déposé en enrobage sur une bille cellulaire, intégré dans une bille cellulaire, ou toute combinaison de ces états.

7. Procédé selon la revendication 3, comprenant en outre l'étape suivante :
(a) l'utilisation du deuxième oligonucléotide rapporteur et de la molécule de code à barres d'acide nucléique de la pluralité de molécules de code à barres d'acide nucléique pour générer une deuxième molécule d'acide nucléique codée par code à barres de glycane comprenant la séquence de code à barres spécifique de compartiment ou une séquence complémentaire de celle-ci et la deuxième séquence de code à barres de rapporteur spécifique de motif glycane ou une séquence complémentaire de celle-ci ;
(b) la détermination de la séquence des deuxièmes molécules d'acide nucléique codées par code à barres de glycane ou de dérivés de celle-ci pour identifier (i) les séquences de code à barres spécifique de compartiment ou des séquences complémentaires de celles-ci et (ii) la deuxième séquence de code à barres de rapporteur spécifique du motif glycane ou une séquence complémentaire de celle-ci ; et
(c) l'utilisation de la séquence de code à barres spécifique de compartiment identifiée ou d'un complément de celle-ci et de la deuxième séquence de code à barres de rapporteur spécifique de motif glycane identifiée ou d'une séquence complémentaire de celle-ci pour déterminer la présence et/ou l'abondance d'un premier glycane et d'un deuxième glycane dans ledit échantillon.

8. Procédé selon la revendication 4, comprenant en outre les étapes suivantes :
(a) l'utilisation du troisième oligonucléotide rapporteur et de la molécule de code à barres d'acide nucléique de la pluralité de molécules de code à barres d'acide nucléique pour générer une troisième molécule d'acide nucléique codée par code à barres de glycane comprenant la séquence de code à barres spécifique de compartiment ou une séquence complémentaire de celle-ci et la troisième séquence de code à barres de rapporteur spécifique de motif glycane ou une séquence complémentaire de celle-ci ;
(b) la détermination de la séquence des troisièmes molécules d'acide nucléique codées par code à barres de glycane ou de dérivés de celle-ci pour identifier (i) la séquence de code à barres spécifique de compartiment ou une séquence complémentaire de celle-ci et (ii) la troisième séquence de code à barres de rapporteur spécifique de motif de glycane ou une séquence complémentaire de celle-ci ; et
(c) l'utilisation de la séquence de code à barres spécifique de compartiment identifiée ou d'une séquence complémentaire de celle-ci et de la troisième séquence de code à barres de rapporteur spécifique de motif glycane identifiée ou d'une séquence complémentaire de celle-ci pour déterminer la présence et/ou l'abondance d'un premier glycane, d'un deuxième motif glycane et d'un troisième glycane dans ledit échantillon.

9. Procédé selon la revendication 5, comprenant en outre les étapes suivantes :
(a) l'utilisation du quatrième oligonucléotide rapporteur et de la molécule de code à barres d'acide nucléique de la pluralité de molécules de code à barres d'acide nucléique pour générer une quatrième molécule d'acide nucléique codée par code à barres de glycane comprenant la séquence de code à barres spécifique de compartiment ou une séquence complémentaire de celle-ci et la quatrième séquence de code à barres de rapporteur spécifique de motif glycane ou une séquence complémentaire de celle-ci ;
(b) la détermination de la séquence des quatrièmes molécules d'acide nucléique codées par code à barres ou de dérivés de celle-ci pour identifier (i) la séquence de code à barres spécifique de compartiment ou une séquence complémentaire de celle-ci et (ii) la quatrième séquence de code à barres de rapporteur spécifique de motif glycane ou une séquence complémentaire de celle-ci ; et
(c) l'utilisation de la séquence de code à barres spécifique de compartiment identifiée ou d'une séquence complémentaire de celle-ci et de la quatrième séquence de code à barres de rapporteur spécifique de motif glycane identifiée ou d'une séquence complémentaire de celle-ci pour déterminer la présence et/ou l'abondance du premier glycane, du deuxième glycane, du troisième glycane et du quatrième glycane dans ledit échantillon.

10. Procédé selon l'une quelconque des revendications 1 à 9, dans lequel :
(a) la première transférase spécifique de glycane est la β-1-4 galactosyl-transférase, la première molécule drapeau est UDP-GalNAc ; et le glycane sur la glycoprotéine modifiée par glycane comprend GlcNAc-OR ;
(b) la première transférase spécifique de glycane est l'α1-3-fucosyl transférase, la première molécule drapeau est GDP-fucose ; et le glycane sur la glycoprotéine modifiée par glycane comprend LacNAc ;
(c) la première transférase spécifique de glycane est la glycosyltransférase d'antigène du groupe sanguin humain A (BgtA), la première molécule drapeau est UDP-GalNAc ; et le glycane sur la glycoprotéine modifiée par glycane comprend Fucα1-2Gal ;
(d) la première transférase spécifique de glycane est l'α1-2-fucosyl transférase, la première molécule drapeau est GDP-fucose ; et le glycane sur la glycoprotéine modifiée par glycane comprend Galβ1-3GalNAc ;
(e) la première transférase spécifique de glycane est la β1-4 N-acétyl-galactosyl-aminotransférase ; la première molécule drapeau est UDP-GalNAc ; et le glycane sur la glycoprotéine modifiée par glycane comprend Neu5Acα2-3Gal ;
(f) la première transférase spécifique de glycane est la β-galactoside α2-6 sialyltransférase 1 ; la première molécule drapeau est CMP-Sia ; et le glycane sur la glycoprotéine modifiée par glycane est le N-glycane ; ou
(g) la première transférase spécifique de glycane est spécifique de GlcNAc-O-R ; LacNAc ; Fucα1-2Gal ; Galβ1-3GalNAc ; Neu5Acα2-3Gal ; N-glycanes ; ou O-glycanes.

11. Procédé de détermination de la présence d'un ou de plusieurs glycanes dans un échantillon comprenant une ou plusieurs cellules vivantes, comprenant les étapes suivantes :
(a) l'incubation de l'échantillon avec une molécule drapeau comprenant un sucre synthétique et une première molécule réactive d'une paire de réaction, dans lequel (i) la molécule drapeau est un substrat pour une ou plusieurs glycosyl-transférases des une ou plusieurs cellules vivantes, (ii) la molécule drapeau est incorporée sur une glycoprotéine modifiée par glycane d'une ou de plusieurs cellules vivantes de l'échantillon ;
(b) l'ajout à l'échantillon d'une molécule de rapporteur comprenant (i) une deuxième molécule réactive de la paire de réaction et (ii) un oligonucléotide rapporteur comprenant une séquence de code à barres de rapporteur spécifique de motif glycane, dans lequel la deuxième molécule réactive de la paire de réaction se couple à la première molécule réactive de la paire de réaction, moyennant quoi la molécule de rapporteur est conjuguée à la glycoprotéine modifiée par glycane par l'intermédiaire de la molécule drapeau ;
(c) l'élimination des molécules de rapporteur non incorporées ;
(d) le compartimentage de l'échantillon et d'une pluralité de molécules de code à barres d'acide nucléique en une pluralité de compartiments de sorte qu'un compartiment comprend une cellule issue de l'échantillon et une pluralité de molécules de code à barres d'acide nucléique comprenant une séquence de code à barres spécifique de compartiment ;
(e) l'utilisation de l'oligonucléotide rapporteur et de la molécule de code à barres d'acide nucléique pour générer une molécule d'acide nucléique codée par code à barres de glycane comprenant la séquence de code à barres spécifique de compartiment ou une séquence complémentaire de celle-ci et la séquence de code à barres de rapporteur spécifique de motif glycane ou une séquence complémentaire de celle-ci de celle-ci ;
(f) la détermination de la séquence de la première molécule d'acide nucléique codée par code à barres de glycane ou d'un dérivé de celle-ci pour identifier (i) la séquence de code à barres spécifique de compartiment ou une séquence complémentaire de celle-ci et (ii) la première séquence de code à barres de rapporteur spécifique de motif glycane ou une séquence complémentaire de celle-ci ; et
(g) l'utilisation de la séquence de code à barres spécifique de compartiment identifiée ou d'une séquence complémentaire de celle-ci et de la première séquence de code à barres de rapporteur spécifique de motif glycane identifiée ou d'une séquence complémentaire de celle-ci pour déterminer la présence et/ou l'abondance d'au moins un glycane dans l'échantillon.

12. Procédé selon la revendication 11, dans lequel :
(a) la paire de réaction est choisie dans le groupe de paires de réaction constitué par
un azide et un alcyne,
un azide et une phosphine,
un aldéhyde et aminooxy,
un aldéhyde et une hydrazine,
un aldéhyde et un hydrazide,
une cétone et un aminooxy,
une hydrazine et une cétone,
cyclopropène et une tétrazine,
norbornène et tétrazine,
trans-cyclooctène et tétrazine,
un alcyne et une tétrazine,
une nitrone et un alcène,
une nitrone et alcyne,
diazo et alcyne, et
isonitrile et tétrazine ; et/ou
(b) la deuxième molécule réactive de la paire de réaction est un azide et la première molécule réactive de la paire de réaction est un alcyne ou une phosphine, ou la deuxième molécule réactive d'une paire de réaction est un alcyne ou une phosphine et la première molécule réactive de la paire de réaction est un azide ; et/ou
(c) le sucre synthétique est choisi dans le groupe constitué par le galactose, l'acide sialique, le fucose, le mannose, la N-acétylmannosamine et la **N-**acétylgalactosamine ; et/ou
(d) le sucre synthétique est incorporé dans un glycane choisi dans le groupe constitué par glycanes sialylés ; glycanes fucosylés ; cytosolique O-GlcNAcylé ; et glycanes liés à la mucine de type O ; et/ou
(e) le sucre synthétique est spécifique de la classe de glycane ; et/ou
(f) le sucre synthétique est spécifique de motif glycane ; et/ou
(g) le sucre synthétique est acétylé ; et/ou
(h) les une ou plusieurs glycosyltransférases sont endogènes ou hétérologues aux une ou plusieurs cellules vivantes.
